# EUROPEAN PATENT APPLICATION

(11) **EP 4 353 725 A1**
(43) Date of publication of application: **17.04.2024**
(21) Application number: 22819604.4
(22) Date of filing: 09.06.2022
(51) Int. Cl.: C07D 487/04, C07D 471/12, A61K 31/519, A61P 29/00

(54) **SALT AND CRYSTAL FORM OF PYRAZOLE-CONTAINING POLYCYCLIC DERIVATIVE, AND PREPARATION METHOD THEREFOR AND USE THEREOF**

(30) Priority: 09.06.2021 CN 202110642255; 11.06.2021 CN 202110655580
(71) Applicant: Shanghai Hansoh Biomedical Co., Ltd., Shanghai 201203 (CN); Jiangsu Hansoh Pharmaceutical Group Co., Ltd., Lianyungang, Jiangsu 222047 (CN)
(72) Inventor: DONG, Hua, Shanghai 201203 (CN); GUO, Linsong, Shanghai 201203 (CN)
(74) Representative: EP&C
(86) International application number: PCT/CN2022/097828
(87) International publication number: WO 2022/258007

(57) **Abstract**

An acid salt and a crystal form of a pyrazole-containing polycyclic derivative represented by formula (I-a), and a preparation method therefor, a pharmaceutical composition containing a therapeutically effective amount of the crystal form, and an intermediate for preparing the derivative. In particular, the present invention relates to the use of the salt and the crystal form of the compound represented by general formula (I-a) as a P2X3 inhibitor in the preparation of a drug for treating neurogenic diseases.

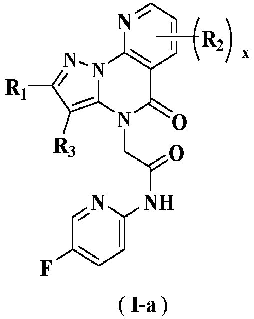

## Description

### FIELD OF THE INVENTION

The present invention belongs to the field of biomedicine, and specifically relates to a salt of pyrazole-containing polycyclic derivative, crystal form thereof, preparation method therefor, and use thereof.

### BACKGROUND OF THE INVENTION

P2X receptors, also known as P2X purinoreceptors, are a family of cation-permeable ATP ligand-gated ion channels that can bind to extracellular ATP. P2X receptors have seven subunits, and exist in the form of homotrimers or heterotrimers. P2X receptors are mainly expressed on nerve endings (presynaptic and postsynaptic) of the nervous system, and regulate synaptic transmission. P2X3 receptor is a member of the P2X family, and is a key sensory receptor for sensing upper airway stimuli and triggering the cough reflex. P2X3 receptor is thought to play a key role in the sensitisation of specific sensory nerves, involve in pain and cough, and in the perception of bone cancer pain. Blocking P2X3 can suppress cough signaling.

Cough is a defensive nerve reflex of the body, which helps to clear respiratory secretions and harmful factors. However, frequent and severe cough will seriously affect the patient's work, life and social activities. Cough is divided into acute, subacute, and chronic cough. Chronic cough is defined as coughing for more than 8 weeks, with cough as the main or only symptom, and no obvious lesions in the lungs on chest imaging examination. Chronic cough has long been considered a consequence of various diseases such as asthma/eosinophilic bronchitis, rhinitis and gastroesophageal acid reflux disease. However, recent evidences show that chronic cough is a clinical symptom of neuroticism with unique intrinsic pathophysiological features. Unexplained chronic cough or idiopathic cough is mainly manifested by chronic irritating dry cough. It is sensitive to external stimuli and generally has high cough sensitivity. Cough hypersensitivity is its physiological and pathological mechanism. Cough-related afferent nerve abnormalities may be the cause of refractory or unexplained chronic cough. Chronic cough can cause complications in cardiovascular, digestive, neurological, urinary, musculoskeletal systems, such as urinary incontinence, syncope, insomnia, anxiety, etc.

In view of the pathophysiology of cough hypersensitivity syndrome, treatment should aim to reduce cough sensitivity. Current treatment options are limited, including pharmacological and non-pharmacological approaches. Clinical study results have shown that the neuromodulator drug gabapentin is effective. Other drugs such as amitriptyline, baclofen, carbamazepine and pregabalin can also be used. Severe cough can be treated by appropriate antitussives. Antitussives are mainly divided into central antitussives and peripheral antitussives. Central antitussives are divided into dependent antitussives (morphine alkaloids and their derivatives) and non-dependent antitussives (synthetic dextromethorphan and pentoverine). Dependent antitussives have side effects such as addiction and anesthesia. Non-dependent antitussives are widely used in clinical practice. Peripheral antitussives, also known as ending antitussives, act by inhibiting a certain link in the cough reflex arc, including local anesthetics (narcotine, benzonatate) and mucosal protectants (benproperine and moguisteine).

At present, there are no approved P2X3 receptor antagonist small molecule drugs on the market. P2X3 receptor antagonist drugs currently in clinical stage include MK-7264 developed by Merck & Co. It is used to treat diseases such as chronic cough, pain and pulmonary fibrosis. It has low selectivity to P2X3/P2X2/3 and good safety, but has side effects such as loss of taste. At present, it has entered the phase III clinical study for the indication of chronic cough. BLU5937 developed by Bellus Health has high selectivity, and no side effects such as taste side effects appeared in phase I clinical trials. On July 6, 2020, Bellus Health announced the main results of the phase 2 RELIEF trial of BLU-5937 in patients with refractory chronic cough: in the phase II clinical study, the RELIEF trial failed to achieve statistical significance for the primary endpoint of placebo-adjusted reduction in cough frequency at any dose. In addition, BAY-1817080 and BAY-1902607 developed by Bayer and S-600918 developed by Shionogi are currently in clinical phase I/II for the indication of chronic cough. Therefore, there is an urgent need to develop safe, non-addictive, non-narcotic and highly selective P2X3 receptor inhibitor drugs for treating diseases such as chronic cough to meet the huge market demand.

The patent application (application number: PCT/CN2020/134264) by Jiangsu Hansoh Pharmaceutical Group Co. Ltd. discloses the structures of a series of pyrazole-containing polycyclic derivative inhibitors. In the subsequent research and development, in order to make the product easy to handle, filter, dry, store, and give the product long-term stability and high bioavailability, the present invention has conducted a comprehensive study on the salts and crystal forms of the above substance to obtain the most suitable crystal form. In addition, the raw materials for preparing the pyrazole-containing polycyclic derivative are expensive, and the reaction conditions are strict, which are not suitable for large-scale industrial production. The present invention develops a preparation method suitable for industrial production.

### SUMMARY OF THE INVENTION

The whole content involved in the patent application PCT/CN2020/134264 is incorporated into the present invention by reference.

The object of the present invention is to provide an acid salt of a compound of formula (I-a) or a stereoisomer thereof, 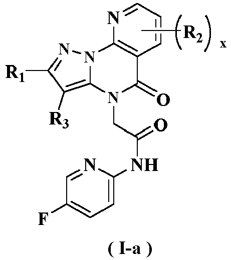 wherein:
R₁ is selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₁₂ cycloalkyl, -(CH₂)ₙC(O)Rₐ, 3 to 12 membered heterocyclyl, C₆₋₁₄ aryl and 5 to 14 membered heteroaryl, the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₁₂ cycloalkyl, -(CH₂)ₙC(O)Rₐ, 3 to 12 membered heterocyclyl, C₆₋₁₄ aryl and 5 to 14 membered heteroaryl are each optionally substituted by one or more substituents selected from the group consisting of deuterium, halogen, amino, hydroxy, cyano, nitro, oxo, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3 to 12 membered heterocyclyl, C₆₋₁₂ aryl and 5 to 12 membered heteroaryl;
Rₐ is selected from the group consisting of hydrogen, deuterium, halogen, amino, nitro, hydroxy, cyano, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally substituted by one or more substituents selected from the group consisting of deuterium, halogen, amino, nitro, hydroxy, cyano, carboxy, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
R₂ is selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₁₂ cycloalkyl, 3 to 12 membered heterocyclyl, C₆₋₁₄ aryl and 5 to 14 membered heteroaryl, the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₁₂ cycloalkyl, 3 to 12 membered heterocyclyl, C₆₋₁₄ aryl and 5 to 14 membered heteroaryl are each optionally substituted by one or more substituents selected from the group consisting of deuterium, halogen, amino, hydroxy, cyano, nitro, oxo, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3 to 12 membered heterocyclyl, C₆₋₁₂ aryl and 5 to 12 membered heteroaryl;
R₃ is selected from the group consisting of hydrogen, deuterium, halogen, amino, hydroxy, cyano, oxo, thioxo, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3 to 12 membered heterocyclyl, C₆₋₁₄ aryl and 5 to 14 membered heteroaryl; and
x is an integer from 0 to 3, preferably 0, 1 or 2, more preferably 0 or 1;
n is an integer from 0 to 3, preferably 0, 1 or 2, more preferably 0 or 1;
the acid in the acid salt is an inorganic acid or an organic acid; preferably, the inorganic acid is selected from the group consisting of hydrochloric acid, sulfuric acid, nitric acid, hydrobromic acid, hydrofluoric acid, hydroiodic acid and phosphoric acid; the organic acid is selected from the group consisting of 2,5-dihydroxybenzoic acid, 1-hydroxy-2-naphthoic acid, acetic acid, ethanesulfonic acid, dichloroacetic acid, trichloroacetic acid, acetohydroxamic acid, adipic acid, benzenesulfonic acid, 4-chlorobenzenesulfonic acid, benzoic acid, 4-acetamidobenzoic acid, 4-aminobenzoic acid, capric acid, caproic acid, caprylic acid, cinnamic acid, citric acid, cyclohexane sulfamic acid, camphorsulfonic acid, aspartic acid, camphoric acid, gluconic acid, glucuronic acid, glutamic acid, erythorbic acid, lactic acid, malic acid, mandelic acid, pyroglutamic acid, tartaric acid, dodecyl sulfuric acid, dibenzoyl tartaric acid, ethane-1,2-disulfonic acid, ethanesulfonic acid, formic acid, fumaric acid, galactonic acid, gentisic acid, glutaric acid, 2-oxoglutaric acid, glycolic acid, hippuric acid, isethionic acid, lactobionic acid, ascorbic acid, aspartic acid, lauric acid, camphoric acid, maleic acid, malonic acid, methanesulfonic acid, 1,5-naphthalenedisulfonic acid, naphthalene-2-sulfonic acid, nicotinic acid, oleic acid, orotic acid, oxalic acid, palmitic acid, pamoic acid, propionic acid, salicylic acid, 4-aminosalicylic acid, sebacic acid, stearic acid, succinic acid, thiocyanic acid, undecenoic acid, trifluoroacetic acid, benzenesulfonic acid, p-toluenesulfonic acid and L-malic acid; and preferably selected from the group consisting of hydrochloric acid, sulfuric acid, phosphoric acid, ethanesulfonic acid, benzenesulfonic acid, methanesulfonic acid, fumaric acid, isethionic acid, oxalic acid and hydrobromic acid.

In a preferred embodiment of the present invention, the formula (I-a) is further as shown in formula (II-a):

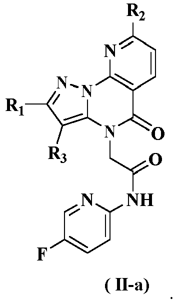

In a preferred embodiment of the present invention, R₁ is selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₈ cycloalkyl, -(CH₂)ₙC(O)Rₐ, 3 to 8 membered heterocyclyl, C₆₋₁₀ aryl and 5 to 10 membered heteroaryl, the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₈ cycloalkyl, -(CH₂)ₙC(O)Rₐ, 3 to 8 membered heterocyclyl, C₆₋₁₀ aryl and 5 to 10 membered heteroaryl are each optionally substituted by one or more substituents selected from the group consisting of deuterium, halogen, amino, hydroxy, cyano, nitro, oxo, C₁₋₃ alkyl, C₂₋₃ alkenyl, C₂₋₃ alkynyl, C₁₋₃ deuterated alkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3 to 8 membered heterocyclyl, C₆₋₁₀ aryl and 5 to 10 membered heteroaryl;
Rₐ is selected from the group consisting of hydrogen, deuterium, halogen, amino, nitro, hydroxy, cyano, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally substituted by one or more substituents selected from the group consisting of deuterium, halogen, amino, nitro, hydroxy, cyano, carboxy, oxo, thioxo, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
preferably, R₁ is selected from the group consisting of hydrogen, halogen, amino, cyano, C₁₋₃ alkyl, C₂₋₆ alkenyl, C₁₋₆ haloalkyl, C₁₋₆ alkoxy, C₁₋₆ haloalkoxy, C₁₋₆ hydroxyalkyl, C₃₋₈ cycloalkyl, -(CH₂)ₙC(O)Rₐ, 3 to 8 membered heterocyclyl containing 1 to 3 atoms selected from the group consisting of nitrogen, oxygen and sulfur, C₆₋₁₀ aryl and 5 to 10 membered heteroaryl containing 1 to 3 atoms selected from the group consisting of nitrogen, oxygen and sulfur, the amino, C₁₋₆ alkyl, C₂₋₆ alkenyl, C₁₋₆ haloalkyl, C₁₋₆ alkoxy, C₁₋₆ haloalkoxy, C₁₋₆ hydroxyalkyl, C₃₋₈ cycloalkyl, 3 to 8 membered heterocyclyl containing 1 to 3 atoms selected from the group consisting of nitrogen, oxygen and sulfur, C₆₋₁₀ aryl and 5 to 10 membered heteroaryl containing 1 to 3 atoms selected from the group consisting of nitrogen, oxygen and sulfur are each optionally substituted by one or more substituents selected from the group consisting of deuterium, halogen, amino, hydroxy, cyano, nitro, oxo, C₁₋₃ alkyl, C₁₋₃ deuterated alkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3 to 8 membered heterocyclyl, C₆₋₁₀ aryl and 5 to 10 membered heteroaryl;
Rₐ is selected from the group consisting of hydrogen, deuterium, halogen, amino, cyano, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl and 4 to 6 membered heterocyclyl containing 1 to 2 atoms selected from the group consisting of N and O, the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl and 4 to 6 membered heterocyclyl containing 1 to 2 atoms selected from the group consisting of N and O are each optionally substituted by one or more substituents selected from the group consisting of deuterium, halogen, amino, hydroxy, cyano, nitro, oxo, C₁₋₃ alkyl, C₁₋₃ deuterated alkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3 to 8 membered heterocyclyl, C₆₋₁₀ aryl and 5 to 10 membered heteroaryl;
more preferably, R₁ is selected from the group consisting of: 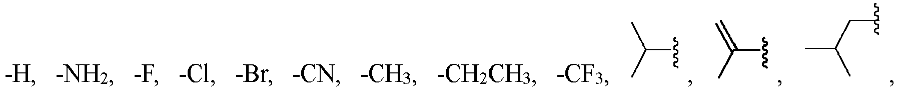 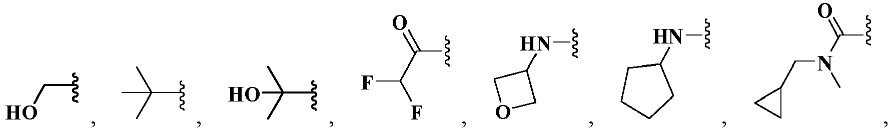 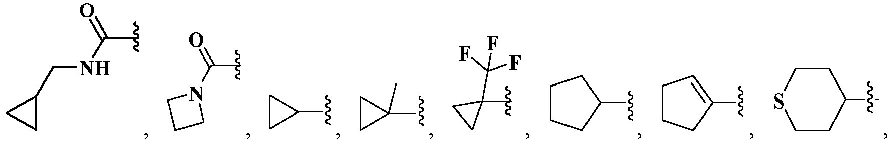 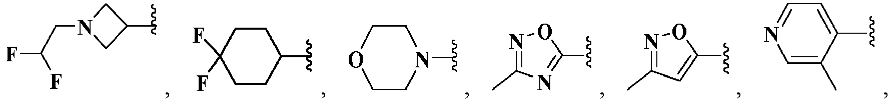 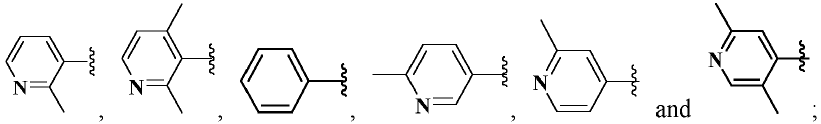
R₂ is selected from the group consisting of hydrogen, deuterium, halogen, amino, hydroxy, cyano, nitro, C₁₋₃ alkyl, C₂₋₃ alkenyl, C₂₋₃ alkynyl, C₁₋₃ deuterated alkyl, C₁₋₃ haloalkyl, C₁₋₃ alkoxy, C₁₋₃ haloalkoxy, C₁₋₃ hydroxyalkyl, C₃₋₈ cycloalkyl, 3 to 8 membered heterocyclyl, C₆₋₁₀ aryl and 5 to 10 membered heteroaryl; and preferably selected from the group consisting of hydrogen, amino, cyano, fluorine, chlorine, bromine, methyl, isopropyl, trifluoromethyl, methoxy, cyclopropyl and morpholinyl;
R₃ is selected from the group consisting of hydrogen, deuterium, halogen, amino, hydroxy, cyano, oxo, thioxo, C₁₋₃ alkyl, C₂₋₃ alkenyl, C₂₋₃ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₈ cycloalkyl, 3 to 8 membered heterocyclyl, C₆₋₁₀ aryl and 5 to 1 membered heteroaryl; and preferably selected from the group consisting of hydrogen and cyano.

In a preferred embodiment of the present invention, the acid in the acid salt is selected from the group consisting of isethionic acid, hydrochloric acid, sulfuric acid, 1,5-naphthalene disulfonic acid, methanesulfonic acid, hydrobromic acid, ethanesulfonic acid, phosphoric acid, benzenesulfonic acid, oxalic acid, maleic acid, adipic acid, hydrochloric acid, citric acid, malonic acid, L-malic acid, pamoic acid, p-toluenesulfonic acid and fumaric acid; and preferably selected from the group consisting of hydrochloric acid, sulfuric acid, methanesulfonic acid, hydrobromic acid and ethanesulfonic acid.

In a preferred embodiment of the present invention, provided is an acid salt of the compound, the number of acid is 0.2 to 3; preferably 0.2, 0.5, 1, 1.5, 2, 2.5 or 3; more preferably 0.5, 1, 2 or 3; and further preferably 1.

In a preferred embodiment of the present invention, provided is an acid salt of the compound, the acid salt is a hydrate or anhydrate; when the acid salt is a hydrate, the number of water is 0.2 to 3; preferably 0.2, 0.5, 1, 1.5, 2, 2.5 or 3; and more preferably 0.5, 1, 2 or 3.

In a preferred embodiment of the present invention, provided is an acid salt of the compound, the acid salt is a crystal form;
preferably, the crystal form is a crystal form of the acid salt of compound 2-(2-(*tert*-butyl)-5-oxopyrazolo[1,5-a]pyrido[3,2-e]pyrimidin-4(5H)-yl)-N-(5-fluoropyri din-2-yl)acetamide;
a crystal form of the acid salt of 2-(2-(*tert*-butyl)-5-oxo-8-(trifluoromethyl)pyrazolo[1,5-a]pyrido[3,2-e]pyrimidin-4(5H) -yl)-N-(5-fluoropyridin-2-yl)acetamide;
a crystal form of the acid salt of 2-(2-ethyl-5-oxo-8-(trifluoromethyl)pyrazolo[1,5-a]pyrido[3,2-e]pyrimidin-4(5H)-yl)-N -(5-fluoropyridin-2-yl)acetamide;
a crystal form of the acid salt of 2-(2-cyclopropyl-5-oxo-8-(trifluoromethyl)pyrazolo[1,5-a]pyrido[3,2-e]pyrimidin-4(5H )-yl)-N-(5-fluoropyridin-2-yl)acetamide;
a crystal form of the acid salt of 2-(2,5-dimethylpyridin-4-yl)-5-oxo-8-(trifluoromethyl)pyrazolo[1,5-a]pyrido[3,2-e]pyri midin-4(5H)-yl)-N-(5-fluoropyridin-2-yl)acetamide;
a crystal form of the acid salt of N-(5-fluoropyridin-2-yl)-2-(2-(1-methylcyclopropyl)-5-oxo-8-(trifluoromethyl)pyrazolo [1,5-a]pyrido[3,2-e]pyrimidin-4(5H)-yl)acetamide;
a crystal form of the acid salt of 2-(2-bromo-5-oxo-8-(trifluoromethyl)pyrazolo[1,5-a]pyrido[3,2-e]pyrimidin-4(5H)-yl)-N-(5-fluoropyridin-2-yl)acetamide;
a crystal form of the acid salt of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide;
a crystal form of the acid salt of N-(5-fluoropyridin-2-yl)-2-(5-oxo-8-(trifluoromethyl)pyrazolo[1,5-a]pyrido[3,2-e]pyri midin-4(5H)-yl)acetamide;
a crystal form of the acid salt of 2-(3-cyano-5-oxo-8-(trifluoromethyl)pyrazolo[1,5-a]pyrido[3,2-e]pyrimidin-4(5H)-yl)-N-(5-fluoropyridin-2-yl)acetamide;
more preferably a crystal form of isethionate, a crystal form of sulfate, a crystal form of hydrochloride, a crystal form of 1,5-naphthalene disulfonate, a crystal form of methanesulfonate, a crystal form of ethanesulfonate, a crystal form of hydrobromide, a crystal form of phosphate, a crystal form of benzenesulfonate, a crystal form of oxalate, a crystal form of maleate, a crystal form of adipate, a crystal form of hydrochloride, a crystal form of citrate, a crystal form of malonate, a crystal form of L-malate, a crystal form of pamoate, a crystal form of *p*-toluenesulfonate or a crystal form of fumarate.

In a preferred embodiment of the present invention, provided are crystal form A of methanesulfonate, crystal form A of ethanesulfonate, crystal forms A to B of sulfate, crystal forms A to B of hydrochloride and crystal forms A to C of hydrobromide of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide.

In a further preferred embodiment of the present invention, the crystal form of the acid salt of the compound is crystal form A of methanesulfonate, the X-ray powder diffraction pattern of which has a diffraction peak of 13.7±0.2°, or a diffraction peak of 21.9±0.2°, or a diffraction peak of 20.4±0.2°, or a diffraction peak of 15.4±0.2°, or a diffraction peak of 19.6±0.2°, or a diffraction peak of 16.4±0.2°, or a diffraction peak of 9.3±0.2°, or a diffraction peak of 5.3±0.2°, or a diffraction peak of 7.9±0.2°, or a diffraction peak of 11.9±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form A of methanesulfonate comprises at least one or more diffraction peaks at 2θ of 13.7±0.2°, 16.4±0.2°, 21.9±0.2°, preferably comprises 2 of the above diffraction peaks, more preferably comprises 3 of the above diffraction peaks; optionally, can further comprise at least one diffraction peak at 2θ of 13.9±0.2°, 20.4±0.2°, 15.4±0.2°, 5.3±0.2°, 11.9±0.2°, 9.3±0.2°, preferably comprises 2, 3, 4 or 5 of the above diffraction peaks;
more preferably, the X-ray powder diffraction pattern of crystal form A of methanesulfonate optionally further comprises one or more diffraction peaks at 2θ of 7.9±0.2°, 19.6±0.2°, 17.6±0.2°, 18.8±0.2°, 21.0±0.2°, 23.3±0.2°, 24.1±0.2°; preferably comprises at least any 2 to 3, or 4 to 5, or 6 to 7 of the above diffraction peaks; further preferably comprises at least any 2, 3, 4, 5, 6, 7 of the above diffraction peaks;
further preferably, the X-ray powder diffraction pattern of crystal form A of methanesulfonate comprises one or more diffraction peaks at 2θ of 5.3±0.2°, 7.9±0.2°, 9.3±0.2°, 11.9±0.2°, 13.7±0.2°, 13.9±0.2°, 15.4±0.2°, 16.4±0.2°, 17.6±0.2°, 18.8±0.2°, 19.6±0.2°, 20.4±0.2°, 21.0±0.2°, 21.9±0.2°, 23.3±0.2°, 24.1±0.2°, preferably comprises any 4, 5, 6, 8 or 10 of the above diffraction peaks;
most preferably, in a further preferred embodiment of the present invention, the characteristic X-ray diffraction peaks of crystal form A of methanesulfonate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide represented by the 2θ angle and the interplanar spacing d value by using Cu-Kα radiation are as shown in Table 1.

**Table 1**

| No. | 2θ (±0.2 °) |
|---|---|
| 1 | 5.328 |
| 2 | 7.917 |
| 3 | 9.277 |
| 4 | 11.865 |
| 5 | 13.674 |
| 6 | 13.852 |
| 7 | 15.444 |
| 8 | 16.353 |
| 9 | 17.584 |
| 10 | 18.763 |
| 11 | 19.553 |
| 12 | 20.422 |
| 13 | 20.967 |
| 14 | 21.921 |
| 15 | 23.346 |
| 16 | 24.093 |
| 17 | 24.819 |
| 18 | 25.503 |

Provided is crystal form A of methanesulfonate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide of the present invention, which has a X-ray powder diffraction pattern substantially as shown in Figure 1, a DSC spectrum substantially as shown in Figure 2, and a TGA spectrum substantially as shown in Figure 3.

In a further preferred embodiment of the present invention, the crystal form of the acid salt of the compound is crystal form A of ethanesulfonate, the X-ray powder diffraction pattern of which has a diffraction peak of 15.0±0.2°, or a diffraction peak of 21.1±0.2°, or a diffraction peak of 23.1±0.2°, or a diffraction peak of 19.8±0.2°, or a diffraction peak of 12.5±0.2°, or a diffraction peak of 9.0±0.2°, or a diffraction peak of 12.3±0.2°, or a diffraction peak of 24.6±0.2°, or a diffraction peak of 10.3±0.2°, or a diffraction peak of 6.1±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form A of ethanesulfonate comprises at least one or more diffraction peaks at 2θ of 15.0±0.2°, 21.1±0.2°, 23.1±0.2°, preferably comprises 2 of the above diffraction peaks, more preferably comprises 3 of the above diffraction peaks; optionally, can further comprise at least one diffraction peak at 2θ of 19.8±0.2°, 12.5±0.2°, 9.0±0.2°, 12.3±0.2°, 24.6±0.2°, preferably comprises 2, 3, 4 or 5 of the above diffraction peaks;
more preferably, the X-ray powder diffraction pattern of crystal form A of ethanesulfonate optionally further comprises one or more diffraction peaks at 2θ of 10.3±0.2°, 6.1±0.2°, 16.1±0.2°, 19.2±0.2°, 23.6±0.2°, 30.7±0.2°, 9.6±0.2°; preferably comprises at least any 2 to 3, or 4 to 5, or 6 to 7 of the above diffraction peaks; further preferably comprises at least any 2, 3, 4, 5, 6, 7 of the above diffraction peaks;
further preferably, the X-ray powder diffraction pattern of crystal form A of ethanesulfonate comprises one or more diffraction peaks at 2θ of 15.0±0.2°, 21.1±0.2°, 23.1±0.2°, 19.8±0.2°, 12.5±0.2°, 9.0±0.2°, 12.3±0.2°, 24.6±0.2°, 10.3±0.2°, 6.1±0.2°, 16.1±0.2°, 19.2±0.2°, 23.6±0.2°, 30.7±0.2°, 9.6±0.2°, preferably comprises any 4, 5, 6, 8 or 10 of the above diffraction peaks;
in a further preferred embodiment of the present invention, the characteristic X-ray diffraction peaks of crystal form A of ethanesulfonate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide represented by the 2θ angle and the interplanar spacing d value by using Cu-Kα radiation are as shown in Table 2.

**Table 2**

| No. | The XRPD data of crystal form A of ethanesulfonate | | | |
|---|---|---|---|---|
| | 2θ (±0.2°) | d value | Peak height | Proportion (I%) |
| 1 | 6.129 | 14.4073 | 1124 | 28.8 |
| 2 | 9.028 | 9.7876 | 1345 | 39.1 |
| 3 | 9.560 | 9.244 | 282 | 9.5 |
| 4 | 10.307 | 8.5755 | 1123 | 32.6 |
| 5 | 12.270 | 7.2073 | 752 | 35.4 |
| 6 | 12.451 | 7.1031 | 1284 | 41.3 |
| 7 | 14.967 | 5.9142 | 3354 | 100 |
| 8 | 16.062 | 5.5133 | 728 | 26.4 |
| 9 | 16.268 | 5.4441 | 134 | 7.4 |
| 10 | 19.204 | 4.6179 | 678 | 25.1 |
| 11 | 19.774 | 4.486 | 984 | 45.1 |
| 12 | 21.068 | 4.2133 | 1619 | 61.2 |
| 13 | 23.058 | 3.854 | 1225 | 50.7 |
| 14 | 23.565 | 3.7723 | 418 | 18.5 |
| 15 | 24.639 | 3.6101 | 875 | 34.5 |
| 16 | 28.634 | 3.1149 | 115 | 8.1 |
| 17 | 30.716 | 2.9084 | 96 | 12.5 |
| 18 | 32.927 | 2.7179 | 119 | 8.2 |
| 19 | 34.247 | 2.6161 | 178 | 8.5 |
| 20 | 39.233 | 2.2944 | 57 | 6.2 |

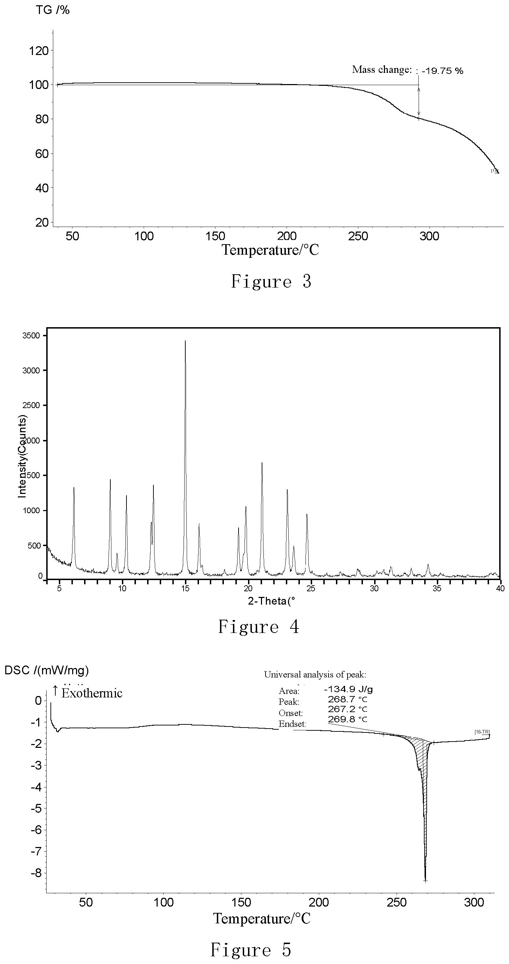

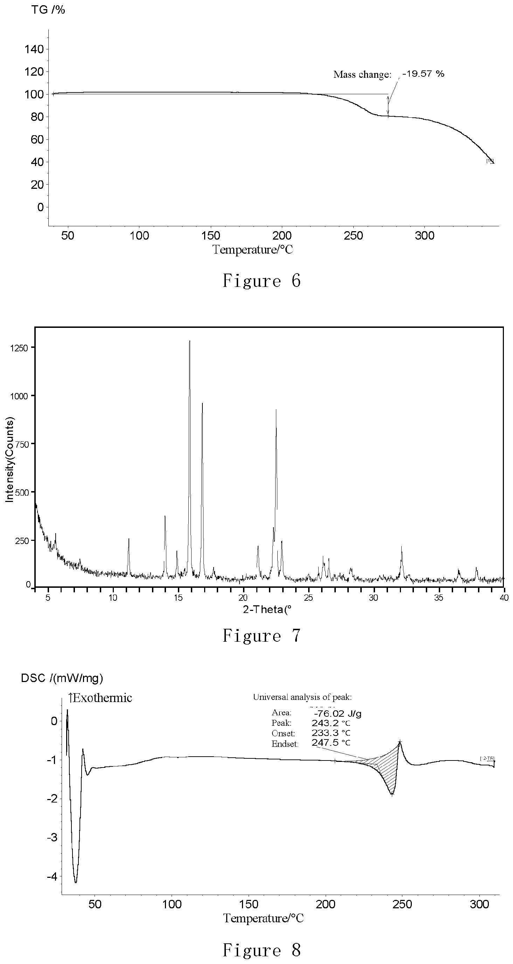

Provided is crystal form A of ethanesulfonate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide of the present invention, which has a X-ray powder diffraction pattern substantially as shown in Figure 4, a DSC spectrum substantially as shown in Figure 5, and a TGA spectrum substantially as shown in Figure 6.

In a further preferred embodiment of the present invention, the acid salt of the compound is crystal form A of sulfate, the X-ray powder diffraction pattern of which has a diffraction peak of 22.5±0.2°, or a diffraction peak of 15.9±0.2°, or a diffraction peak of 22.3±0.2°, or a diffraction peak of 16.8±0.2°, or a diffraction peak of 22.9±0.2°, or a diffraction peak of 32.1±0.2°, or a diffraction peak of 14.0±0.2°, or a diffraction peak of 21.1±0.2°, or a diffraction peak of 11.2±0.2°, or a diffraction peak of 26.1±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form A of sulfate comprises at least one or more diffraction peaks at 2θ of 22.5±0.2°, 15.9±0.2°, 22.3±0.2°, preferably comprises 2 of the above diffraction peaks, more preferably comprises 3 of the above diffraction peaks; optionally, can further comprise at least one diffraction peak at 2θ of 16.8±0.2°, 22.9±0.2°, 32.1±0.2°, 14.0±0.2°, 21.1±0.2°, preferably comprises 2, 3, 4 or 5 of the above diffraction peaks;
more preferably, or, the X-ray powder diffraction pattern of crystal form A of sulfate optionally further comprises one or more diffraction peaks at 2θ of 11.2±0.2°, 26.1±0.2°, 28.2±0.2°, 37.8±0.2°, 15.5±0.2°, 26.5±0.2°, 36.4±0.2°; preferably comprises at least any 2 to 3, or 4 to 5, or 6 to 7 of the above diffraction peaks; further preferably comprises at least any 2, 3, 4, 5, 6, 7 of the above diffraction peaks;
further preferably, the X-ray powder diffraction pattern of crystal form A of sulfate comprises one or more diffraction peaks at 2θ of 22.5±0.2°, 15.9±0.2°, 22.3±0.2°, 16.8±0.2°, 22.9±0.2°, 32.1±0.2°, 14.0±0.2°, 21.1±0.2°, 11.2±0.2°, 26.1±0.2°, 28.2±0.2°, 37.8±0.2°, 15.5±0.2°, 26.5±0.2°, 36.4±0.2°, preferably comprises any 4, 5, 6, 8 or 10 of the above diffraction peaks;
in a further preferred embodiment of the present invention, the characteristic X-ray diffraction peaks of crystal form A of sulfate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide represented by the 2θ angle and the interplanar spacing d value by using Cu-Kα radiation are as shown in Table 3.

**Table 3**

| No. | The XRPD data of crystal form A of sulfate | | | |
|---|---|---|---|---|
| | 2θ (±0.2 °) | d value | Peak height | Proportion (I%) |
| 1 | 5.581 | 15.8229 | 99 | 4.8 |
| 2 | 7.431 | 11.8866 | 51 | 3.9 |
| 3 | 11.190 | 7.9003 | 193 | 12.8 |
| 4 | 13.977 | 6.3307 | 318 | 23.8 |
| 5 | 14.892 | 5.9439 | 131 | 6.8 |
| 6 | 15.473 | 5.7221 | 43 | 8.7 |
| 7 | 15.857 | 5.5842 | 1226 | 96.8 |
| 8 | 16.812 | 5.2691 | 908 | 67.7 |
| 9 | 17.678 | 5.013 | 57 | 5.2 |
| 10 | 20.197 | 4.3929 | 29 | 1.6 |
| 11 | 21.108 | 4.2055 | 171 | 19.1 |
| 12 | 22.269 | 3.9887 | 265 | 75.2 |
| 13 | 22.471 | 3.9534 | 881 | 100 |
| 14 | 22.931 | 3.8751 | 204 | 27.9 |
| 15 | 24.982 | 3.5614 | 34 | 4.7 |
| 16 | 25.734 | 3.459 | 64 | 1.7 |
| 17 | 26.081 | 3.4138 | 117 | 12.3 |
| 18 | 26.523 | 3.3578 | 105 | 7.8 |
| 19 | 28.187 | 3.1633 | 61 | 11 |
| 20 | 30.417 | 2.9363 | 26 | 4.2 |
| 21 | 30.728 | 2.9072 | 30 | 6.8 |
| 22 | 32.103 | 2.7858 | 179 | 26.5 |
| 23 | 32.695 | 2.7367 | 28 | 2.7 |
| 24 | 36.443 | 2.4634 | 65 | 7.8 |
| 25 | 37.836 | 2.3758 | 71 | 8.9 |

Provided is crystal form A of sulfate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide of the present invention, which has a X-ray powder diffraction pattern substantially as shown in Figure 7, and a DSC spectrum substantially as shown in Figure 8.

In a further preferred embodiment of the present invention, the acid salt of the compound is crystal form B of sulfate, the X-ray powder diffraction pattern of which has a diffraction peak of 15.3±0.2°, or a diffraction peak of 21.5±0.2°, or a diffraction peak of 10.6±0.2°, or a diffraction peak of 19.8±0.2°, or a diffraction peak of 20. 1±0.2°, or a diffraction peak of 12.6±0.2°, or a diffraction peak of 25.2±0.2°, or a diffraction peak of 9.2±0.2°, or a diffraction peak of 9.9±0.2°, or a diffraction peak of 23.4±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form B of sulfate comprises at least one or more diffraction peaks at 2θ of 15.3±0.2°, 21.5±0.2°, 10.6±0.2°, preferably comprises 2 of the above diffraction peaks, more preferably comprises 3 of the above diffraction peaks; optionally, can further comprise at least one diffraction peak at 2θ of 19.8±0.2°, 20.1±0.2°, 12.6±0.2°, 25.2±0.2°, 9.2±0.2°, preferably comprises 2, 3, 4 or 5 of the above diffraction peaks;
more preferably, the X-ray powder diffraction pattern of crystal form B of sulfate optionally further comprises one or more diffraction peaks at 2θ of 9.9±0.2°, 23.4±0.2°, 6.3±0.2°, 16.7±0.2°, 23.9±0.2°, 33.8±0.2°, 16.3±0.2°; preferably comprises at least any 2 to 3, or 4 to 5, or 6 to 7 of the above diffraction peaks; further preferably comprises at least any 2, 3, 4, 5, 6, 7 of the above diffraction peaks;
further preferably, the X-ray powder diffraction pattern of crystal form B of sulfate comprises one or more diffraction peaks at 2θ of 15.3±0.2°, 21.5±0.2°, 10.6±0.2°, 19.8±0.2°, 20.1±0.2°, 12.6±0.2°, 25.2±0.2°, 9.2±0.2°, 9.9±0.2°, 23.4±0.2°, 6.3±0.2°, 16.7±0.2°, 23.9±0.2°, 33.8±0.2°, 16.3±0.2°, preferably comprises any 4, 5, 6, 8 or 10 of the above diffraction peaks;
in a further preferred embodiment of the present invention, the characteristic X-ray diffraction peaks of crystal form B of sulfate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide represented by the 2θ angle and the interplanar spacing d value by using Cu-Kα radiation are as shown in Table 4.

**Table 4**

| No. | The XRPD data of crystal form B of sulfate | | | |
|---|---|---|---|---|
| | 2θ (±0.2°) | d value | Peak height | Proportion (I%) |
| 1 | 6.288 | 14.044 | 758 | 14.5 |
| 2 | 9.209 | 9.5948 | 1484 | 29 |
| 3 | 9.875 | 8.9493 | 1078 | 22.2 |
| 4 | 10.607 | 8.3338 | 3523 | 74.8 |
| 5 | 12.578 | 7.032 | 1022 | 34.8 |
| 6 | 15.275 | 5.7959 | 4356 | 100 |
| 7 | 16.327 | 5.4246 | 292 | 6.5 |
| 8 | 16.731 | 5.2944 | 464 | 11.6 |
| 9 | 19.773 | 4.4863 | 2060 | 59.8 |
| 10 | 20.137 | 4.4061 | 1266 | 43.3 |
| 11 | 21.066 | 4.2137 | 84 | 5 |
| 12 | 21.519 | 4.1261 | 3100 | 86.6 |
| 13 | 23.421 | 3.7951 | 703 | 20.1 |
| 14 | 23.931 | 3.7153 | 261 | 10 |
| 15 | 25.230 | 3.527 | 1015 | 30.9 |
| 16 | 28.972 | 3.0794 | 102 | 5.6 |
| 17 | 30.801 | 2.9005 | 114 | 5.7 |
| 18 | 32.121 | 2.7843 | 81 | 5.4 |
| 19 | 33.820 | 2.6482 | 216 | 8 |
| 20 | 34.838 | 2.5731 | 126 | 4.5 |

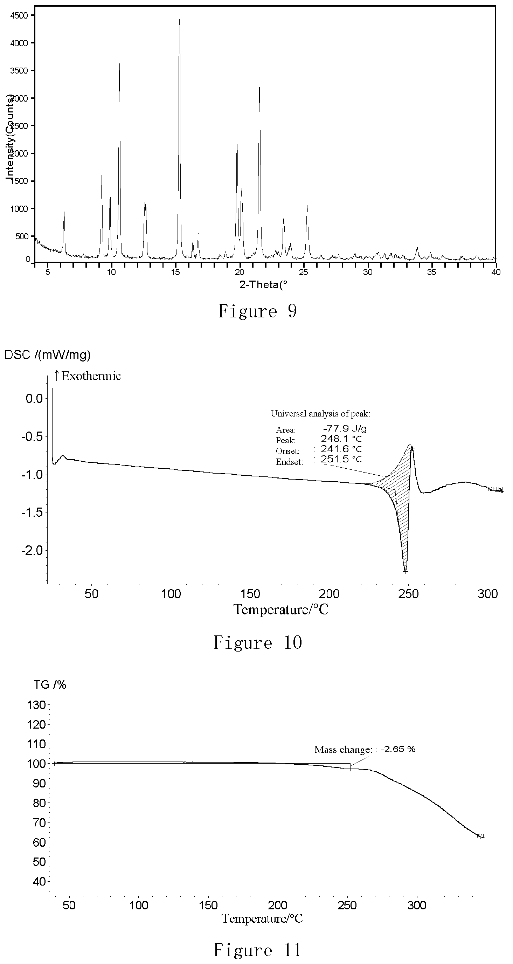

Provided is crystal form B of sulfate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide of the present invention, which has a X-ray powder diffraction pattern substantially as shown in Figure 9, a DSC spectrum substantially as shown in Figure 10, and a TGA spectrum substantially as shown in Figure 11.

In a further preferred embodiment of the present invention, the acid salt of the compound is crystal form A of hydrochloride, the X-ray powder diffraction pattern of which has a diffraction peak of 15.0±0.2°, or a diffraction peak of 23.9±0.2°, or a diffraction peak of 9.7±0.2°, or a diffraction peak of 5.3±0.2°, or a diffraction peak of 24.8±0.2°, or a diffraction peak of 29.5±0.2°, or a diffraction peak of 7.5±0.2°, or a diffraction peak of 21.8±0.2°, or a diffraction peak of 21.3±0.2°, or a diffraction peak of 10.6±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form A of hydrochloride comprises at least one or more diffraction peaks at 2θ of 15.0±0.2°, 23.9±0.2°, 9.7±0.2°, preferably comprises 2 of the above diffraction peaks, more preferably comprises 3 of the above diffraction peaks; optionally, can further comprise at least one diffraction peak at 2θ of 5.3±0.2°, 24.8±0.2°, 29.5±0.2°, 7.5±0.2°, 21.8±0.2°, preferably comprises 2, 3, 4 or 5 of the above diffraction peaks;
more preferably, the X-ray powder diffraction pattern of crystal form A of hydrochloride optionally further comprises one or more diffraction peaks at 2θ of 21.3±0.2°, 10.6±0.2°, 16.9±0.2°, 16.0±0.2°, 18.4±0.2°, 25.8±0.2°, 28.4±0.2°; preferably comprises at least any 2 to 3, or 4 to 5, or 6 to 7 of the above diffraction peaks; further preferably comprises at least any 2, 3, 4, 5, 6, 7 of the above diffraction peaks;
further preferably, the X-ray powder diffraction pattern of crystal form A of hydrochloride comprises one or more diffraction peaks at 2θ of 15.0±0.2°, 23.9±0.2°, 9.7±0.2°, 5.3±0.2°, 24.8±0.2°, 29.5±0.2°, 7.5±0.2°, 21.8±0.2°, 21.3±0.2°, 10.6±0.2°, 16.9±0.2°, 16.0±0.2°, 18.4±0.2°, 25.8±0.2°, 28.4±0.2°, preferably comprises any 4, 5, 6, 8 or 10 of the above diffraction peaks;
in a further preferred embodiment of the present invention, the characteristic X-ray diffraction peaks of crystal form A of hydrochloride of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide represented by the 2θ angle and the interplanar spacing d value by using Cu-Kα radiation are as shown in Table 5.

**Table 5**

| No. | The XRPD data of crystal form A of hydrochloride | | | |
|---|---|---|---|---|
| | 2θ (±0.2 °) | d value | Peak height | Proportion (I%) |
| 1 | 5.317 | 16.6057 | 688 | 24.7 |
| 2 | 7.507 | 11.7668 | 309 | 13.3 |
| 3 | 8.699 | 10.1564 | 97 | 3 |
| 4 | 9.659 | 9.1492 | 503 | 28.1 |
| 5 | 10.629 | 8.3162 | 227 | 9.8 |
| 6 | 15.048 | 5.8825 | 1626 | 100 |
| 7 | 16.020 | 5.5279 | 200 | 7.8 |
| 8 | 16.508 | 5.3654 | 70 | 3.4 |
| 9 | 16.856 | 5.2553 | 133 | 8.2 |
| 10 | 18.433 | 4.8092 | 93 | 7.1 |
| 11 | 21.335 | 4.1612 | 136 | 10.1 |
| 12 | 21.840 | 4.0662 | 151 | 11.1 |
| 13 | 22.656 | 3.9215 | 67 | 3.3 |
| 14 | 23.929 | 3.7157 | 473 | 39 |
| 15 | 24.781 | 3.5898 | 320 | 17.3 |
| 16 | 25.838 | 3.4453 | 109 | 6.7 |
| 17 | 26.878 | 3.3144 | 33 | 1.5 |
| 18 | 28.351 | 3.1454 | 129 | 6.4 |
| 19 | 29.462 | 3.0292 | 239 | 13.6 |
| 20 | 31.525 | 2.8356 | 34 | 2.9 |
| 21 | 32.415 | 2.7597 | 48 | 3.6 |
| 22 | 33.272 | 2.6905 | 70 | 4.6 |
| 23 | 34.820 | 2.5744 | 42 | 3.5 |
| 24 | 37.573 | 2.3919 | 43 | 3.7 |

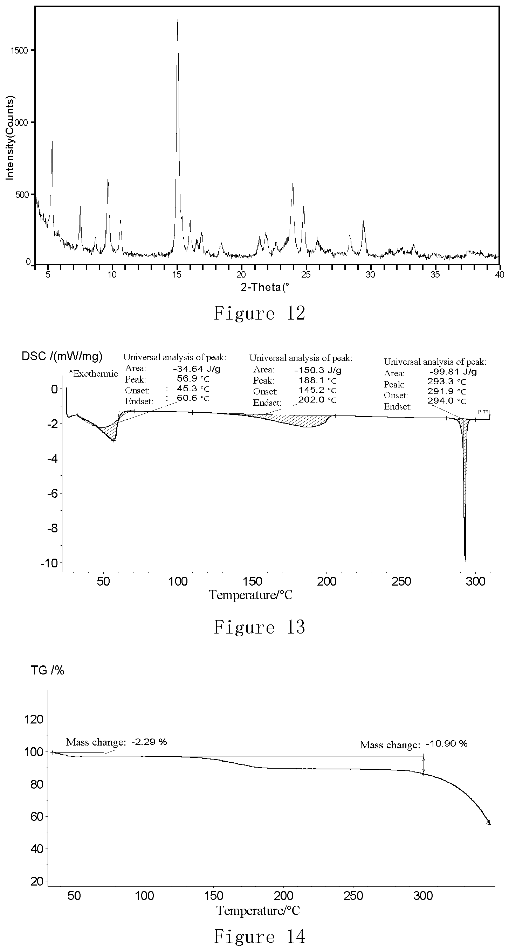

Provided is crystal form A of hydrochloride of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide of the present invention, which has a X-ray powder diffraction pattern substantially as shown in Figure 12, a DSC spectrum substantially as shown in Figure 13, and a TGA spectrum substantially as shown in Figure 14.

In a further preferred embodiment of the present invention, the acid salt of the compound is crystal form B of hydrochloride, the X-ray powder diffraction pattern of crystal form B of hydrochloride has a diffraction peak of 15.9±0.2°, or a diffraction peak of 22.2±0.2°, or a diffraction peak of 5.2±0.2°, or a diffraction peak of 21.7±0.2°, or a diffraction peak of 26.0±0.2°, or a diffraction peak of 4.6±0.2°, or a diffraction peak of 28.4±0.2°, or a diffraction peak of 9.2±0.2°, or a diffraction peak of 17.3±0.2°, or a diffraction peak of 15.2±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form B of hydrochloride comprises at least one or more diffraction peaks at 2θ of 15.9±0.2°, 22.2±0.2°, 5.2±0.2°, preferably comprises 2 of the above diffraction peaks, more preferably comprises 3 of the above diffraction peaks; optionally, can further comprise at least one diffraction peak at 2θ of 21.7±0.2°, 26.0±0.2°, 4.6±0.2°, 28.4±0.2°, 9.2±0.2°, preferably comprises 2, 3, 4 or 5 of the above diffraction peaks;
more preferably, or, the X-ray powder diffraction pattern of crystal form B of hydrochloride optionally further comprises one or more diffraction peaks at 2θ of 17.3±0.2°, 15.2±0.2°, 10.5±0.2°, 38.0±0.2°, 20.3±0.2°, 23.8±0.2°, 29.5±0.2°; preferably comprises at least any 2 to 3, or 4 to 5, or 6 to 7 of the above diffraction peaks; further preferably comprises at least any 2, 3, 4, 5, 6, 7 of the above diffraction peaks;
further preferably, the X-ray powder diffraction pattern of crystal form B of hydrochloride comprises one or more diffraction peaks at 2θ of 15.9±0.2°, 22.2±0.2°, 5.2±0.2°, 21.7±0.2°, 26.0±0.2°, 4.6±0.2°, 28.4±0.2°, 9.2±0.2°, 17.3±0.2°, 15.2±0.2°, 10.5±0.2°, 38.0±0.2°, 20.3±0.2°, 23.8±0.2°, 29.5±0.2°, preferably comprises any 4, 5, 6, 8 or 10 of the above diffraction peaks;
in a further preferred embodiment of the present invention, the characteristic X-ray diffraction peaks of hydrochloride crystal form B of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide represented by the 2θ angle and the interplanar spacing d value by using Cu-Kα radiation are as shown in Table 6.

**Table 6**

| No. | The XRPD data of crystal form B of hydrochloride | | | |
|---|---|---|---|---|
| | 2θ (±0.2°) | d value | Peak height | Proportion (I%) |
| 1 | 4.6 | 19.132 | 65 | 46.9 |
| 2 | 5.2 | 16.9207 | 267 | 83.9 |
| 3 | 7.6 | 11.6707 | 62 | 8.7 |
| 4 | 9.2 | 9.5956 | 41 | 31 |
| 5 | 10.5 | 8.3823 | 76 | 29.9 |
| 6 | 15.2 | 5.8133 | 68 | 30.4 |
| 7 | 15.9 | 5.5841 | 125 | 100 |
| 8 | 17.3 | 5.1157 | 61 | 31 |
| 9 | 20.3 | 4.3678 | 38 | 18 |
| 10 | 21.7 | 4.0924 | 53 | 72.1 |
| 11 | 22.2 | 3.9966 | 121 | 84.8 |
| 12 | 23.8 | 3.7416 | 34 | 12.8 |
| 13 | 26.0 | 3.4251 | 73 | 67.6 |
| 14 | 28.4 | 3.1369 | 85 | 33.5 |
| 15 | 29.5 | 3.0275 | 57 | 11.7 |
| 16 | 32.5 | 2.7495 | 32 | 10 |
| 17 | 38.0 | 2.3685 | 42 | 28.1 |

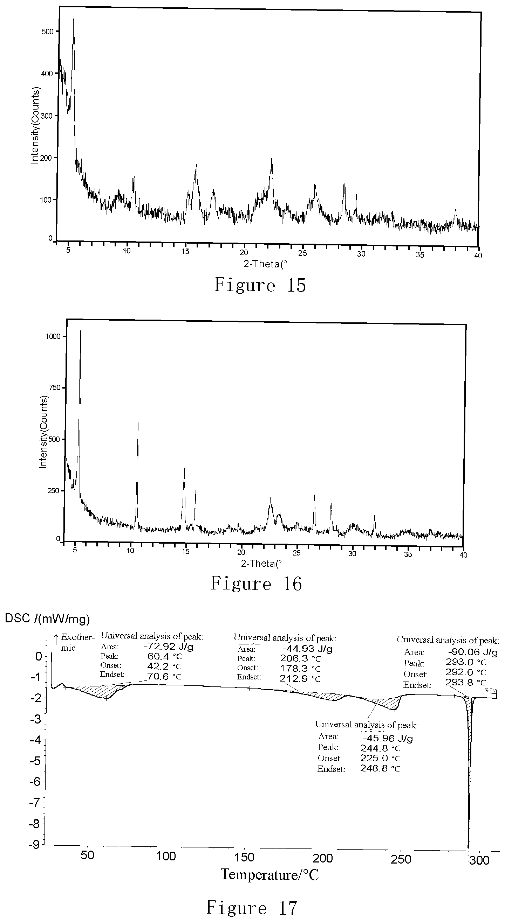

Provided is crystal form B of hydrochloride of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide of the present invention, which has a X-ray powder diffraction pattern substantially as shown in Figure 15.

In a further preferred embodiment of the present invention, the crystal form of the acid salt of the compound is crystal form A of hydrobromide, the X-ray powder diffraction pattern of which has a diffraction peak of 5.3±0.2°, or a diffraction peak of 22.7±0.2°, or a diffraction peak of 14.8±0.2°, or a diffraction peak of 10.5±0.2°, or a diffraction peak of 22.5±0.2°, or a diffraction peak of 28.0±0.2°, or a diffraction peak of 30.0±0.2°, or a diffraction peak of 23.4±0.2°, or a diffraction peak of 23.3±0.2°, or a diffraction peak of 26.5±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form A of hydrobromide comprises at least one or more diffraction peaks at 2θ of 5.3±0.2°, 22.7±0.2°, 14.8±0.2°, preferably comprises 2 of the above diffraction peaks, more preferably comprises 3 of the above diffraction peaks; optionally, can further comprise at least one diffraction peak at 2θ of 10.5±0.2°, 22.5±0.2°, 28.0±0.2°, 30.0±0.2°, 23.4±0.2°, preferably comprises 2, 3, 4 or 5 of the above diffraction peaks;
more preferably, the X-ray powder diffraction pattern of crystal form A of hydrobromide optionally further comprises one or more diffraction peaks at 2θ of 23.3±0.2°, 26.5±0.2°, 34.9±0.2°, 15.8±0.2°, 25.0±0.2°, 31.9±0.2°, 37.0±0.2°; preferably comprises at least any 2 to 3, or 4 to 5, or 6 to 7 of the above diffraction peaks; further preferably comprises any 2, 3, 4, 5, 6, 7 of the above diffraction peaks;
further preferably, or, the X-ray powder diffraction pattern of crystal form A of hydrobromide comprises one or more diffraction peaks at 2θ of 5.3±0.2°, 22.7±0.2°, 14.8±0.2°, 10.5±0.2°, 22.5±0.2°, 28.0±0.2°, 30.0±0.2°, 23.4±0.2°, 23.3±0.2°, 26.5±0.2°, 34.9±0.2°, 15.8±0.2°, 25.0±0.2°, 31.9±9.2°, 37.0±0.2°, preferably comprises any 4, 5, 6, 8 or 10 of the above diffraction peaks;
in a further preferred embodiment of the present invention, the characteristic X-ray diffraction peaks of crystal form A of hydrobromide of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide represented by the 2θ angle and the interplanar spacing d value by using Cu-Kα radiation are as shown in Table 7.

**Table 7**

| No. | The XRPD data of crystal form A of hydrobromide | | | |
|---|---|---|---|---|
| | 2θ (±0.2 °) | d value | Peak height | Proportion (I%) |
| 1 | 5.3 | 16.7947 | 798 | 100 |
| 2 | 10.5 | 8.3973 | 517 | 60.1 |
| 3 | 14.8 | 5.9951 | 296 | 64 |
| 4 | 15.8 | 5.5982 | 182 | 21.2 |
| 5 | 19.7 | 4.5039 | 36 | 3.7 |
| 6 | 22.5 | 3.9433 | 133 | 44.7 |
| 7 | 22.7 | 3.9056 | 119 | 77.7 |
| 8 | 23.3 | 3.8178 | 52 | 23.6 |
| 9 | 23.4 | 3.7915 | 67 | 23.9 |
| 10 | 25.0 | 3.5552 | 39 | 18 |
| 11 | 26.5 | 3.3626 | 175 | 23.4 |
| 12 | 28.0 | 3.1859 | 145 | 31.6 |
| 13 | 28.5 | 3.1312 | 36 | 5.5 |
| 14 | 30.0 | 2.9733 | 43 | 25.7 |
| 15 | 31.9 | 2.8001 | 95 | 12.6 |
| 16 | 34.9 | 2.5661 | 36 | 22.4 |
| 17 | 37.0 | 2.4297 | 31 | 6.9 |

Provided is crystal form A of hydrobromide of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide of the present invention, which has a X-ray powder diffraction pattern substantially as shown in Figure 16, and a DSC spectrum substantially as shown in Figure 17.

In a further preferred embodiment of the present invention, the acid salt of the compound is crystal form B of hydrobromide, the X-ray powder diffraction pattern of which has a diffraction peak of 23.4±0.2°, or a diffraction peak of 15.9±0.2°, or a diffraction peak of 16.2±0.2°, or a diffraction peak of 14.2±0.2°, or a diffraction peak of 5.3±0.2°, or a diffraction peak of 10.6±0.2°, or a diffraction peak of 23.1±0.2°, or a diffraction peak of 24.1±0.2°, or a diffraction peak of 14.8±0.2°, or a diffraction peak of 9.5±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, or, the X-ray powder diffraction pattern of crystal form B of hydrobromide comprises at least one or more diffraction peaks at 2θ of 23.4±0.2°, 15.9±0.2°, 16.2±0.2°, preferably comprises 2 of the above diffraction peaks, more preferably comprises 3 of the above diffraction peaks; optionally, can further comprise at least one diffraction peak at 2θ of 14.2±0.2°, 5.3±0.2°, 10.6±0.2°, 23.1±0.2°, 24.1±0.2°, preferably comprises 2, 3, 4 or 5 of the above diffraction peaks;
more preferably, the X-ray powder diffraction pattern of crystal form B of hydrobromide optionally further comprises one or more diffraction peaks at 2θ of 14.8±0.2°, 9.5±0.2°, 16.9±0.2°, 13.9±0.2°, 29.5±0.2°, 32.2±0.2°, 22.2±0.2°; preferably comprises at least any 2 to 3, or 4 to 5, or 6 to 7 of the above diffraction peaks; further preferably comprises any 2, 3, 4, 5, 6, 7 of the above diffraction peaks;
further preferably, the X-ray powder diffraction pattern of crystal form B of hydrobromide comprises one or more diffraction peaks at 2θ of 23.4±0.2°, 15.9±0.2°, 16.2±0.2°, 14.2±0.2°, 5.3±0.2°, 10.6±0.2°, 23.1±0.2°, 24.1±0.2°, 14.8±0.2°, 9.5±0.2°, 16.9±0.2°, 13.9±0.2°, 29.5±0.2°, 32.2±0.2°, 22.2±0.2°, preferably comprises any 4, 5, 6, 8 or 10 of the above diffraction peaks;
in a further preferred embodiment of the present invention, the characteristic X-ray diffraction peaks of crystal form B of hydrobromide of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide represented by the 2θ angle and the interplanar spacing d value by using Cu-Kα radiation are as shown in Table 8.

**Table 8**

| No. | The XRPD data of crystal form B of hydrobromide | | | |
|---|---|---|---|---|
| | 2θ (±0.2 °) | d value | Peak height | Proportion (I%) |
| 1 | 5.316 | 16.611 | 736 | 61.7 |
| 2 | 9.492 | 9.3098 | 229 | 34.6 |
| 3 | 10.607 | 8.3333 | 548 | 55.8 |
| 4 | 13.854 | 6.3867 | 116 | 26.9 |
| 5 | 14.199 | 6.2325 | 246 | 72.2 |
| 6 | 14.805 | 5.9785 | 474 | 49.6 |
| 7 | 15.899 | 5.5695 | 532 | 99.5 |
| 8 | 16.184 | 5.4722 | 207 | 87.7 |
| 9 | 16.936 | 5.2308 | 294 | 29.7 |
| 10 | 18.012 | 4.9206 | 140 | 19 |
| 11 | 19.751 | 4.4912 | 78 | 19.4 |
| 12 | 21.883 | 4.0583 | 84 | 21.3 |
| 13 | 22.208 | 3.9995 | 101 | 24.7 |
| 14 | 23.078 | 3.8507 | 221 | 53.4 |
| 15 | 23.401 | 3.7983 | 316 | 100 |
| 16 | 24.128 | 3.6854 | 317 | 50.7 |
| 17 | 24.858 | 3.5788 | 173 | 18.5 |
| 18 | 29.504 | 3.0251 | 181 | 25.7 |
| 19 | 31.800 | 2.8116 | 51 | 19 |
| 20 | 32.201 | 2.7775 | 72 | 25.3 |

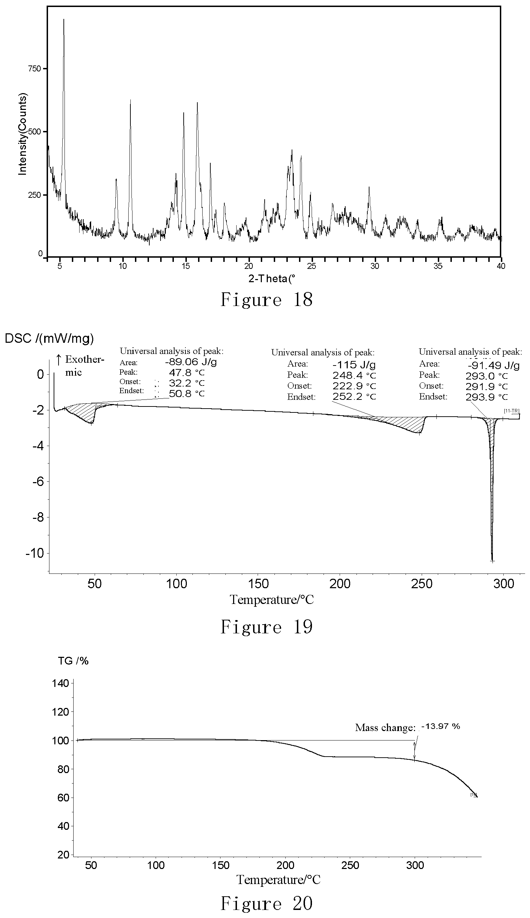

Provided is crystal form B of hydrobromide of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide of the present invention, which has a X-ray powder diffraction pattern substantially as shown in Figure 18, a DSC spectrum substantially as shown in Figure 19, and a TGA spectrum substantially as shown in Figure 20.

In a preferred embodiment of the present invention, the acid salt of the compound is crystal form C of hydrobromide, the X-ray powder diffraction pattern of which has a diffraction peak of 5.2±0.2°, or a diffraction peak of 15.7±0.2°, or a diffraction peak of 22.3±0.2°, or a diffraction peak of 10.5±0.2°, or a diffraction peak of 17.4±0.2°, or a diffraction peak of 38.0±0.2°, or a diffraction peak of 26.3±0.2°, or a diffraction peak of 28.0±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form C of hydrobromide comprises at least one or more diffraction peaks at 2θ of 5.2±0.2°, 15.7±0.2°, 22.3±0.2°, preferably comprises 2 of the above diffraction peaks, more preferably comprises 3 of the above diffraction peaks; optionally, can further comprise at least one diffraction peak at 2θ of 10.5±0.2°, 17.4±0.2°, 38.0±0.2°, 26.3±0.2°, 28.0±0.2°, preferably comprises 2, 3, 4 or 5 of the above diffraction peaks;
more preferably, the X-ray powder diffraction pattern of crystal form C of hydrobromide comprises one or more diffraction peaks at 2θ of 5.2±0.2°, 15.7±0.2°, 22.3±0.2°, 10.5±0.2°, 17.4±0.2°, 38.0±0.2°, 26.3±0.2°, 28.0±0.2°, preferably comprises any 4, 5, 6 or 8 of the above diffraction peaks;
in a further preferred embodiment of the present invention, the characteristic X-ray diffraction peaks of crystal form C of hydrobromide of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide represented by the 2θ angle and the interplanar spacing d value by using Cu-Kα radiation are as shown in Table 9.

**Table 9**

| No. | The XRPD data of crystal form C of hydrobromide | | | |
|---|---|---|---|---|
| | 2θ (±0.2°) | d value | Peak height | Proportion (I%) |
| 1 | 5.235 | 16.8674 | 643 | 100 |
| 2 | 10.488 | 8.4278 | 348 | 36.9 |
| 3 | 15.718 | 5.6332 | 408 | 60.2 |
| 4 | 17.422 | 5.0861 | 221 | 33.3 |
| 5 | 22.328 | 3.9783 | 260 | 44.4 |
| 6 | 26.32 | 3.3833 | 86 | 12.1 |
| 7 | 28.045 | 3.179 | 53 | 4.1 |
| 8 | 38.04 | 2.3636 | 120 | 16.2 |

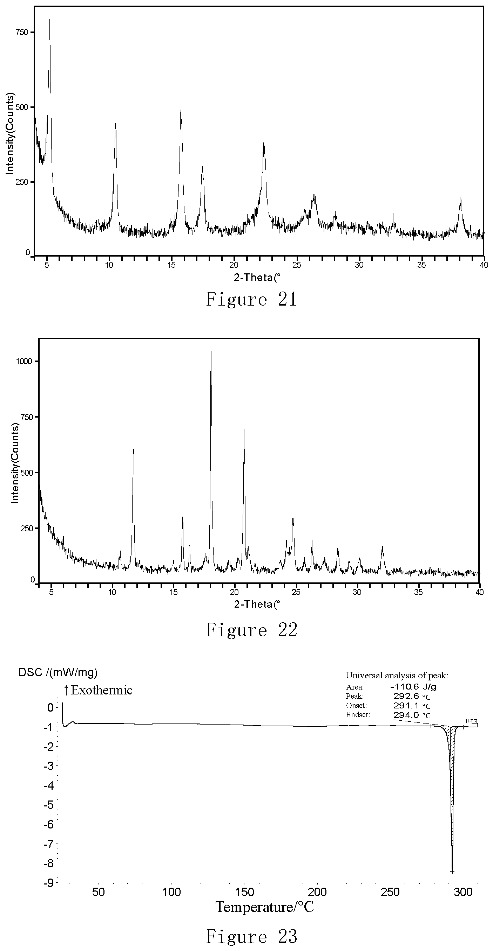

Provided is crystal form C of hydrobromide of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide of the present invention, which has a X-ray powder diffraction pattern substantially as shown in Figure 21.

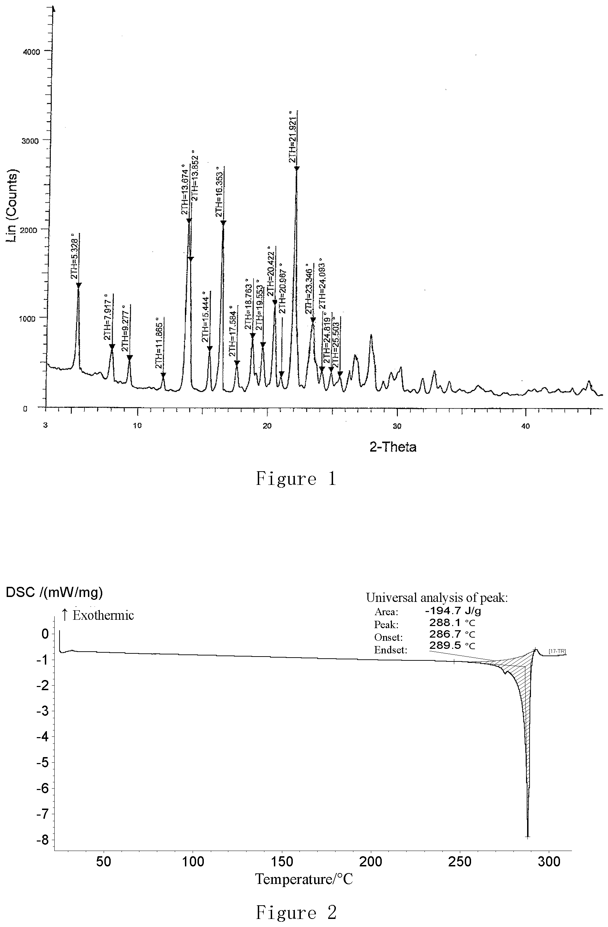

In a further preferred embodiment of the present invention, the positions of diffraction peaks with relative peak intensity of top ten in the X-ray powder diffraction pattern of the crystal form of acid salt of the compound have a 2θ error of ±0.2° to ±0.5°, preferably ±0.2° to ±0.3°, most preferably ±0.2° relative to the diffraction peaks at the corresponding positions in figure 1.

In a further preferred embodiment of the present invention, the positions of diffraction peaks with relative peak intensity of top ten in the X-ray powder diffraction pattern of crystal form A of methanesulfonate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide have a 2θ error of ±0.2° to ±0.5°, preferably ±0.2° to ±0.3°, most preferably ±0.2° relative to the diffraction peaks at the corresponding positions in figure 1;
or, positions of diffraction peaks with relative peak intensity of top ten in the X-ray powder diffraction pattern of crystal form A of ethanesulfonate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide have a 2θ error of ±0.2° to ±0.5°, preferably ±0.2° to ±0.3°, most preferably ±0.2° relative to the diffraction peaks at the corresponding positions in figure 4;
or, positions of diffraction peaks with relative peak intensity of top ten in the X-ray powder diffraction pattern of crystal form A of sulfate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide have a 2θ error of ±0.2° to ±0.5°, preferably ±0.2° to ±0.3°, most preferably ±0.2° relative to the diffraction peaks at the corresponding positions in figure 7;
or, positions of diffraction peaks with relative peak intensity of top ten in the X-ray powder diffraction pattern of crystal form B of sulfate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide have a 2θ error of ±0.2° to ±0.5°, preferably ±0.2° to ±0.3°, most preferably ±0.2° relative to the diffraction peaks at the corresponding positions in figure 9;
or, positions of diffraction peaks with relative peak intensity of top ten in the X-ray powder diffraction pattern of crystal form A of hydrochloride of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide have a 2θ error of ±0.2° to ±0.5°, preferably ±0.2° to ±0.3°, most preferably ±0.2° relative to the diffraction peaks at the corresponding positions in figure 12;
or, positions of diffraction peaks with relative peak intensity of top ten in the X-ray powder diffraction pattern of crystal form B of hydrochloride of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide have a 2θ error of ±0.2° to ±0.5°, preferably ±0.2° to ±0.3°, most preferably ±0.2° relative to the diffraction peaks at the corresponding positions in figure 15;
or, positions of diffraction peaks with relative peak intensity of top ten in the X-ray powder diffraction pattern of crystal form A of hydrobromide of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide have a 2θ error of ±0.2° to ±0.5°, preferably ±0.2° to ±0.3°, most preferably ±0.2° relative to the diffraction peaks at the corresponding positions in figure 16;
or, positions of diffraction peaks with relative peak intensity of top ten in the X-ray powder diffraction pattern of crystal form B of hydrobromide of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide have a 2θ error of ±0.2° to ±0.5°, preferably ±0.2° to ±0.3°, most preferably ±0.2° relative to the diffraction peaks at the corresponding positions in figure 18;
or, positions of diffraction peaks with relative peak intensity of top ten in the X-ray powder diffraction pattern of hydrobromide crystal form C of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide have a 2θ error of ±0.2° to ±0.5°, preferably ±0.2° to ±0.3°, most preferably ±0.2° relative to the diffraction peaks at the corresponding positions in figure 21.

In a further preferred embodiment of the present invention, the crystal form of acid salt of the above compound is a hydrate or anhydrate; when the crystal form of acid salt is a hydrate, the number of water is 0.2 to 3, preferably 0.2, 0.5, 1, 1.5, 2, 2.5 or 3, more preferably 0.5, 1, 2 or 3; further, the water in the hydrate is pipeline water or crystal water or a combination of both.

In a further preferred embodiment of the present invention, provided is a method for preparing the above acid salt, comprising the following steps of:
1) weighing an appropriate amount of free base and dissolving it in a solvent;
2) adding an appropriate amount of acid and stirring;
3) centrifuging rapidly or standing to obtain a salt of the compound;

the solvent is an organic solvent, preferably at least one of methanol, ethanol, tetrahydrofuran, 2-methyltetrahydrofuran, toluene, isopropyl acetate, tert-butanol, n-butanol, acetone, 2-butanone, dichloromethane, ethyl acetate or 1,4-dioxane;
the acid is selected from the group consisting of hydrochloric acid, sulfuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetamidobenzoic acid, 4-aminobenzoic acid, capric acid, caproic acid, caprylic acid, cinnamic acid, citric acid, cyclohexane sulfamic acid, camphorsulfonic acid, aspartic acid, camphoric acid, gluconic acid, glucuronic acid, glutamic acid, erythorbic acid, lactic acid, malic acid, mandelic acid, pyroglutamic acid, tartaric acid, dodecyl sulfuric acid, dibenzoyl tartaric acid, ethane-1,2-disulfonic acid, methanesulfonic acid, ethanesulfonic acid, formic acid, fumaric acid, galactonic acid, gentisic acid, glutaric acid, 2-oxoglutaric acid, glycolic acid, hippuric acid, isethionic acid, lactobionic acid, ascorbic acid, aspartic acid, lauric acid, camphoric acid, maleic acid, malonic acid, 1,5-naphthalenedisulfonic acid, naphthalene-2-sulfonic acid, nicotinic acid, oleic acid, orotic acid, oxalic acid, palmitic acid, pamoic acid, propionic acid, salicylic acid, 4-aminosalicylic acid, sebacic acid, stearic acid, succinic acid, thiocyanic acid, undecenoic acid, trifluoroacetic acid, benzenesulfonic acid, p-toluenesulfonic acid and L-malic acid; and preferably selected from the group consisting of hydrochloric acid, sulfuric acid, phosphoric acid, ethanesulfonic acid, benzenesulfonic acid, methanesulfonic acid, fumaric acid, isethionic acid, oxalic acid and hydrobromic acid.

In a further preferred embodiment of the present invention, provided is a method for preparing the acid salt of the above compound and crystal form thereof, comprising the following steps of:
1) weighing an appropriate amount of free base and dissolving it in a reaction solvent;
2) adding an appropriate amount of acid and stirring;
3) centrifuging and drying to obtain a crystal form of acid salt of the compound;

the solvent is an organic solvent, preferably at least one of methanol, ethanol, tetrahydrofuran, 2-methyltetrahydrofuran, toluene, isopropyl acetate, tert-butanol, n-butanol, acetone, 2-butanone, dichloromethane, ethyl acetate or 1,4-dioxane;
the acid is selected from the group consisting of hydrochloric acid, sulfuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetamidobenzoic acid, 4-aminobenzoic acid, capric acid, caproic acid, caprylic acid, cinnamic acid, citric acid, cyclohexane sulfamic acid, camphorsulfonic acid, aspartic acid, camphoric acid, gluconic acid, glucuronic acid, glutamic acid, erythorbic acid, lactic acid, malic acid, mandelic acid, pyroglutamic acid, tartaric acid, dodecyl sulfuric acid, dibenzoyl tartaric acid, ethane-1,2-disulfonic acid, methanesulfonic acid, ethanesulfonic acid, formic acid, fumaric acid, galactonic acid, gentisic acid, glutaric acid, 2-oxoglutaric acid, glycolic acid, hippuric acid, isethionic acid, lactobionic acid, ascorbic acid, aspartic acid, lauric acid, camphoric acid, maleic acid, malonic acid, 1,5-naphthalenedisulfonic acid, naphthalene-2-sulfonic acid, nicotinic acid, oleic acid, orotic acid, oxalic acid, palmitic acid, pamoic acid, propionic acid, salicylic acid, 4-aminosalicylic acid, sebacic acid, stearic acid, succinic acid, thiocyanic acid, undecenoic acid, trifluoroacetic acid, benzenesulfonic acid, *p*-toluenesulfonic acid and L-malic acid; and preferably selected from the group consisting of hydrochloric acid, sulfuric acid, phosphoric acid, ethanesulfonic acid, benzenesulfonic acid, methanesulfonic acid, fumaric acid, isethionic acid, oxalic acid and hydrobromic acid.

The present invention also provides a preferred embodiment, and relates to a pharmaceutical composition comprising a therapeutically effective amount of the acid salt of the above compound or crystal form thereof, and one or more pharmaceutically acceptable carriers, diluents or excipients.

The present invention further relates to a use of any one of the acid salts of the compound of formula (I-a) or the crystal forms thereof or the pharmaceutical composition in the preparation of a P2X3 inhibitor drug.

In some embodiments, provided is a use of the pharmaceutically acceptable salt of the compound of the present invention and crystal form thereof or the pharmaceutical composition in the preparation of a medicament for the treatment of neurogenic disease; the neurogenic disease is preferably selected from the group consisting of gynecological disease, urinary tract disease state, respiratory disorder and pain-related disease or condition, more preferably selected from the group consisting of endometriosis, overactive bladder, pulmonary fibrosis and chronic cough.

In a further preferred embodiment of the present invention, the pain-related disease or condition is selected from the group consisting of neuropathic pain and uterine fibroid-related pain or discomfort.

Another object of the present invention is to provide a method for preparing a pyrazole-containing polycyclic derivative.

Specifically, the present invention provides a compound of formula (I), formula (I) is: 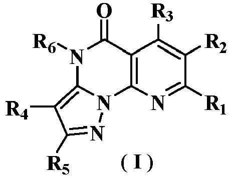 wherein:
R₁, R₂, R₃ are each independently selected from the group consisting of hydrogen, deuterium, halogen, amino, nitro, hydroxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl, heteroaryloxy and -(CH₂)ₙ₁C(O)Rₐ;
R₄, R₅ are each independently selected from the group consisting of hydrogen, deuterium, halogen, amino, nitro, hydroxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl, heteroaryloxy and -(CH₂)ₙ₁C(O)Rₐ;
R₆ is selected from the group consisting of hydrogen and a protecting group;
the protecting group may be an amino protecting group, such as a common amino protecting group, preferably *tert*-butoxycarbonyl, benoxycarbonyl, 2-biphenyl-2-propoxycarbonyl, *p*-toluenesulfonyl, trityl, formyl, trifluoroacetyl, etc.;
the compound of formula (I) in which R₆ is a protecting group can be obtained by reacting the compound of formula (I) in which R₆ is H with the corresponding protecting reagent, and the protecting group can be removed as needed;
Rₐ is selected from the group consisting of hydrogen, deuterium, halogen, amino, nitro, hydroxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl and heteroaryloxy;
the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl, heteroaryloxy and -(CH₂)ₙ₁- are each optionally substituted by one or more substituents selected from the group consisting of hydrogen, deuterium, halogen, amino, nitro, hydroxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl and heteroaryloxy;
n1 is 0, 1, 2, 3 or 4;
preferably:
when R₁, R₂, R₃, R₄ and R₆ are hydrogen at the same time, R₅ is not -C(CH₃)₃ or -COOCH₃;
when R₁ is -CF₃, and R₂, R₃, R₄ and R₆ are hydrogen at the same time, R₅ is not -CH₂CH₃, H or Br;
when R₁ is -CF₃, and R₂, R₃, R₅ and R₆ are hydrogen at the same time, R₄ is not -CN.

In some specific embodiments, the present invention provides a compound of formula (I-1), formula (I-1) is: 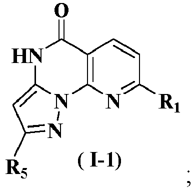
preferably, the structure of the compound is selected from the group consisting of formula (I-1-1), formula (I-1-2) and formula (I-1-3): 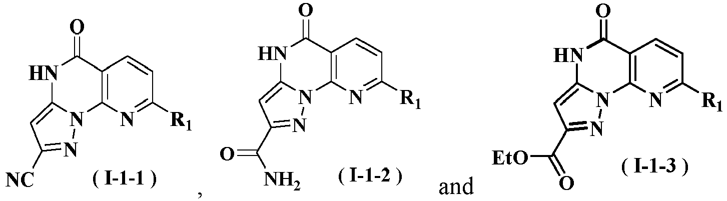
wherein,
   R₁ is selected from the group consisting of hydrogen, deuterium, halogen, cyano, C₁₋₈ alkyl, C₁₋₈ alkoxy, C₁₋₈ haloalkyl and C₁₋₈ haloalkoxy;
   preferably selected from the group consisting of cyano, C₁₋₃ alkyl, C₁₋₃ alkoxy, C₁₋₃ haloalkyl and C₁₋₃ haloalkoxy;
   more preferably selected from the group consisting of methyl, ethyl, trifluoromethyl, methoxy and cyano;
   R₅ is selected from the group consisting of hydrogen, deuterium, halogen, cyano, C₁₋₈ alkyl, C₁₋₈ alkoxy, C₁₋₈ haloalkyl, C₁₋₈ haloalkoxy and -C(O)Rₐ;
   preferably selected from the group consisting of hydrogen, deuterium, halogen, cyano and -C(O)Rₐ;
   more preferably selected from the group consisting of hydrogen, deuterium, fluorine, chlorine, bromine and -C(O)Rₐ;
   Rₐ is selected from the group consisting of hydrogen, deuterium, halogen, amino, C₁₋₃ alkyl, C₁₋₃ alkoxy, C₁₋₃ haloalkyl and C₁₋₃ haloalkoxy;
   preferably selected from the group consisting of amino, methoxy, ethoxy and isopropoxy.

The present invention provides a method for preparing a compound of formula (I) or a compound of formula (I'), comprising step (a): 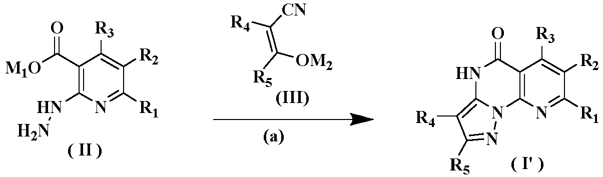
reacting a compound of formula (II) with a compound of formula (III) under a condition that can obtain the compound of formula (I') or a salt thereof to obtain the compound of formula (I') or a salt thereof;
the reaction is optionally carried out in the presence of an acid; the acid is preferably hydrochloric acid, and more preferably an organic solution of hydrochloric acid; the concentration of hydrochloric acid in the organic solution of hydrochloric acid can be 1 to 10 M/L, preferably 3 to 6 M/L, and more preferably 4 M/L; the molar ratio of the acid to the compound of formula (II) is more than 1, preferably 1 to 5:1, and more preferably 3:1;
   and/or,
the reaction is optionally carried out in a solvent, the solvent is preferably an organic solvent; the concentration of the compound of formula (II) in the solvent is 0.05 g/ml to 0.5 g/ml;
the organic solvent is preferably dioxane, tetrahydrofuran, toluene, methyltetrahydrofuran, ethyl acetate, trimethylbenzene, ethylene glycol, methanol, ethanol, isopropanol or dimethyl ether, and more preferably dioxane;
the organic solvent may also preferably be N,N-dimethylformamide, N,N-dimethylacetamide, N-methylpyrrolidone, dimethyl sulfoxide, and more preferably N,N-dimethylformamide;
the molar ratio of the compound of formula (III) to the compound of formula (II) can be 0.5 to 5.0:1, preferably 1 to 1.5:1, and more preferably 1.2:1;
the reaction temperature can be -20 to 200°C, preferably 50 to 150°C, more preferably 80 to 120°C, and further preferably 105 to 110°C;
the reaction duration can be 1 to 48 hours, preferably 10 to 30 hours, and more preferably 16 to 24 hours;
M₁, M₂ are each independently selected from the group consisting of H, Li, Na, K and Cs, M₁ is preferably H, M₂ is preferably Na or K;
R₁, R₂, R₃, R₅ are as defined in the compound of formula (I) or (I-1).

The present invention also provides a method for preparing a compound of formula (VII), comprising step (b): 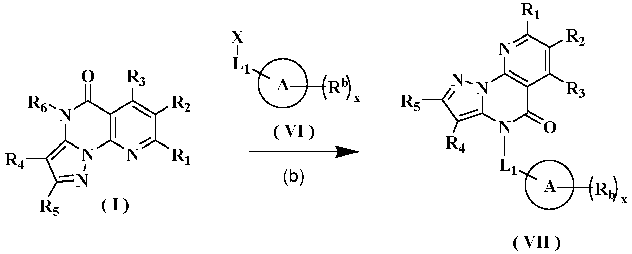
reacting a compound of formula (I) with a compound of formula (VI) to obtain the compound of formula (VII);
the molar ratio of the compound of formula (VI) to the compound of formula (I) can be 0.8 to 3:1, preferably 0.9 to 1.2:1, and more preferably 1:1;
the reaction is optionally carried out in the presence of a base;
and/or, the reaction is optionally carried out in a solvent;
wherein, the base is preferably one or more of organic bases and inorganic bases, and more preferably one or more of sodium carbonate, potassium carbonate, sodium hydroxide, potassium hydroxide, triethylamine, DIPEA, DBU, DABCO; the molar ratio of the base to the compound of formula (I) is more than 1, preferably 1 to 10:1, and more preferably 2:1;
the solvent is preferably an organic solvent, more preferably one or more of DMF, DMA, THF, and further preferably one or more of DMF, DMA; the concentration of the compound of formula (I) in the solvent is 0.05 g/ml to 0.5 g/ml;
the reaction temperature can be 0 to 50°C, preferably 20 to 25°C or 45°C;
X is halogen, preferably fluorine, chlorine or bromine, and more preferably chlorine or bromine;
L₁ is selected from the group consisting of -(CH₂)ₙ₂-, -(CH₂)ₙ₂O-, -(CH₂)ₙ₂S-, -(CH₂)ₙ₂NR_{c}-, -(CH₂)ₙ₂C(O)NR_{c}- and -(CH₂)ₙ₂NR_{c}C(O)-, preferably -CH₂C(O)NH-;
n2 is 0, 1, 2 or 3; x is 0, 1, 2 or 3;
R_{b}, R_{c} are each independently selected from the group consisting of hydrogen, deuterium, halogen, amino, nitro, hydroxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl and heteroaryloxy;
the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl, heteroaryloxy and -(CH₂)ₙ₂- (-(CH₂)ₙ₂- can also be the alkylene chain in -(CH₂)ₙ₂O-, -(CH₂)ₙ₂S-, -(CH₂)ₙ₂NR_{c}-, -(CH₂)ₙ₂C(O)NR_{c}- or -(CH₂)ₙ₂NR_{c}C(O)-) are each optionally substituted by one or more substituents selected from the group consisting of hydrogen, deuterium, halogen, amino, nitro, hydroxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl and heteroaryloxy; R_{b} is preferably halogen; R_{c} is preferably H;
ring A is selected from the group consisting of cycloalkyl, heterocyclyl, aryl and heteroaryl, preferably 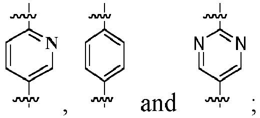 and/or,
the compound of formula (VI) is preferably 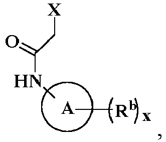 more preferably 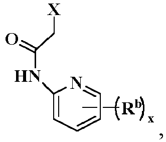 and further preferably 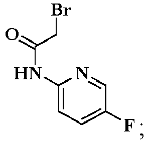
R₁, R₂, R₃, R₅ are as defined in the compound of formula (I) or (I-1);
optionally, the method for preparing the compound of formula (VII) also comprises the step of preparing the compound of formula (I).

The present invention also provides a method for preparing a compound of formula (I-3), comprising step (c-1): 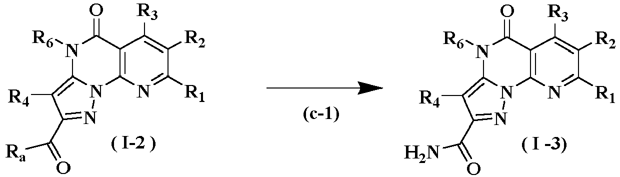
subjecting a compound of formula (I-2) to an amination reduction reaction in the presence of ammonia gas or ammonia gas equivalents to obtain the compound of formula (I-3);
the ammonia gas equivalent is an organic solution of ammonia or aqueous ammonia; the organic solution of ammonia is preferably ammonia in methanol, ammonia in ethanol, ammonia in isopropanol, or ammonia in dioxane;
preferably, subjecting a compound of formula (I-2) to an amination reduction reaction in the presence of aqueous ammonia and/or organic solution of ammonia to obtain the compound of formula (I-3); more preferably, subjecting a compound of formula (I-2) to an amination reduction reaction in the presence of aqueous ammonia and/or ammonia in methanol to obtain the compound of formula (I-3);
the reaction temperature can be 0 to 50°C, and preferably room temperature 25°C;
the reaction duration is 1 to 72 hours, and preferably 48 to 60 hours;
Rₐ is selected from the group consisting of hydrogen, deuterium, halogen, hydroxy, alkoxy, haloalkoxy, aryloxy and heteroaryloxy, the alkoxy, haloalkoxy, aryloxy and heteroaryloxy are each optionally substituted by one or more substituents selected from the group consisting of hydrogen, deuterium, halogen, amino, nitro, hydroxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl and heteroaryloxy;
R₁, R₂, R₃, R₆ are as defined in the compound of formula (I) or (I-1).

The present invention also provides a method for preparing a compound of formula (I-4), comprising step (d-1): 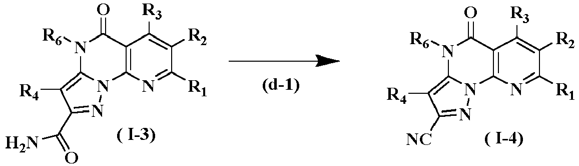
reacting a compound of formula (I-3) in the presence of a dehydrating agent to obtain the compound of formula (I-4);
the dehydrating agent is preferably one or more of acetic anhydride, trifluoroacetic anhydride, P₂O₅, cyanuric chloride, phosphorus oxychloride, phosphorus trichloride, and concentrated sulfuric acid; the molar ratio of the dehydrating agent to the compound of formula (I-3) is more than 1, preferably 1 to 10:1, and more preferably 2 to 2.5:1;
the reaction is optionally carried out in the presence of pyridine; the molar ratio of pyridine to the compound of formula (I-3) is more than 1, preferably 1 to 10:1, more preferably 2.5 to 3:1, and further preferably 3:1;
   and/or,
the reaction is optionally carried out in a solvent; the solvent is preferably an organic solvent, more preferably one or more of DMF, DMA, THF, and further preferably one or more of DMF, THF; the concentration of the compound of formula (I-3) in the solvent is 0.05 g/ml to 0.5 g/ml;
the reaction temperature can be -20 to 80°C, preferably room temperature;
the reaction duration is 0.1 to 10 hours, preferably 0.5 to 4 hours, and more preferably 1 to 2 hours;
R₁, R₂, R₃, R₆ are as defined in the compound of formula (I) or (I-1).

The present invention also relates to a method for preparing a compound of formula (VII-4), characterized in that the structure of the compound of formula (VII-4) is: 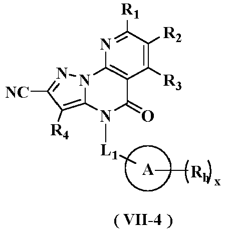
L₁, x, R_{b}, ring A, R₁, R₂, R₃, R₄ are as defined in the compound of formula (I) or (I-1) and the compound of formula (VII);
the method comprises the following steps of:
   1-1: preparing the compound of formula (VII-4) from the compound of formula (I-4) according to the above method;
   1-2: optionally, preparing the compound of formula (I-4) from the compound of formula (I-3) according to the above method;
   1-3: optionally, preparing the compound of formula (I-3) from the compound of formula (I-2) according to the above method;
   1-4: optionally, preparing the compound of formula (I-2) according to the above method.

The present invention also provides a compound of formula (I), a compound of formula (II) and a compound of formula (III), the compound of formula (I), the compound of formula (II) and the compound of formula (III) are used as intermediates in the preparation of P2X3 inhibitor such as the substance with P2X3 inhibitory activity disclosed in the patent application PCT/CN2020/134264. The patent application PCT/CN2020/134264 is incorporated into the present application in its entirety by reference. The P2X3 inhibitor is preferably a P2X3 inhibitor containing a core structure of 4,5-dihydropyrazolo[1,5-a]pyrido[3,2-e]pyrimidine, and more preferably a compound of formula (VII-4):

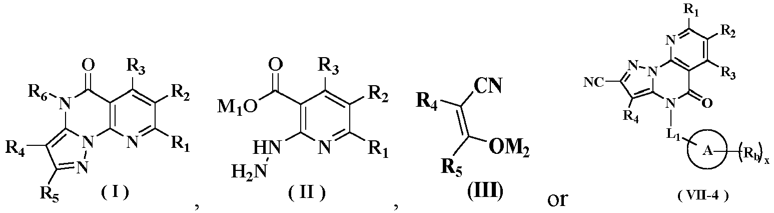

L₁, x, R_{b}, ring A, R₁, R₂, R₃, R₅, R₆, M₁, M₂ are as defined in the compound of formula (I) or (I-1), the compound of formula (I'), and the compound of formula (VII).

The present invention also relates to a method for preparing a compound of formula (II') or a compound of formula (V'), the method also comprises step (e) and/or step (c); or optionally further comprises step (g), 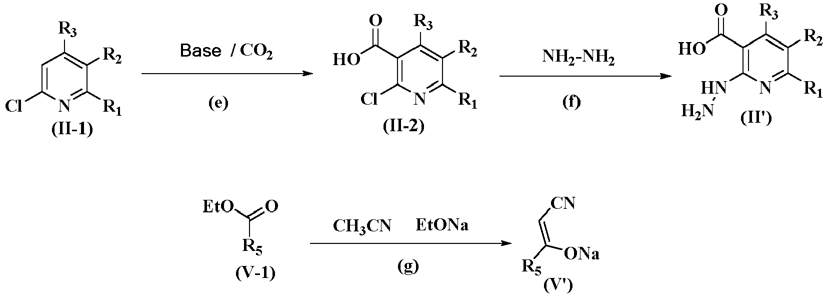
step (e): reacting a compound of formula (II-1) in a solvent in the presence of a base and CO₂ to obtain a compound of formula (II-2); the base is preferably LDA, butyl lithium or sodium hydroxide, etc.; the solvent is preferably tetrahydrofuran, toluene, ethylene glycol dimethyl ether or dichloromethane;
the molar ratio of the compound of formula (II-I) to the base is 1:1 to 10, preferably 1:1 to 5, and more preferably 1:1 to 1.5;
step (f): reacting the compound of formula (II-2) in a solvent in the presence of hydrazine hydrate to obtain a compound of formula (II'); the solvent is preferably hydrazine hydrate, methanol, ethanol, isopropanol or tert-butyl alcohol;
the molar ratio of the compound of formula (II-2) to hydrazine hydrate is 1:1 to 10, preferably 1:1 to 5, and more preferably 1:1 to 1.5;
step (g): reacting a compound of formula (V-1) with acetonitrile in the presence of sodium ethoxide to obtain a compound of formula (V');
the molar ratio of the compound of formula (V-1) to acetonitrile is 1:1 to 10, preferably 1:1 to 5, and more preferably 1:1 to 1.5;
R₁, R₂, R₃, R₅ are as defined in the compound of formula (I) or (I-1).

The method for preparing a pyrazole-containing polycyclic derivative of the present invention avoids the use of expensive raw materials such as pyrazolamide derivatives and precious metal catalysts. The raw materials are cheap and easy to obtain. The cost is low. The reaction conditions are mild. The yield is high. The process is mature. And the quality is stable. The method is suitable for industrial scale-up, and is more in line with safety and environmental protection requirements.

### DEFINITIONS

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 8 carbon atoms, more preferably an alkyl having 1 to 6 carbon atoms, and most preferably an alkyl having 1 to 3 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. More preferably, the alkyl is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl can be substituted or unsubstituted. When substituted, the substituent group(s) can be substituted at any available connection point. The substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl. The alkyl of the present invention is preferably selected from the group consisting of methyl, ethyl, isopropyl, *tert*-butyl, haloalkyl, deuterated alkyl, alkoxy-substituted alkyl and hydroxy-substituted alkyl.

"Deuterated alkyl" refers to an alkyl in which one or more hydrogens are substituted by deuterium, wherein the alkyl is as defined above.

The term "alkylene" refers to an alkyl of which a hydrogen atom is further substituted, for example, "methylene" refers to -CH₂-, "ethylene" refers to -(CH₂)₂-, "propylene" refers to -(CH₂)₃-, "butylene" refers to -(CH₂)₄- and the like. The term "alkenyl" refers to an alkyl as defined above that consists of at least two carbon atoms and at least one carbon-carbon double bond, for example, ethenyl, 1-propenyl, 2-propenyl, 1-, 2- or 3-butenyl and the like. The alkenyl can be substituted or unsubstituted. When substituted, the substituent(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "cycloalkyl" refers to a saturated or partially unsaturated monocyclic or polycyclic hydrocarbon substituent having 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring. The cycloalkyl is preferably cyclopropyl, cyclobutyl, cyclohexyl, cyclopentyl and cycloheptyl.

The cycloalkyl ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like. The cycloalkyl can be optionally substituted or unsubstituted. When substituted, the substituent(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "heterocyclyl" refers to a 3 to 20 membered saturated or partially unsaturated monocyclic or polycyclic hydrocarbon group, wherein one or more ring atoms are heteroatoms selected from the group consisting of nitrogen, oxygen and S(O)ₘ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; more preferably, 3 to 8 ring atoms; most preferably 3 to 8 ring atoms; and further preferably, 3 to 8 ring atoms with 1 to 3 nitrogen atoms. Optionally, the heterocyclyl is substituted by 1 to 2 oxygen atom, sulfur atom, oxo. The heterocyclyl includes nitrogen-containing monocyclic heterocyclyl, nitrogen-containing spiro heterocyclyl and nitrogen-containing fused heterocyclyl.

Non-limiting examples of monocyclic heterocyclyl include oxetanyl, thietanyl, pyrrolidinyl, imidazolidinyl, tetrahydrofuranyl, tetrahydrothienyl, tetrahydropyranyl, dihydroimidazolyl, dihydrofuranyl, dihydropyrazolyl, dihydropyrrolyl, piperidinyl, piperazinyl, morpholinyl, thiomorpholinyl, homopiperazinyl, azetyl, 1,4-diazacycloheptyl, pyranyl, tetrahydrothiapyran dioxide group and the like, preferably oxetanyl, thietanyl, tetrahydrofuranyl, tetrahydropyranyl, tetrahydrothienyl, tetrahydrothiapyranyl, tetrahydrothiapyranyl dioxide group, pyrrolidinyl, morpholinyl, piperidinyl, azetyl, 1,4-diazacycloheptyl and piperazinyl, and more preferably oxetanyl, piperidinyl, tetrahydropyranyl and tetrahydrothiapyranyl. Polycyclic heterocyclyl includes a heterocyclyl having a spiro ring, fused ring or bridged ring. The heterocyclyl having a spiro ring, fused ring or bridged ring is optionally bonded to other group via a single bond, or further bonded to other cycloalkyl, heterocyclyl, aryl and heteroaryl via any two or more atoms on the ring.

The heterocyclyl ring can be fused to the ring of aryl, heteroaryl or cycloalkyl, wherein the ring bound to the parent structure is heterocyclyl. Non-limiting examples include: 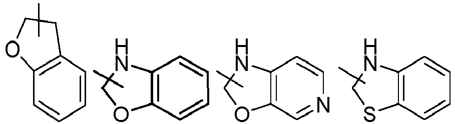 and the like.

The heterocyclyl can be optionally substituted or unsubstituted. When substituted, the substituent(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "aryl" refers to a 6 to 14 membered all-carbon monocyclic ring or polycyclic fused ring (*i.e.* each ring in the system shares an adjacent pair of carbon atoms with another ring in the system) having a conjugated π-electron system, preferably a 6 to 12 membered aryl, for example, phenyl and naphthyl. The aryl is more preferably phenyl. The aryl ring can be fused to the ring of heteroaryl, heterocyclyl or cycloalkyl. The aryl includes benzo 5 to 10 membered heteroaryl, benzo 3 to 8 membered cycloalkyl and benzo 3 to 8 membered heterocyclyl, preferably benzo 5 to 6 membered heteroaryl, benzo 3 to 6 membered cycloalkyl and benzo 3 to 6 membered heterocyclyl, wherein the heterocyclyl is a heterocyclyl containing 1 to 3 nitrogen atoms, oxygen atoms or sulfur atoms. The aryl also includes 3 membered nitrogen-containing fused ring containing a benzene ring.

The aryl can be substituted or unsubstituted. When substituted, the substituent(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

"Aryloxy" refers to an -O-(aryl), wherein the aryl is as defined above.

The term "heteroaryl" refers to a 5 to 14 membered heteroaromatic system having 1 to 4 heteroatoms selected from the group consisting of oxygen, sulfur and nitrogen. The heteroaryl is preferably a 5 to 12 membered heteroaryl, and more preferably a 5 or 6 membered heteroaryl, for example imidazolyl, furanyl, thienyl, thiazolyl, pyrazolyl, oxazolyl, pyrrolyl, triazolyl, tetrazolyl, pyridyl, pyrimidinyl, thiadiazolyl, pyridazinyl, pyrazinyl and the like, preferably pyridyl, oxadiazolyl, triazolyl, thienyl, imidazolyl, pyrazolyl, oxazolyl, pyrimidinyl, furyl, thienyl, pyridazinyl, pyrazinyl and thiazolyl, and more preferably pyridyl, furyl, thienyl, pyrimidinyl, oxazolyl, oxadiazolyl, pyrazolyl, pyrrolyl, thiazolyl, pyridazinyl, pyrazinyl and oxazolyl. The heteroaryl ring can be fused to the ring of aryl, heterocyclyl or cycloalkyl, wherein the ring bound to the parent structure is heteroaryl ring. Non-limiting examples thereof include: 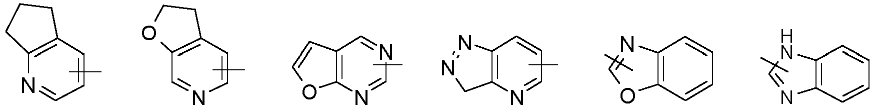 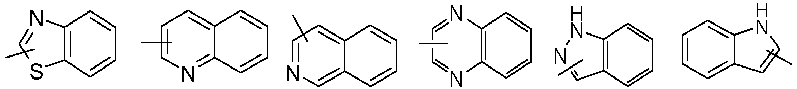 and the like.

The heteroaryl can be optionally substituted or unsubstituted. When substituted, the substituent(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

"Heteroaryloxy" refers to an -O-(heteroaryl), wherein the heteroaryl is as defined above.

The term "alkoxy" refers to an -O-(alkyl) or an -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "alkylthio" refers to an -S-(alkyl) or an -S-(unsubstituted cycloalkyl) group, wherein the alkyl is as defined above. Non-limiting examples of alkylthio include methoxy, ethoxy, propoxy, butoxy, cyclopropyloxy, cyclobutyloxy, cyclopentyloxy, cyclohexyloxy. The alkylthio can be optionally substituted or unsubstituted. When substituted, the substituent(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

"Haloalkyl" refers to an alkyl substituted by one or more halogen(s), wherein the alkyl is as defined above, for example trifluoromethyl.

"Haloalkoxy" refers to an alkoxy substituted by one or more halogen(s), wherein the alkoxy is as defined above.

"Hydroxyalkyl" refers to an alkyl substituted by hydroxy(s), wherein the alkyl is as defined above.

"Alkenyl" refers to a chain olefin, also known as alkene group. The alkenyl can be further substituted by other related group, for example hydrogen, deuterium, amino, alkyl, deuterated alkyl, haloalkyl, hydroxyalkyl, alkenyl, alkynyl, alkoxy, haloalkoxy, alkylthio, alkylamino, halogen, thiol, hydroxy, nitro, cyano, cycloalkyl, heterocyclyl, aryl, heteroaryl, heterocyclyl, aryloxy, heteroaryloxy, cycloalkoxy, heterocycloalkoxy, cycloalkylthio, heterocyclylthio, carboxy or alkoxycarbonyl.

"Alkynyl" refers to (CH=C-). The alkynyl can be further substituted by other related group, for example hydrogen, deuterium, amino, alkyl, deuterated alkyl, haloalkyl, hydroxyalkyl, alkenyl, alkynyl, alkoxy, haloalkoxy, alkylthio, alkylamino, halogen, thiol, hydroxy, nitro, cyano, cycloalkyl, heterocyclyl, aryl, heteroaryl, heterocyclyl, aryloxy, heteroaryloxy, cycloalkoxy, heterocycloalkoxy, cycloalkylthio, heterocyclylthio, carboxy or alkoxycarbonyl. The term "alkenylcarbonyl" refers to -C(O)-(alkenyl), wherein the alkenyl is as defined above. Non-limiting examples of alkenylcarbonyl include: vinylcarbonyl, propenylcarbonyl, butenylcarbonyl. The alkenylcarbonyl can be optionally substituted or unsubstituted. When substituted, the substituent(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

"Hydroxy" refers to an -OH group.

"Halogen" refers to fluorine, chlorine, bromine or iodine.

"Amino" refers to a -NH₂ group.

"Cyano" refers to a -CN group.

"Nitro" refers to a -NO₂ group.

"Carbonyl" refers to a -C(O)- group.

"Carboxy" refers to a -C(O)OH group.

"Alcoholic solvents" refer to alkane compounds containing hydroxy groups in the molecules, such as methanol, ethanol, and isopropanol.

"THF" refers to tetrahydrofuran.

"EtOAc" refers to ethyl acetate.

"MeOH" refers to methanol.

"DMF" refers to N,N-dimethylformamide.

"TFA" refers to trifluoroacetic acid.

"MeCN" refers to acetonitrile.

"DMA" refers to N,N-dimethylacetamide. "Et₂O" refers to diethyl ether.

"DCE" refers to 1,2-dichloroethane.

"DIPEA" refers to N,N-diisopropylethylamine.

"DBU" refers to 1,8-diazabicyclo[5.4.0]undec-7-ene.

"DABCO" refers to 1,4-diazabicyclo[2.2.2]octane.

"NBS" refers to N-bromosuccinimide.

"NIS" refers to N-iodosuccinimide.

"Cbz-Cl" refers to benzyl chloroformate.

"Pd₂(dba)₃" refers to tris(dibenzylideneacetone)dipalladium.

"Dppf' refers to 1,1'-bisdiphenylphosphinoferrocene.

"HATU" refers to 2-(7-azabenzotriazol-1-yl)-N,N,N',N'-tetramethyluronium hexafluorophosphate.

"KHMDS" refers to potassium hexamethyldisilazide.

"LiHMDS" refers to lithium bis(trimethylsilyl)amide.

"MeLi" refers to methyl lithium.

"n-BuLi" refers to n-butyl lithium.

"NaBH(OAc)₃" refers to sodium triacetoxyborohydride.

Different expressions such as "X is selected from the group consisting of A, B or C", "X is selected from the group consisting of A, B and C", "X is A, B or C", "X is A, B and C" are the same meaning, that is, X can be any one or more of A, B and C.

In the present invention, the expressions plurality, multiple refer to 2, 3, 4, 5, 6, 7, etc.

The hydrogen atom of the present invention can be replaced by its isotope deuterium. Any of the hydrogen atoms in the compounds of the examples of the present invention can also be substituted by deuterium atom.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without excessive efforts. For example, the combination of amino or hydroxy having free hydrogen and carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components such as physiologically/pharmaceutically acceptable carriers and excipients. The purpose of the pharmaceutical composition is to facilitate administration of a compound to an organism, which is conducive to the absorption of the active ingredient so as to exert biological activity.

A "pharmaceutically acceptable salt" refers to a salt of the compound of the present invention, which is safe and effective in mammals and has the desired biological activity.

X-ray powder diffraction (XRPD) pattern refers to the experimentally observed diffraction pattern or the parameters derived from it, and the X-ray powder diffraction pattern is characterized by peak position (abscissa) and peak intensity (ordinate). Those skilled in the art will appreciate that the experimental error therein depends on the conditions of the instrument, the preparation of the sample, and the purity of the sample. In particular, it is well known to those skilled in the art that X-ray diffraction pattern generally varies with the conditions of the instrument. Those skilled in the art will appreciate that suitable error tolerances for XRPD may be: 2θ±0.5°, 2θ±0.4°, 2θ±0.3°, 2θ±0.2°. In particular, it is important to point out that the relative intensity in X-ray diffraction pattern may vary with experimental conditions, so the order of peak intensity cannot be used as the sole or decisive factor. In addition, due to the influence of experimental factors such as the height of the sample, the overall deviation of the peak angle will occur, and a certain deviation is usually allowed. Therefore, those skilled in the art can understand that any crystal form having the same or similar characteristic peaks as the pattern of the present invention falls within the scope of the present invention.

### DESCRIPTION OF THE DRAWINGS

Figure 1 is the XRPD pattern of crystal form A of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide methanesulfonate.
Figure 2 is the DSC spectrum of crystal form A of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide methanesulfonate.
Figure 3 is the TGA spectrum of crystal form A of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide methanesulfonate.
Figure 4 is the XRPD pattern of crystal form A of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide ethanesulfonate.
Figure 5 is the DSC spectrum of crystal form A of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide ethanesulfonate.
Figure 6 is the TGA spectrum of crystal form A of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide ethanesulfonate.
Figure 7 is the XRPD pattern of crystal form A of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide sulfate.
Figure 8 is the DSC spectrum of crystal form A of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide sulfate.
Figure 9 is the XRPD pattern of crystal form B of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide sulfate.
Figure 10 is the DSC spectrum of crystal form B of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide sulfate.
Figure 11 is the TGA spectrum of crystal form B of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide sulfate.
Figure 12 is the XRPD pattern of crystal form A of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide hydrochloride.
Figure 13 is the DSC spectrum of crystal form A of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide hydrochloride.
Figure 14 is the TGA spectrum of crystal form A of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide hydrochloride.
Figure 15 is the XRPD pattern of crystal form B of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide hydrochloride.
Figure 16 is the XRPD pattern of crystal form A of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide hydrobromide.
Figure 17 is the DSC spectrum of crystal form A of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide hydrobromide.
Figure 18 is the XRPD pattern of crystal form B of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide hydrobromide.
Figure 19 is the DSC spectrum of crystal form B of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide hydrobromide.
Figure 20 is the TGA spectrum of crystal form B of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide hydrobromide.
Figure 21 is the XRPD pattern of crystal form C of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide hydrobromide.
Figure 22 is the XRPD pattern of crystal form I of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide free base.
Figure 23 is the DSC spectrum of crystal form I of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide free base.

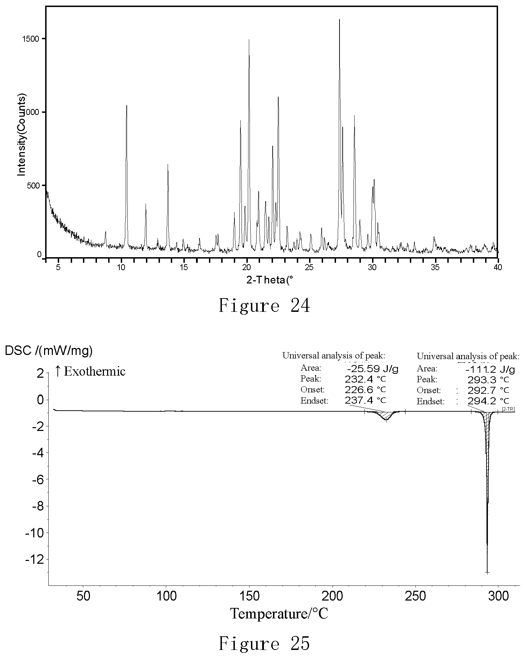

Figure 24 is the XRPD pattern of crystal form II of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide free base.
Figure 25 is the DSC spectrum of crystal form II of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide free base.

### DETAILED DESCRIPTION OF THE INVENTION

The present invention will be further described with reference to the following examples, but the examples should not be considered as limiting the scope of the present invention.

### EXAMPLES

The structures of the compounds of the present invention were identified by nuclear magnetic resonance (NMR) and/or liquid chromatography-mass spectrometry (LC-MS). NMR shifts (δ) are given in parts per million (ppm). NMR is determined by a Bruker AVANCE-400 instrument. The solvents for determination are deuterated-dimethyl sulfoxide (DMSO-*d₆*), deuterated-methanol (CD₃OD) and deuterated-chloroform (CDCl₃), and the internal standard is tetramethylsilane (TMS).

Liquid chromatography-mass spectrometry (LC-MS) is determined on an Agilent 1200 Infinity Series mass spectrometer. High performance liquid chromatography (HPLC) is determined on an Agilent 1200DAD high pressure liquid chromatograph (Sunfire C18 150×4.6 mm column), and a Waters 2695-2996 high pressure liquid chromatograph (Gimini C₁₈ 150×4.6 mm column).

Yantai Huanghai HSGF254 or Qingdao GF254 silica gel plate is used as the thin-layer silica gel chromatography (TLC) plate. The dimension of the silica gel plate used in TLC is 0.15 mm to 0.2 mm, and the dimension of the silica gel plate used in product purification is 0.4 mm to 0.5 mm. Yantai Huanghai 200 to 300 mesh silica gel is generally used as a carrier for column chromatography.

The HPLC detection method in the present invention is as follows:

| | | | | |
|---|---|---|---|---|
| **Instrument information** | Agilent 1260 Infinity | | | |
| **Detector model** | G4212B 1260 DAD | | | |
| **Column** | Agilent ZORBAX SB-C18, 4.6×150mm, 5µm | | | |
| **Column temperature** | 40°C | | Flow rate | 1.0 ml/min |
| **Detection wavelength** | 205nm 254nm 214nm 225nm | | Autosampler temperature | room temperature |
| **Mobile phase** | Mobile phase A | 0.05% TFA in H₂O | | |
| | Mobile phase B | 0.05% TFAinACN | | |

| | time (min) | Mobile phase A (%) | | Mobile phase B (%) |
|---|---|---|---|---|
| **Gradient** | 0.00 | 95 | | 5 |
| | 13.00 | 5 | | 95 |
| | 16.00 | 5 | | 95 |
| | Post-run time: 5 min | | | |

The raw materials used in the examples of the present invention are known and commercially available, or can be synthesized by or according to known methods in the art.

Unless otherwise stated, all reactions of the present invention are carried out under continuous magnetic stirring under a dry nitrogen or argon atmosphere. The solvent is dry, and the reaction temperature is in degrees celsius.

### Intermediate 1

### Preparation of 2-chloro-6-trifluoromethylnicotinic acid

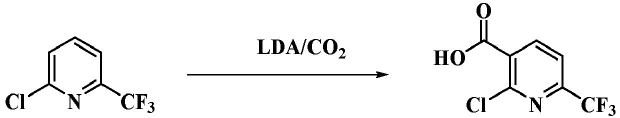

2-Chloro-6-(trifluoromethyl)pyridine (50 g, 275.42 mmol) and tetrahydrofuran (600 mL) were added into a 1 L three-necked flask. The reaction solution was cooled to -50°C under a nitrogen atmosphere. Lithium diisopropylamide (206.6 mL, 413.13 mmol) was slowly added dropwise to the reaction solution, and stirred for 2 hours after the addition was completed. Dry ice (750 g) was weighed, the reaction solution was poured into the container containing the dry ice, and stirred at room temperature for 3 hours, TLC showed that the reaction of the raw materials was completed. Saturated ammonium chloride solution (200 mL) was added to the reaction solution and stirred for 5 minutes. Water (200 mL) was added, and the solution was extracted with ethyl acetate (200 mL×3). The organic phase was washed with water (200 ml × 3), and the aqueous phases were collected. The aqueous phase was adjusted to pH 2 with 4 M hydrochloric acid, and extracted with ethyl acetate (500 mL×3). The organic phases were combined, washed with saturated sodium chloride solution (300 mL×2), dried over anhydrous sodium sulfate and filtered. The filtrate was concentrated under reduced pressure, and purified by pulping in dichloromethane (100 mL) to obtain 2-chloro-6-trifluoromethylnicotinic acid (32 g, white solid), yield: 51.5%, HPLC purity: 95.0%.

MS (ESI) m/z: 226.55 [M+1]⁺.

¹H NMR (400 MHz, DMSO-*d*₆): *δ*/ppm 12.67 (s, 1H), 8.32 (d, J = 8.0 Hz, 1H), 7.68 (d, J = 8.0 Hz, 1H).

### Intermediate 2

### Preparation of 2-hydrazineyl-6-(trifluoromethyl)nicotinic acid

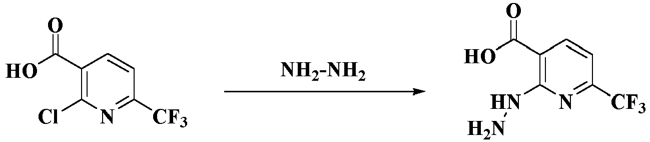

Hydrazine hydrate (210mL) and 2-chloro-6-(trifluoromethyl)nicotinic acid (30 g, 0.133 mol) were successively added to a reaction flask under stirring to obtain a clear solution. The reaction flask was heated in an oil bath until the internal temperature was 75 to 80°C, the reaction was conducted for 12 to 16 hours, and the raw materials were reacted completely. The reaction solution was cooled to room temperature, cooled in an ice-water bath, and the pH was adjusted to 4~5 with 1 N HCl. A large amount of yellow solid were precipitated, and the mixture was stirred for 30 minutes. The mixture was filtered under reduced pressure, and the filter cake was washed twice with water. The filter cake was dried under vacuum to constant weight to obtain a yellow solid, mass m=26.3 g, yield: 89.4%, HPLC purity of the solid: 99.1%.

MS (ESI) m/z: 222.14 [M+1]⁺.

¹H NMR (400 MHz, DMSO-*d*₆): *δ*/ppm 12.67 (s, 1H), 10.27 (m, 1H), 7.82 (d, J = 8.0 Hz, 1H), 7.70 (d, J = 8.0 Hz, 1H), 4.61 (m, 2H).

### Intermediate 3

### Preparation of sodium ethyl cyanopyruvate

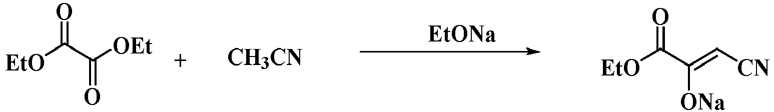

Sodium ethoxide (450 ml, 20% mass fraction) was added under stirring under a nitrogen atmosphere, and cooled to 0~5°C in an ice water bath. Diethyl oxalate (96.6 g, 0.66 mol) was added into the reaction solution over 30 minutes (there was an exothermic phenomenon), and stirred for 20 to 30 minutes. Acetonitrile (27.1 g, 0.66 mol) was added to the reaction solution, and the system became a clear solution. The reaction solution was warmed to 35°C in an oil bath and reacted for 20 to 24 hours, then a large amounts of solid were gradually precipitated. The heating was turned off. The reaction solution was cooled to room temperature, and filtered at 20 to 25°C to obtain an off-white solid. The filter cake was washed with 100 ml of ice ethanol, and dried under vacuum to constant weight, m=80 g, yield: 74%.

MS (ESI) m/z: 140.11 [M-Na]⁻.

¹H NMR (400 MHz, DMSO--*d*₆): *δ*/ppm 7.52 (s, 1H), 4.01 (d, J = 8.0 Hz, 2H), 1.18 (m, 3H).

### Intermediate 4

### Preparation of 2-bromo-N-(5-fluoropyridin-2-yl)acetamide

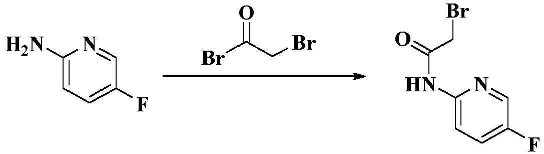

2-Amino-5-fluoropyridine (25 g, 0.223 mol) and THF (250 ml) were added into a 500 ml reaction flask under stirring and a nitrogen atmosphere. The temperature was kept at 20°C to 25°C, and DIPEA (37.4 g, 0.289 mol) was added. Bromoacetyl bromide (49.5 g, 0.245 mol) was added dropwise, and a large amount of salt were precipitated and the solution color became darker. After the addition was completed, the reaction was conducted for 1 hour, and the raw materials were completely reacted. The reaction solution was poured into 500 ml of water and extracted with ethyl acetate (150 ml×2). The organic phases were combined, washed once with 150 ml of water and once with 150 ml of brine, and dried over anhydrous sodium sulfate. The solution was filtered, and concentrated under reduced pressure until 100 ml of ethyl acetate remained. 100 ml of *n*-heptane was added dropwise and stirred for crystallization. The mixture was filtered under reduced pressure to obtain an off-white solid, which was dried under vacuum to constant weight, m=31.8 g, yield: 61.2%, HPLC purity: 98.6%.

MS (ESI) m/z: 234.04 [M+1]⁺.

¹H NMR (400 MHz, DMSO-*d*₆): *δ*/ppm 10.92 (m, 1H), 8.36 (m,1H), 8.12 (m, 1H), 7.82-7.76 (m, 1H), 4.36 (m, 2H).

### Example 1

### 2-(2-(Tert-butyl)-5-oxopyrazolo[1,5-a]pyrido[3,2-e]pyrimidin-4(5H)-yl)-N-(5-fluoro pyridin-2-yl)acetamide

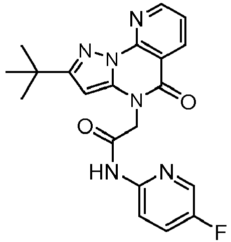

### Step 1: Preparation of N-(3-(tert-butyl)-1H-pyrazol-5-yl)-2-chloronicotinamide

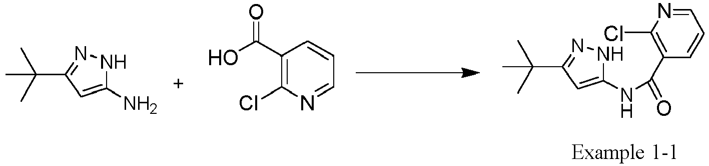

3-(Tert-butyl)-1H-pyrazol-5-amine (2.77 g, 19.93 mmol), DIPEA (6.2 g, 49.8 mmol) and HATU (5.4 g, 0.144 mmol) were added successively to a solution of 2-chloronicotinic acid (1.57 g, 9.96 mmol) in DMF (30 mL) under an ice bath condition. The ice bath was removed, and the reaction solution was stirred for 1 hour. The mixture was treated to obtain Example 1-1 (2.5 g, 90%).

MS m/z (ESI): 279.7 [M+H]⁺.

### Step 2: Preparation of 2-(tert-butyl)pyrazolo[1,5-a]pyrido[3,2-e]pyrimidin-5(4H)-one

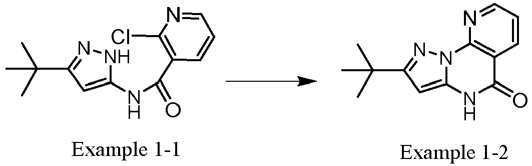

Potassium carbonate (1.61 g, 11.66 mmol) and 1,4-diazabicyclo[2.2.2]octane (DABCO) (150.9 mg, 1.35 mmol) were added to a solution of Example 1-1 (2.5 g, 8.97 mmol) in DMF (50 mL). The reaction solution was stirred at room temperature for 16 hours. The mixture was treated to obtain Example 1-2 (2.1 g, 97%).

MS m/z (ESI): 279.7 [M+H]⁺.

### Step 3: Preparation of 2-(2-(tert-butyl)-5-oxopyrazolo[1,5-a]pyrido[3,2-e]pyrimidin-4(5H)-yl)-N-(5-fluoropyri din-2-yl)acetamide

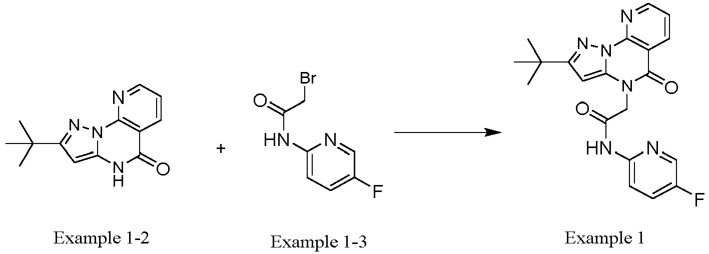

Potassium carbonate (4.28 g, 30.96 mmol) and Example 1-3 (4.33 g, 18.57 mmol) were added to a solution of Example 1-2 (1.5 g, 6.19 mmol) in DMF (30 mL) at room temperature. The mixture was heated to 80°C and stirred for 2 hours. The reaction solution was cooled followed by addition of water. The precipitate was filtered, washed with ethyl acetate, and purified to obtain Example 1 (656 mg, yield: 27%).

MS m/z (ESI): 395.4 [M+H]⁺.

¹H NMR (400 MHz, DMSO-d6) δ 11.01 (s, 1H), 8.80 - 8.78 (m, 1H), 8.47 (d, J = 7.6 Hz, 1H), 8.30 (d, J = 2.8 Hz, 1H), 8.01 - 7.94 (m, 1H), 7.73 - 7.66 (m, 1H), 7.49 (dd, J = 8.0, 4.8 Hz, 1H), 6.34 (s, 1H), 4.87 (s, 2H), 1.26 (s, 9H).

### Example 2

### 2-(2-Bromo-5-oxopyrazolo[1,5-a]pyrido[3,2-e]pyrimidin-4(5H)-yl)-N-(5-fluoropyri din-2-yl)acetamide

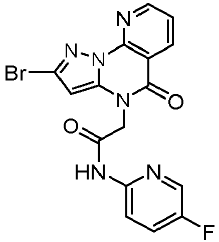

Example 2 was synthesized according to the method of Example 1. The target compound (500 mg, yield: 68%) was obtained by replacing 3-(tert-butyl)-1H-pyrazol-5-amine with 3-bromo-1H-pyrazol-5-amine.

MS m/z (ESI): 418.2 [M+H]⁺.

¹H NMR (400 MHz, DMSO-d6) δ 11.32 (s, 1H), 9.85 (d, J = 7.6 Hz, 1H), 8.74 (d, J = 6.4 Hz, 1H), 8.40 (d, J = 2.8 Hz, 1H), 8.05 - 8.00 (m, 1H), 7.78 - 7.73 (m, 1H), 7.23 - 7.17 (m, 1H), 6.31 (s, 1H), 5.52 (s, 2H).

### Example 3

### N-(5-Fluoropyridin-2-yl)-2-(2-methyl-5-oxopyrazolo[1,5-a]pyrido[3,2-e]pyrimidin-4(5H)-yl)acetamide

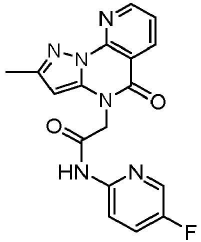

Example 3 was synthesized according to the method of Example 1. The target compound (20 mg, yield: 26%) was obtained by replacing 3-(tert-butyl)-1H-pyrazol-5-amine with 3-methyl-1H-pyrazol-5-amine.

MS m/z (ESI): 353.3 [M+H]⁺.

### Example 4

### N-(5-Fluoropyridin-2-yl)-2-(2-ethyl-5-oxopyrazolo[1,5-a]pyrido[3,2-e]pyrimidin-4( 5H)-yl)acetamide

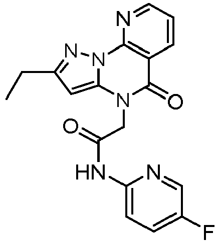

Example 4 was synthesized according to the method of Example 1. The target compound (15 mg, yield: 36%) was obtained by replacing 3-(tert-butyl)-1H-pyrazol-5-amine with 3-ethyl-1H-pyrazol-5 -amine.

MS m/z (ESI): 367.4 [M+H]⁺.

### Example 5

### N-(5-Fluoropyridin-2-yl)-2-(2-isopropyl-5-oxopyrazolo [1,5-a] pyrido [3,2-e] pyrimidi n-4(5H)-yl)acetamide

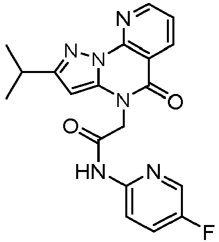

Example 5 was synthesized according to the method of Example 1. The target compound (15 mg, yield: 36%) was obtained by replacing 3-(tert-butyl)-1H-pyrazol-5-amine with 3-isopropyl-1H-pyrazol-5-amine.

MS m/z (ESI): 381.4 [M+H]⁺.

### Example 6

### N-(5-Fluoropyridin-2-yl)-2-(2-isopropenyl-5-oxopyrazolo[1,5-a]pyrido[3,2-e]pyrimi din-4(5H)-yl)acetamide

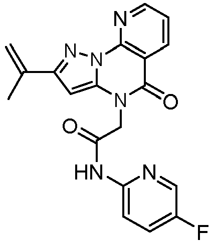

Example 2 (100 mg, 0.24 mmol), isopropenylboronic acid (41.2 mg, 0.48 mmol), [1,1'-bis(diphenylphosphino)ferrocene]dichloropalladium dichloromethane complex (19.2 mg, 0.024 mmol) and cesium carbonate (232.8 mg, 0.72 mmol) were stirred in dioxane (4 mL) and water (1 mL) at 100°C under microwave for 1 hour. The reaction solution was concentrated to dryness by rotary evaporation, and purified by preparative high performance liquid chromatography to obtain Example 6 (54 mg, yield: 60%).

MS m/z (ESI): 379.4 [M+H]⁺.

### Example 7

### N-(5-Fluoropyridin-2-yl)-2-(5-oxo-2-(trifluoromethyl)pyrazolo[1,5-a]pyrido[3,2-e]p yrimidin-4(5H)-yl)acetamide

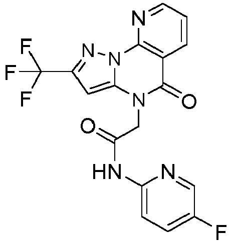

Example 7 was synthesized according to the method of Example 1. The target compound (15 mg, yield: 36%) was obtained by replacing 3-(tert-butyl)-1H-pyrazol-5-amine with 3-trifluoromethyl-1H-pyrazol-5-amine.

MS m/z (ESI): 407.3 [M+H]⁺.

¹H NMR (400 MHz, DMSO-d6) δ 11.08 (s, 1H), 8.96 (dd, J = 8.0, 1.6 Hz, 1H), 8.65 (dd, J = 8.0, 1.6 Hz, 1H), 8.37 (d, J = 3.2 Hz, 1H), 8.07 - 8.02 (m, 1H), 7.78 - 7.73 (m, 2H), 7.05 (s, 1H), 5.02 (s, 2H).

### Example 8

### 2-(2-Amino-5-oxopyrazolo[1,5-a]pyrido[3,2-e]pyrimidin-4(5H)-yl)-N-(5-fluoropyri din-2-yl)acetamide

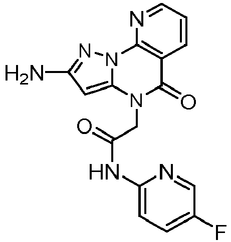

### Step 1: Preparation of methyl 5-oxo-4,5-dihydropyrazolo[1,5-a]pyrido[3,2-e]pyrimidine-2-carboxylate

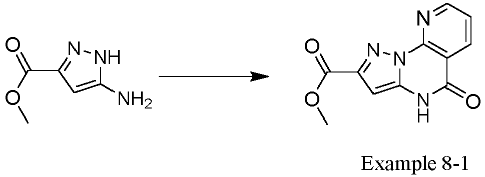

The synthetic method of Example 8-1 was according to the synthetic method of Example 1-2. Example 8-1 (500 mg, 73%) was obtained by replacing 3-(tert-butyl)-1H-pyrazol-5-amine with methyl 5-amino-1H-pyrazole-3-carboxylate.

MS: m/z (ESI): 245.2 [M+H]⁺.

### Step 2: Preparation of methyl 4-(2-((5-fluoropyridin-2-yl)amino)-2-oxoethyl)-5-oxo-4,5-dihydropyrazolo[1,5-a]pyrid o[3,2-e]pyrimidine-2-carboxylate

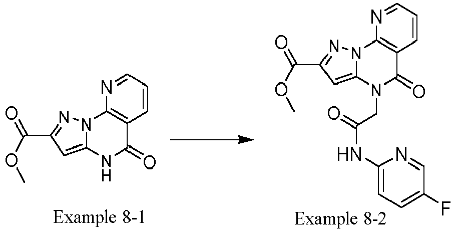

The synthetic method of Example 8-2 was according to the synthetic method of Example 1. The title compound Example 8-2 (500 mg, 51%) was obtained by using Example 8-1 as the starting material.

MS m/z (ESI): 397.3 [M+H]⁺.

### Step 3: Preparation of 4-(2-((5-fluoropyridin-2-yl)amino)-2-oxoethyl)-5-oxo-4,5-dihydropyrazolo[1,5-a]pyrid o[3,2-e]pyrimidine-2-carboxylic acid

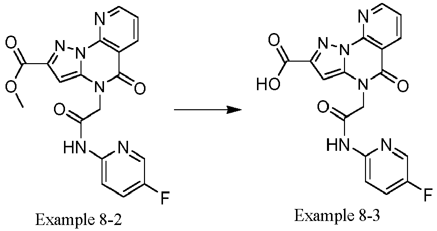

A solution of LiOH (519 mg, 12.36 mmol) in water (2 mL) was added to a solution of Example 8-2 (490 mg, 1.24 mmol) in tetrahydrofuran (10 mL) at room temperature. The mixture was stirred at room temperature for 3 hours, then the pH was adjusted to about 3 with 1M HCl. The solution was concentrated to dryness to obtain Example 8-3 (470 mg, 99%).

MS m/z (ESI): 383.3 [M+H]⁺.

### Step 4: Preparation of 2-(2-amino-5-oxopyrazolo[1,5-a]pyrido[3,2-e]pyrimidin-4(5H)-yl)-N-(5-fluoropyridin-2-yl)acetamide

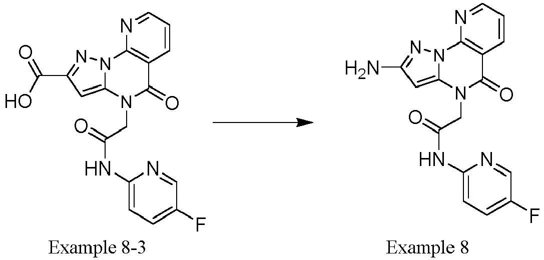

Ammonia was added to a solution of Example 8-3 (450 mg, 1.2 mmol) in 1,4-dioxane (10 mL), Et3N (33 µL, 0.24 mmol) and BOP reagent (598 mg, 1.35 mmol), and stirred at room temperature for 20 minutes. Sodium azide (160 mg, 2.46 mmol) and tetrabutylammonium bromide (786 mg, 2.46 mmol) were added, and the reaction solution was stirred for 1 hour. The reaction solution was diluted with 1,4-dioxane (12 mL), followed by addition of 2 M aqueous H₂SO₄ solution (4 mL) and heating at 100°C for 2 hours. The solvent was evaporated, and the residues were diluted with water and extracted with ethyl acetate. The organic layer was washed with brine and dried over anhydrous sodium sulfate, and the solvent was evaporated. The resulting residues were purified by column chromatography to obtain Example 8 (360 mg, 86%).

MS m/z (ESI): 354.3 [M+H]⁺.

### Example 9

### 2-(2-Cyclopropyl-5-oxopyrazolo [1,5-a] pyrido [3,2-e] pyrimidin-4(5H)-yl)-N-(5-fluor opyridin-2-yl)acetamide

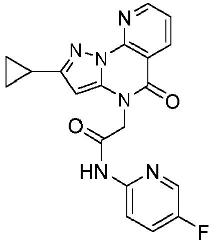

The synthetic method of Example 9 was according to the synthetic method of Example 6. The title compound Example 9 (8 mg, 51%) was obtained by replacing isopropenylboronic acid with cyclopropylboronic acid.

MS m/z (ESI): 378.4 [M+H]⁺.

### Example 10

### 2-(2-(Tert-butyl)-5-oxo-8-(trifluoromethyl)pyrazolo[1,5-a]pyrido[3,2-e]pyrimidin-4( 5H)-yl)-N-(5-fluoropyridin-2-yl)acetamide

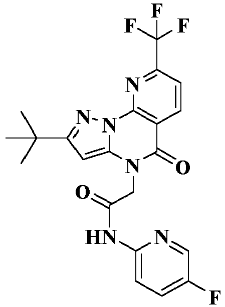

The synthetic method of Example 10 was according to the synthetic method of Example 1. The title compound Example 10 (25 mg, 46%) was obtained by using 2-chloro-6-trifluoromethylnicotinic acid as the starting material.

MS m/z (ESI): 463.1 [M+H]⁺.

¹H NMR (400 MHz, DMSO-*d*₆) δ 11.09 (s, 1H), 7.78 (d, J = 8.0 Hz, 1H), 8.38 (d, *J* = 2.8 Hz, 1H), 8.09 - 8.04 (m, 1H), 8.00 (d, *J* = 8.0 Hz, 1H), 7.79 - 7.74 (m, 1H), 6.52 (s, 1H), 4.95 (s, 2H), 1.34 (s, 9H).

### Example 11

### N-(5-Fluoropyridin-2-yl)-2-(2-methyl-5-oxo-8-(trifluoromethyl)pyrazolo[1,5-a]pyri do[3,2-e]pyrimidin-4(5H)-yl)acetamide

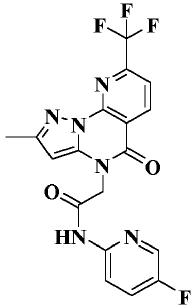

The synthetic method of Example 11 was according to the synthetic method of Example 2. The title compound Example 11 (18 mg, 30%) was obtained.

MS m/z (ESI): 421.1 [M+H]⁺.

¹H NMR (400 MHz, DMSO-d6) δ 11.06 (s, 1H), 8.80 (d, J = 8.0 Hz, 1H), 8.37 (s, 1H), 8.07 - 8.03 (m, 1H), 8.00 (d, J = 8.0 Hz, 1H), 7.79 - 7.73 (m, 1H), 6.28 (s, 1H), 4.95 (s, 2H), 2.33 (s, 3H).

### Example 12

### 2-(2-Ethyl-5-oxo-8-(trifluoromethyl)pyrazolo[1,5-a]pyrido[3,2-e]pyrimidin-4(5H)-y l)-N-(5-fluoropyridin-2-yl)acetamide

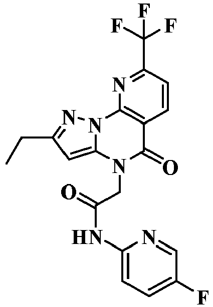

### Step 1: Preparation of tert-butyl 5-amino-3-ethyl-1H-pyrazole-1-carboxylate

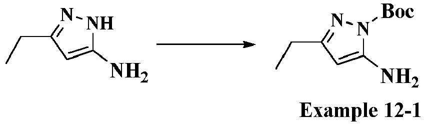

3-Ethyl-1H-pyrazol-5-amine (2.0 g, 18.0 mmol) was dissolved in anhydrous dichloromethane (50 mL), followed by addition of triethylamine (2.2 g, 21.6 mmol) and di-tert-butyl dicarbonate (4.7 g, 21.6 mmol). The reaction solution was reacted at room temperature for 16 hours. The reaction solution was washed successively with water (50 mL * 2) and saturated sodium chloride solution (50 mL), dried over anhydrous sodium sulfate and filtered. The filtrate was concentrated under reduced pressure, and the resulting crude product was purified by column chromatography (ethyl acetate/dichloromethane= 0 to 20%) to obtain the title product Example 12-1 (3.4 g), yield: 89.5%.

MS: m/z (ESI): 212.1 [M+H]⁺.

### Step 2: Preparation of tert-butyl 5-amino-3-ethyl-1H-pyrazole-1-carboxylate

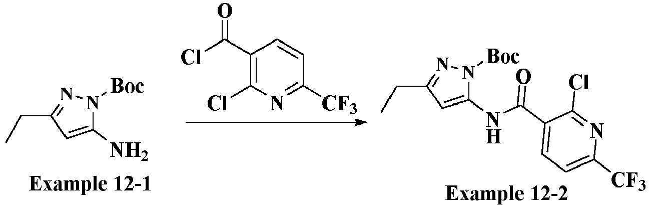

Example 12-1 (3.4 g, 16.1 mmol) was dissolved in anhydrous dichloromethane (60 mL), followed by addition of triethylamine (5.4 g, 53.1 mmol). A solution (50 mL) of freshly prepared 2-chloro-6-(trifluoromethyl)nicotinoyl chloride (4.3 g, 17.7 mmol) in dichloromethane was added dropwise under a nitrogen atmosphere at 0°C. After completion of the addition, the reaction solution was reacted at room temperature for 30 minutes. The reaction solution was washed successively with water (200 mL * 2) and saturated sodium chloride solution (200 mL), dried over anhydrous sodium sulfate and filtered. The filtrate was concentrated under reduced pressure, and the resulting crude product was purified by silica gel column chromatography (ethyl acetate/petroleum ether= 0 to 20%) to obtain Example 12-2 (2.6 g), yield: 38.2%.

MS: m/z (ESI): 319.1 [M-Boc+H]⁺.

### Step 3: Preparation of N-(3-ethyl-1H-pyrazol-5-yl)-2-chloro-6-(trifluoromethyl)nicotinamide

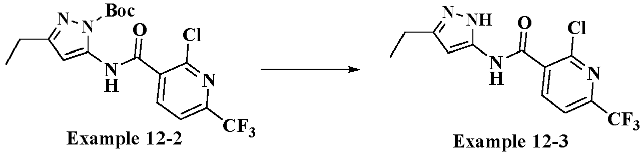

Example 12-2 (2.6 g, 6.2 mmol) was dissolved in anhydrous dichloromethane (10 mL), followed by addition of a solution (4 M, 20 mL) of hydrochloric acid in dioxane. The reaction solution was reacted at room temperature for 4 hours. The reaction solution was directly concentrated to dryness by rotary evaporation to obtain Example 12-3 (1.9 g), yield: 96.0%.

MS: m/z (ESI): 319.0 [M+H]⁺.

### Step 4: 2-Ethyl-8-(trifluoromethyl)pyrazolo[1,5-a]pyrido[3,2-e]pyrimidin-5(4H)-one

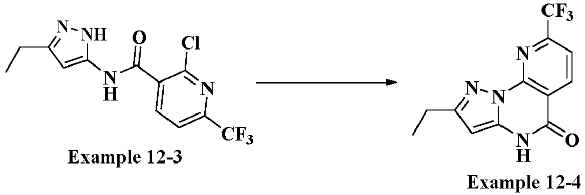

Example 12-3 (1.9 g, 6.0 mmol) was dissolved in N,N-dimethylformamide (20 mL), followed by addition of potassium carbonate (2.5 g, 18.0 mmol). The reaction solution was heated to 120°C and reacted for 2 hours. The reaction solution was cooled to room temperature and used directly in the next step.

MS: m/z (ESI): 283.1[M+H]⁺.

### Step 5: 2-(2-Ethyl-5-oxo-8-(trifluoromethyl)pyrazolo[1,5-a]pyrido[3,2-e]pyrimidin-4(5H)-yl)-N -(5-fluoropyridin-2-yl)acetamide

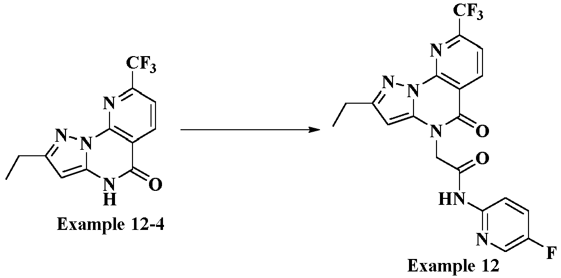

Potassium carbonate (1.5 g, 10.6 mmol) and 2-bromo-N-(5-fluoropyridin-2-yl)acetamide (0.99 g, 4.2 mmol) were added to the reaction solution of Example 12-4 (1.0 g, 3.5 mmol) in N,N-dimethylformamide (20 mL), and reacted at 40°C for 2 hours. The reaction solution was cooled to room temperature, poured into 300 mL of water, and extracted with ethyl acetate (200 mL * 3). The organic phases were combined, washed successively with water (200 mL * 2) and saturated sodium chloride solution (200 mL), dried over anhydrous sodium sulfate and filtered. The filtrate was concentrated under reduced pressure, and the resulting crude product was recrystallized from ethyl acetate to obtain Example 12.

¹H NMR (400 MHz, DMSO-d₆) δ 11.06 (s, 1H), 8.79 (d, *J* = 7.6 Hz, 1H), 8.37 (s, 1H), 8.07 - 8.03 (m, 1H), 8.00 (d, *J* = 8.0 Hz, 1H), 7.79 - 7.72 (m, 1H), 6.36 (s, 1H), 4.96 (s, 2H), 2.70 (q, *J* = 7.6 Hz, 2H), 1.25 (t, *J* = 7.6 Hz, 3H).

MS m/z (ESI): 435.1 [M+H]⁺.

### Example 13

### 2-(2-Cyclopropyl-5-oxo-8-(trifluoromethyl)pyrazolo [1,5-a] pyrido [3,2-e] pyrimidin-4(5H)-yl)-N-(5-fluoropyridin-2-yl)acetamide

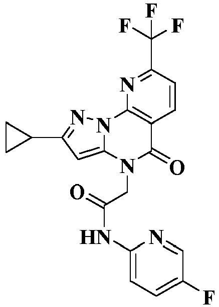

The synthetic method of Example 13 was according to the synthetic method of Example 1. The title compound Example 13 (17 mg, 28%) was obtained.

¹H NMR (400 MHz, DMSO-d6) δ 11.05 (s, 1H), 8.78 (d, J = 8.0 Hz, 1H), 8.37 (s, 1H), 8.08 - 8.02 (m, 1H), 7.98 (d, J = 8.4 Hz, 1H), 7.79 - 7.73 (m, 1H), 6.23 (s, 1H), 4.91 (s, 2H), 2.11 - 2.04 (m, 1H), 1.04 - 0.98 (m, 2H), 0.82 - 0.78 (m, 2H).

MS m/z (ESI): 447.1 [M+H]⁺.

### Example 14

### N-(5-Fluoropyridin-2-yl)-2-(2-isopropyl-5-oxo-8-(trifluoromethyl)pyrazolo[1,5-a]p yrido[3,2-e]pyrimidin-4(5H)-yl)acetamide

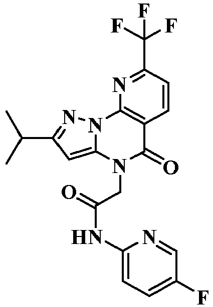

The synthetic method of Example 14 was according to the synthetic method of Example 4. The title compound Example 31 (10 mg, 22%) was obtained.

MS m/z (ESI): 449.1 [M+H]⁺.

¹H NMR (400 MHz, DMSO-d6) δ 11.06 (s, 1H), 8.79 (d, J = 8.0 Hz, 1H), 8.37 (s, 1H), 8.09 - 8.03 (m, 1H), 8.00 (d, J = 8.0 Hz, 1H), 7.79 - 7.74 (m, 1H), 6.42 (s, 1H), 4.96 (s, 2H), 3.08 - 3.01 (m, 1H), 1.29 (s, 3H), 1.27 (s, 3H).

### Example 15

### Preparation of 2-(2-bromo-5-oxo-8-(trifluoromethyl)pyrazolo[1,5-a]pyrido[3,2-e]pyrimidin-4(5H)-yl)-N-(5-fluoropyridin-2-yl)acetamide

### Step 1: Preparation of tert-butyl 5-amino-3-bromo-1H-pyrazole-1-carboxylate

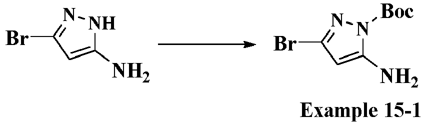

3-Bromo-1H-pyrazol-5-amine (10.0 g, 61.7 mmol) was dissolved in anhydrous dichloromethane (100 mL), followed by addition of triethylamine (7.48 g, 74.1 mmol) and di-*tert*-butyl dicarbonate (16.0 g, 74.1 mmol). The reaction solution was reacted at room temperature for 16 hours. The reaction solution was washed successively with water (50 mL * 2) and saturated sodium chloride solution (50 mL), dried over anhydrous sodium sulfate and filtered. The filtrate was concentrated under reduced pressure, and the resulting crude product was purified by column chromatography (ethyl acetate/dichloromethane = 0 to 20%) to obtain the title product *tert*-butyl 5-amino-3-bromo-1H-pyrazole-1-carboxylate Example 15-1 (14.5 g), yield: 89.5%.

MS: m/z (ESI): 262.0 [M+H]⁺

¹H NMR (400 MHz, DMSO-d6) δ 6.62 (s, 2H), 5.41 (s, 1H), 1.56 (s, 9H).

### Step 2: Preparation of tert-butyl 5-amino-3-bromo-1H-pyrazole-1-carboxylate

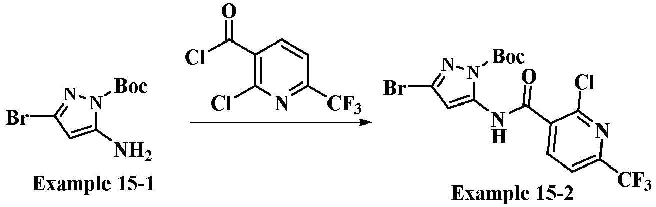

*Tert*-butyl 5-amino-3-bromo-1H-pyrazole-1-carboxylate Example 15-1 (14.5 g, 55.3 mmol) was dissolved in anhydrous dichloromethane (200 mL), followed by addition of triethylamine (18.5 g, 183 mmol). A solution (50 mL) of freshly prepared 2-chloro-6-(trifluoromethyl)nicotinoyl chloride (13.0 g, 61.0 mmol) in dichloromethane was added dropwise under a nitrogen atmosphere at 0°C. After completion of the addition, the reaction solution was reacted at room temperature for 30 minutes. The reaction solution was washed successively with water (200 mL * 2) and saturated sodium chloride solution (200 mL), dried over anhydrous sodium sulfate and filtered. The filtrate was concentrated under reduced pressure, and the resulting crude product was purified by silica gel column chromatography (ethyl acetate/petroleum ether = 0 to 20%) to obtain *tert*-butyl 5-amino-3-bromo-1H-pyrazole-1-carboxylate Example 15-2 (9.5 g), yield: 38.2%.

MS: m/z (ESI): 371.0 [M-Boc+H] ⁺

¹H NMR (400 MHz, DMSO-d6) δ 11.08 (s, 1H), 8.40 (d, J = 7.6 Hz, 1H), 8.14 (d, J = 7.6 Hz, 1H), 6.96 (s, 1H), 1.58 (s, 9H).

### Step 3: Preparation of N-(3-bromo-1H-pyrazol-5-yl)-2-chloro-6-(trifluoromethyl)nicotinamide

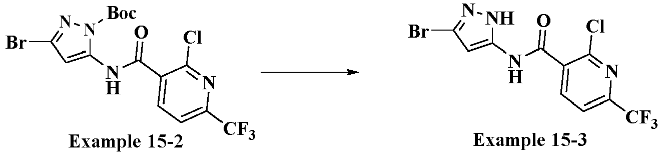

*Tert*-butyl 5-amino-3-bromo-1H-pyrazole-1-carboxylate Example 51-2 (8.0 g, 17.1 mmol) was dissolved in anhydrous dichloromethane (20 mL), followed by addition of a solution (4 M, 40 mL) of hydrochloric acid in dioxane. The reaction solution was reacted at room temperature for 4 hours. The reaction solution was directly concentrated to dryness by rotary evaporation to obtain N-(3-bromo-1H-pyrazol-5-yl)-2-chloro-6-(trifluoromethyl)nicotinamide Example 15-3 (6.2 g), yield: 98.4%.

MS: m/z (ESI): 368.9 [M+H]⁺

¹H NMR (400 MHz, DMSO-d6) δ 11.50 (s, 1H), 8.39 (d, J = 7.6 Hz, 1H), 8.10 (d, J = 7.6 Hz, 1H), 6.53 (s, 1H).

### Step 4: Preparation of 2-bromo-8-(trifluoromethyl)pyrazolo[1,5-a]pyrido[3,2-e]pyrimidin-5(4H)-one

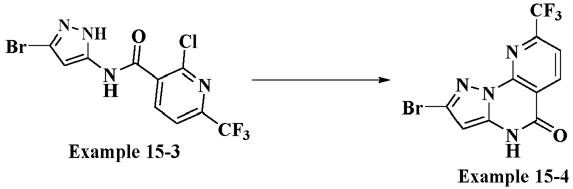

N-(3-Bromo-1H-pyrazol-5-yl)-2-chloro-6-(trifluoromethyl)nicotinamide Example 15-3 (6.2 g, 16.8 mmol) was dissolved in N,N-dimethylformamide (80 mL), followed by addition of potassium carbonate (6.96 g, 50.4 mmol). The reaction solution was heated to 120°C and reacted for 2 hours. The reaction solution was cooled to room temperature and used directly in the next step.

MS: m/z (ESI): 333.0 [M+H]⁺.

### Step 5: Preparation of 2-(2-bromo-5-oxo-8-(trifluoromethyl)pyrazolo[1,5-a]pyrido[3,2-e]pyrimidin-4(5H)-yl)-N-(5-fluoropyridin-2-yl)acetamide

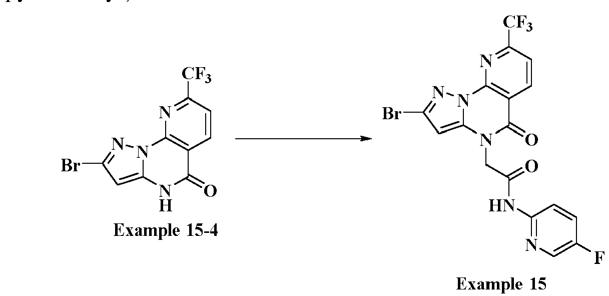

Potassium carbonate (6.96 g, 50.4 mmol) and 2-bromo-N-(5-fluoropyridin-2-yl)acetamide (4.7 g, 20.2 mmol) were added to the reaction solution of 2-bromo-8-(trifluoromethyl)pyrazolo[1,5-a]pyrido[3,2-e]pyrimidin-5(4H)-one Example 51-4 (NA, 16.8 mmol) in N,N-dimethylformamide (80 mL), and reacted at 40°C for 2 hours. The reaction solution was cooled to room temperature, poured into 300 mL of water, and extracted with ethyl acetate (200 mL * 3). The organic phases were combined, washed successively with water (200 mL * 2) and saturated sodium chloride solution (200 mL), dried over anhydrous sodium sulfate and filtered. The filtrate was concentrated under reduced pressure, and the resulting crude product was recrystallized from ethyl acetate to obtain the title product 2-(2-bromo-5-oxo-8-(trifluoromethyl)pyrazolo[1,5-a]pyrido[3,2-e]pyrimidin-4(5H)-yl)-N-(5-fluoropyridin-2-yl)acetamide Example 15.

¹H NMR (400 MHz, DMSO-d6) δ 11.05 (s, 1H), 8.84 (d, J = 8.0 Hz, 1H), 8.37 (s, 1H), 8.09 (d, J = 8.0 Hz, 1H), 8.07 - 8.02 (m, 1H), 7.80 - 7.73 (m, 1H), 6.78 (s, 1H), 4.96 (s, 2H).

MS m/z (ESI): 486.2 [M+H]⁺.

### Example 16

### N-(5-Fluoropyridin-2-yl)-2-(5-oxo-2,8-bis(trifluoromethyl)pyrazolo[1,5-a]pyrido[3, 2-e]pyrimidin-4(5H)-yl)acetamide

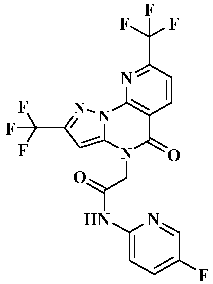

The synthetic method of Example 16 was according to the synthetic method of Example 1. The title compound Example 16 (10 mg, 33%) was obtained.

MS m/z (ESI): 475.3 [M+H]⁺.

¹H NMR (400 MHz, DMSO-*d*₆) δ 11.07 (s, 1H), 8.91 (d, J = 8.0 Hz, 1H), 8.37 (d, J = 3.1 Hz, 1H), 8.19 (d, J = 8.1 Hz, 1H), 8.14 - 7.98 (m, 1H), 7.76 (t, J = 8.8 Hz, 1H), 7.13 (s, 1H), 5.04 (s, 2H).

### Example 17

### 2-(2-Cyano-5-oxo-8-(trifluoromethyl)pyrazolo[1,5-a]pyrido[3,2-e]pyrimidin-4(5H)-yl)-N-(5-fluoropyridin-2-yl)acetamide

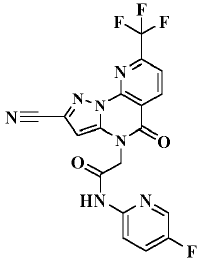

### Step 1: Preparation of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide

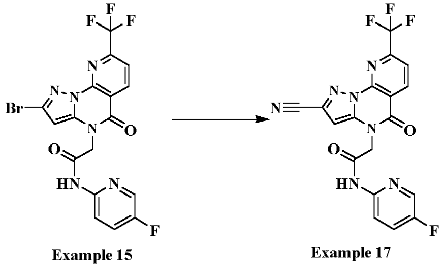

Example 15 (300 mg, 0.619 mmol), Zn(CN)₂ (300 mg, 2.56 mmol), Pd₂(dba)₃ (20 mg, 0.022 mmol), Pd(dppf)Cl₂ (30 mg, 0.036 mmol) and Zn powder (10 mg, 0.154 mmol) were dissolved in DMA (10 mL) at room temperature, followed by purging nitrogen for 2 minutes. The reaction solution was heated by microwave to 140 degrees and reacted for 8 hours. The reaction solution was cooled to room temperature and extracted with ethyl acetate (50 mL). The organic phase was washed twice with saturated brine. The organic phase was dried (Na₂SO₄), concentrated under reduced pressure, and purified by p-HPLC (FA) to obtain 100 mg of the title compound (yield: 38%).

¹H NMR (400 MHz, DMSO-d6) δ 11.07 (s, 1H), 8.92 (d, J = 8.2 Hz, 1H), 8.37 (d, J = 3.1 Hz, 1H), 8.22 (d, J = 7.9 Hz, 1H), 8.05 (s, 1H), 7.77 (t, J = 8.6 Hz, 1H), 7.24 (s, 1H), 5.01 (s, 2H).

MS m/z (ESI): 432.3 [M+H]⁺.

The title compound can also be prepared by the following method:

### Step 1: Preparation of ethyl 5-oxo-8-(trifluoromethyl)-4,5-dihydropyrazolo[1,5-a]pyrido[3,2-e]pyrimidine-2-carbox ylate

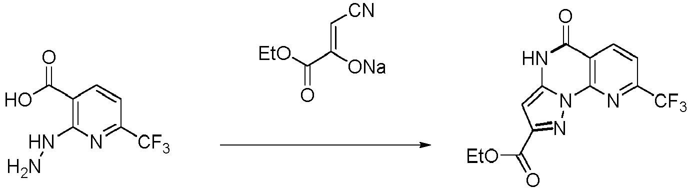

Sodium ethyl cyanopyruvate (17.7 g, 0.108 mol) and dioxane (200 ml) were added to a reaction flask under stirring and a nitrogen atmosphere. The solids were insoluble. A 4 M/L solution (68 ml, 0.27 mol) of hydrochloric acid in dioxane was added at 20 to 25°C, and stirred for 15 to 30 minutes. The starting material 2-hydrazineyl-6-(trifluoromethyl)nicotinic acid (20 g, 0.09 mol) was added, and the reaction solution was heated to 105 to 110°C in an oil bath and reacted for 24 hours. 400 ml of water (1.5 V relative to the organic solvent) was added dropwise, and a brown solid was precipitated. The mixture was filtered, and the filter cake was washed with 400 ml of water. The resulting crude product was added to methanol (100 ml), stirred at room temperature for 2 hours, and filtered to obtain a white solid, m = 12.5 g, yield: 40.3%, HPLC purity: 99.2%.

MS (ESI) m/z: 327.24 [M+1]⁺.

¹H NMR (400 MHz, DMSO-*d*₆): *δ*/ppm 12.68 (s, 1H), 8.81 (d, J = 8.0 Hz, 1H), 8.11 (d, J = 8.0 Hz, 1H), 6.30 (s, 1H), 4.35-4.40 (m, 2H), 1.34-1.38 (m, 3H).

Ethyl 5-oxo-8-(trifluoromethyl)-4,5-dihydropyrazolo[1,5-a]pyrido[3,2-e]pyrimidine-2-carbox ylate can also be prepared by the following method:
Sodium ethyl cyanopyruvate (35.4 g, 0.22 mol) and N,N-dimethylformamide (400 ml) were added to a reaction flask under stirring and a nitrogen atmosphere. The solids were insoluble. The temperature was kept at 0 to 10°C, and a 4 M/L solution (135 ml, 0.54 mol) of hydrochloric acid in dioxane followed by the starting material 2-hydrazineyl-6-(trifluoromethyl)nicotinic acid (40 g, 0.18 mol) were added. The reaction solution was heated to 105 to 110°C in an oil bath and reacted for 24 hours. 800 ml of water (1.5 V relative to the organic solvent) was added dropwise, and a brown solid was precipitated. The mixture was filtered, and the filter cake was washed with 800 ml of water. The resulting crude product was added to methanol (200 ml), stirred at room temperature for 2 hours, and filtered to obtain a white solid, m = 33 g, yield: 56.2%, HPLC purity: 99.4%.

MS (ESI) m/z: 327.24 [M+1]⁺.

¹H NMR (400 MHz, DMSO-*d*₆): *δ*/ppm 12.68 (s, 1H), 8.81 (d, J = 8.0 Hz, 1H), 8.11 (d, J = 8.0 Hz, 1H), 6.30 (s, 1H), 4.35-4.40 (m, 2H), 1.34-1.38 (m, 3H).

### Step 2: Preparation of 2-carboxamide-5-oxo-8-(trifluoromethyl)-4,5-dihydropyrazolo[1,5-a]pyrido[3,2-e]pyri midine

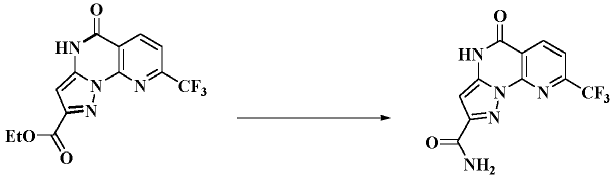

At 25°C, ethyl 5-oxo-8-(trifluoromethyl)-4,5-dihydropyrazolo[1,5-a]pyrido[3,2-e]pyrimidine-2-carbox ylate (11.5 g, 35.3 mmol) and aqueous ammonia (120 ml) were added to a 500 ml single-neck flask under stirring, and the yellow solids were insoluble. The mixture was stirred at room temperature for 48 to 60 hours, and the raw materials were reacted completely. The reaction solution was concentrated under reduced pressure, and pulped with 120 ml of methanol for 1 hour. The solution was cooled to 0~5°C in an ice water bath, stirred for 30 minutes, and filtered under reduced pressure. The filter cake was rinsed with a small amount of ice-cold methanol to obtain a light yellow solid, which was dried under reduced pressure to constant weight, mass m = 8.9 g, yield: 85.0%, HPLC purity: 98.6%.

MS (ESI) m/z: 298.20 [M+1]⁺.

¹H NMR (400 MHz, DMSO-*d*₆): *δ*/ppm 11.68 (s, 1H), 8.74 (d, J = 8.0 Hz, 1H), 8.01 (d, J = 8.0 Hz, 1H), 7.81 (s, 1H) ,7.46 (s, 1H), 6.18 (s, 1H).

2-Carboxamide-5-oxo-8-(trifluoromethyl)-4,5-dihydropyrazolo[1,5-a]pyrido[3,2-e] pyrimidine can also be prepared by the following method:
At 25°C, ethyl 5-oxo-8-(trifluoromethyl)-4,5-dihydropyrazolo[1,5-a]pyrido[3,2-e]pyrimidine-2-carbox ylate (11.5 g, 35.3 mmol), ammonia in methanol (7 M/L, 115 ml) and aqueous ammonia (58 ml) were added to a 500 ml single-neck flask under stirring, and the yellow solids were insoluble. The mixture was stirred at room temperature for 48 to 60 hours, and the raw materials were reacted completely. The reaction solution was concentrated under reduced pressure, and pulped with 120 ml of methanol for 1 hour. The solution was cooled to 0~5°C in an ice water bath, stirred for 30 minutes, and filtered under reduced pressure. The filter cake was rinsed with a small amount of ice-cold methanol to obtain a light yellow solid, which was dried under reduced pressure to constant weight, mass m = 9.1 g, yield: 87.0%, HPLC purity: 99.0%.

MS (ESI) m/z: 298.20 [M+1]⁺.

¹H NMR (400 MHz, DMSO-*d*₆): *δ*/ppm 11.68 (s, 1H), 8.74 (d, J = 8.0 Hz, 1H), 8.01 (d, J = 8.0 Hz, 1H), 7.81 (s, 1H) ,7.46 (s, 1H), 6.18 (s, 1H).

### Step 3: Preparation of 2-cyano-5-oxo-8-(trifluoromethyl)-4,5-dihydropyrazolo[1,5-a]pyrido[3,2-e]pyrimidine

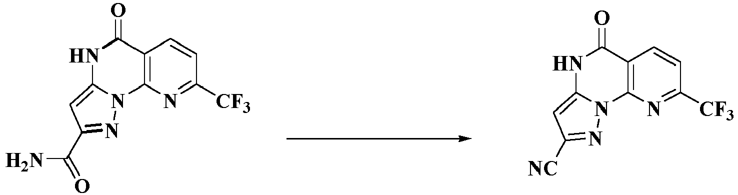

2-Carboxamide-5-oxo-8-(trifluoromethyl)-4,5-dihydropyrazolo[1,5-a]pyrido[3,2-e] pyrimidine (6.2 g, 0.021 mol) and N,N-dimethylformamide (62 ml) were added to a 250 ml reaction flask under stirring and a nitrogen atmosphere, followed by addition of pyridine (4.9 g, 0.062 mol). The reaction solution was cooled in an ice-water bath, and kept at 0°C to 5°C. Phosphorus oxychloride (8 g, 0.052 mol) was added dropwise, and the reaction system turned into a gray turbid liquid. After the addition was completed, the ice-water bath was removed. The reaction solution was warmed to room temperature and reacted for 1 to 2 hours, and the raw materials were completely reacted. The reaction solution was cooled in an ice-water bath, followed by addition of 186 ml of water. The solution was stirred for 20 to 30 minutes, and filtered under reduced pressure. The filter cake was washed with water to obtain an off-white solid. The wet product was transferred to a 250 ml single-neck flask, dissolved well in 60 ml of N,N-dimethylformamide, and filtered to remove mechanical impurities. 180 ml of water was added dropwise. The solution was cooled in an ice-water bath, and stirred for 30 minutes. The mixture was filtered under reduced pressure to obtain an off-white solid, which was dried under vacuum to constant weight, mass m = 4.7 g, yield: 80.7%, HPLC purity: 99.0%.

MS (ESI) m/z: 280.18 [M+1]⁺.

¹H NMR (400 MHz, DMSO-*d*₆): *δ*/ppm 12.86 (s, 1H), 8.82 (d, J = 8.0 Hz, 1H), 8.15 (d, J = 8.0 Hz, 1H), 6.67 (s, 1H).

2-Cyano-5-oxo-8-(trifluoromethyl)-4,5-dihydropyrazolo[1,5-a]pyrido[3,2-e]pyrimi dine can also be prepared by the following method:
2-Carboxamide-5-oxo-8-(trifluoromethyl)-4,5-dihydropyrazolo[1,5-a]pyrido[3,2-e] pyrimidine (15 g, 0.05 mol) and tetrahydrofuran (150 ml) were added to a 500 ml reaction flask under stirring and a nitrogen atmosphere, followed by addition of pyridine (11.9 g, 0.15 mol). The reaction solution was cooled in an ice-water bath, and kept at 0°C to 5°C. Trifluoroacetic anhydride (26.3 g, 0.125 mol) was added dropwise, and the reaction system turned into a gray turbid liquid. After the addition was completed, the ice-water bath was removed. The reaction solution was warmed to room temperature and reacted for 1 to 2 hours, and the raw materials were completely reacted. The reaction solution was cooled in an ice-water bath, followed by addition of 450 ml of water. The solution was stirred for 20 to 30 minutes, and filtered under reduced pressure. The filter cake was washed with water to obtain an off-white solid. The wet product was transferred to a 500 ml single-neck flask, dissolved well in 145 ml of N,N-dimethylformamide, and filtered to remove mechanical impurities. 435 ml of water was added dropwise. The solution was cooled in an ice-water bath, and stirred for 30 minutes. The mixture was filtered under reduced pressure to obtain an off-white solid, which was dried under vacuum to constant weight, mass m = 12.2 g, yield: 87.1%, HPLC purity: 99.0%.

MS (ESI) m/z: 280.18 [M+1]⁺.

¹H NMR (400 MHz, DMSO-*d*₆): *δ*/ppm 12.86 (s, 1H), 8.82 (d, J = 8.0 Hz, 1H), 8.15 (d, J = 8.0 Hz, 1H), 6.67 (s, 1H).

### Step 4: Preparation of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide

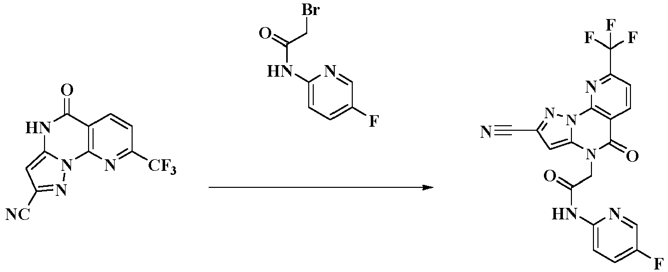

2-Cyano-5-oxo-8-(trifluoromethyl)-4,5-dihydropyrazolo[1,5-a]pyrido[3,2-e]pyrimi dine (6.0 g, 0.0215 mol) and N,N-dimethylformamide (60 mL) were added to a 500 ml three-necked flask and stirred to dissolve, followed by addition of potassium carbonate (5.9 g, 0.043 mol). 2-Bromo-N-(5-fluoropyridin-2-yl)acetamide (5.0 g, 0.0215 mol) was dissolved in DMF (30 ml), and added dropwise to the reaction system. The reaction solution was warmed to 45°C and stirred for 1 to 2 hours, and the raw materials were completely reacted. The heating was turned off, and the reaction solution was cooled to room temperature. Water (135 ml) was added, and a large amount of solid was precipitated and was stirred for 30 minutes. The mixture was filtered under reduced pressure, and the filter cake was washed twice with water. The crude product was dissolved in DCM/acetone = 3:1 (250 ml), followed by addition of activated carbon (0.5 g) and anhydrous sodium sulfate (15 g), and stirring at 40°C for 30 minutes. The mixture was filtered. The filtrate was concentrated to dryness under reduced pressure, dissolved in 50 ml of acetone, and concentrated to dryness. 100 ml of acetone was added, and the solution was heated to 65°C to reflux, and the solid was still not dissolved well. 200 ml of n-heptane was added dropwise, and the solution was heated and stirred for 1 hour. The heating was turned off, and the solution was cooled to room temperature naturally, and stirred for 1 hour. The mixture was filtered to obtain an off-white solid, which was dried under vacuum to constant weight, mass m = 8.0 g, yield: 86.4%, HPLC purity: 99.2%.

MS (ESI) m/z: 432.31 [M+1]⁺.

¹H NMR (400 MHz, DMSO-*d*₆): *δ*/ppm 11.06 (s, 1H), 8.92 (d, J = 8.0 Hz, 1H), 8.36 (d, J = 4.0 Hz, 1H), 8.22 (d, J = 8.0 Hz, 1H), 8.05 (m, 1H). 7.76 (m, 1H), 7.24 (s, 1H), 5.01 (m, 2H).

### Example 18

### N-(5-Fluoropyridin-2-yl)-2-(5-oxo-8-(trifluoromethyl)pyrazolo[1,5-a]pyrido[3,2-e]p yrimidin-4(5H)-yl)acetamide

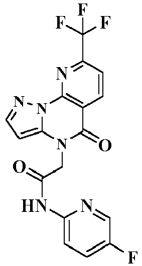

### Step 1: Preparation of N-(1H-pyrazol-5-yl)-2-chloro-nicotinamide

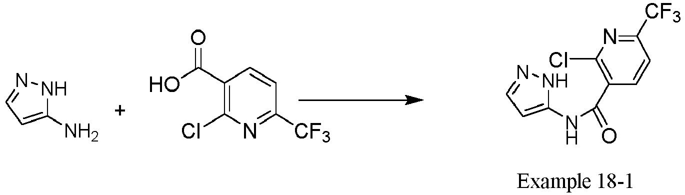

1H-Pyrazol-5-amine (1.66 g, 19.93 mmol), DIPEA (6.2 g, 49.8 mmol) and HATU (5.4 g, 0.144 mmol) were added successively to a solution of 2-chloronicotinic acid (1.57 g, 9.96 mmol) in DMF (30 mL) under an ice bath condition. The ice bath was removed, and the reaction solution was stirred for 1 hour. The mixture was treated to obtain Example 18-1 (2.0 g, 90%).

MS m/z (ESI): 291.0 [M+H]⁺.

### Step 2: Preparation of pyrazolo[1,5-a]pyrido[3,2-e]pyrimidin-5(4H)-one

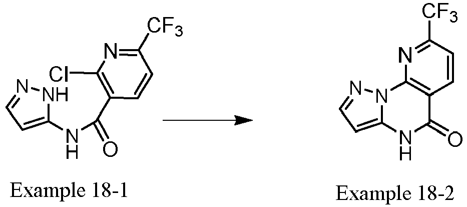

Potassium carbonate (1.61 g, 11.66 mmol) and 1,4-diazabicyclo[2.2.2]octane (DABCO) (150.9 mg, 1.35 mmol) were added to a solution of Example 18-1 (2.0 g, 8.97 mmol) in DMF (50 mL). The reaction solution was stirred at room temperature for 16 hours. The mixture was treated to obtain Example 18-2 (1.6 g, 97%).

MS m/z (ESI): 255.0[M+H]⁺.

### Step 3: Preparation of 2-(2-(tert-butyl)-5-oxopyrazolo[1,5-a]pyrido[3,2-e]pyrimidin-4(5H)-yl)-N-(5-fluoropyri din-2-yl)acetamide

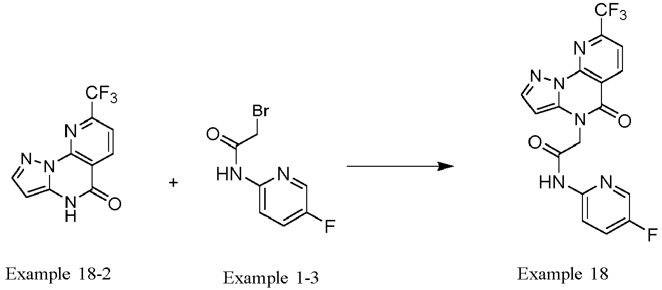

Potassium carbonate (2.23 g, 16.11 mmol) and Example 1-3 (2.25 g, 9.67 mmol) were added to a solution of Example 18-2 (1.5 g, 8.06 mmol) in DMF (30 mL) at room temperature. The mixture was heated to 80°C and stirred for 2 hours. The reaction solution was cooled followed by addition of water. The precipitate was filtered, washed with ethyl acetate, and purified to obtain Example 18 (2.1 g, yield: 78%).

¹H NMR (400 MHz, DMSO-d6) δ 11.06 (s, 1H), 8.83 (d, J = 8.0 Hz, 1H), 8.37 (d, J = 3.2 Hz, 1H), 8.06 (d, J = 8.0 Hz, 1H), 8.05 - 8.02 (m, 1H), 7.98 (d, J = 2.0 Hz, 1H), 7.78 - 7.73 (m, 1H), 6.46 (s, 1H), 5.00 (s, 2H).

MS m/z (ESI): 407.3 [M+H]⁺.

### Example 19

### 2-(2-Chloro-5-oxo-8-(trifluoromethyl)pyrazolo[1,5-a]pyrido[3,2-e]pyrimidin-4(5H) -yl)-N-(5-fluoropyridin-2-yl)acetamide

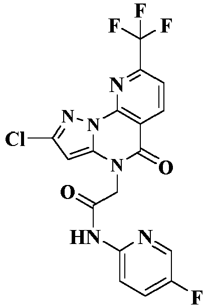

Example 19 was synthesized according to the method of Example 1. The target compound (31 mg, yield: 26%) was obtained by replacing 3-(tert-butyl)-1H-pyrazol-5-amine with 3-chloro-1H-pyrazol-5-amine.

¹H NMR (400 MHz, DMSO-d6) δ 11.05 (s, 1H), 8.84 (d, J = 8.1 Hz, 1H), 8.36 (s, 1H), 8.15 - 7.99 (m, 2H), 7.76 (t, J = 9.0 Hz, 1H), 6.73 (s, 1H), 4.96 (s, 2H).

MS m/z (ESI): 441.7 [M+H]⁺.

### Example 20

### 2-(3-Cyano-5-oxo-8-(trifluoromethyl)pyrazolo[1,5-a]pyrido[3,2-e]pyrimidin-4(5H)-yl)-N-(5-fluoropyridin-2-yl)acetamide

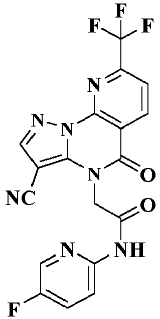

Example 20 was synthesized according to the method of Example 1. The target compound was obtained by replacing 3-(tert-butyl)-1H-pyrazol-5-amine with 4-cyano-1H-pyrazol-5-amine.

### Step 1: Preparation of tert-butyl 5-amino-4-cyano-1H-pyrazole-1-carboxylate

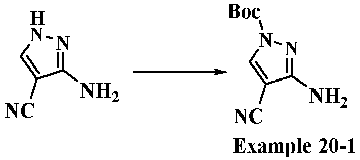

5-Amino-1H-pyrazole-4-carbonitrile (2.0 g, 18.5 mmol) was dissolved in anhydrous dichloromethane (40 mL), followed by addition of triethylamine (3.74 g, 37.0 mmol) and di-*tert*-butyl dicarbonate (4.44 g, 20.4 mmol). The reaction solution was reacted at room temperature for 16 hours. The reaction solution was concentrated under reduced pressure, and made slurry in petroleum ether (50 mL) to obtain the title product *tert*-butyl 5-amino-4-cyano-1H-pyrazole-1-carboxylate Example 20-1 (3.5 g), yield: 90.9%.

¹H NMR (400 MHz, DMSO-d6) δ 7.77 (s, 1H), 7.63 (s, 2H), 1.56 (s, 9H).

### Step 2: Preparation of tert-butyl 5-(2-chloro-6-(trifluoromethyl)nicotinamido)-4-cyano-1H-pyrazole-1-carboxylate

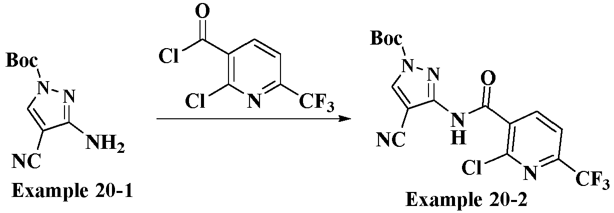

*Tert*-butyl 5-amino-4-cyano-1H-pyrazole-1-carboxylate Example 20-1 (3.5 g, 16.8 mmol) was dissolved in anhydrous dichloromethane (50 mL), followed by addition of triethylamine (5.35 g, 7.37 mmol). A solution (50 mL) of freshly prepared 2-chloro-6-(trifluoromethyl)nicotinoyl chloride (4.3 g, 17.6 mmol) in dichloromethane was added dropwise under a nitrogen atmosphere at 0°C. After completion of the addition, the reaction solution was reacted at room temperature for 1 hour. The reaction solution was washed successively with water (50 mL * 2) and saturated sodium chloride solution (50 mL), dried over anhydrous sodium sulfate and filtered. The filtrate was concentrated under reduced pressure, and the resulting crude product was purified by silica gel column chromatography (ethyl acetate/ petroleum ether= 0 to 40%) to obtain *tert*-butyl 5-(2-chloro-6-(trifluoromethyl)nicotinamido)-4-cyano-1H-pyrazole-1-carboxylate Example 20-2 (2.8 g), yield: 38.2%.
MS: m/z (ESI): 432.8 [M+NH₄] ⁺
¹H NMR (400 MHz, DMSO-d6) δ 11.87 (s, 1H), 9.23 (s, 1H), 8.43 (d, J = 7.6 Hz, 1H), 8.13 (d, J = 7.6 Hz, 1H), 1.59 (s, 9H).

### Step 3: Preparation of 2-chloro-N-(4-cyano-1H-pyrazol-5-yl)-6-(trifluoromethyl)nicotinamide

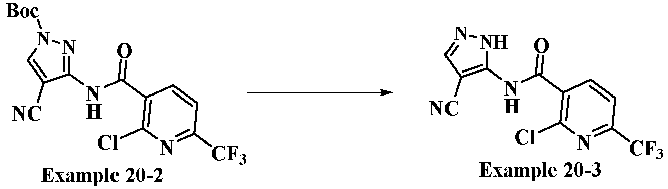

*Tert*-butyl 5-(2-chloro-6-(trifluoromethyl)nicotinamido)-4-cyano-1H-pyrazole-1-carboxylate Example 20-2 (2.8 g, 6.73 mmol) was dissolved in anhydrous dichloromethane (10 mL), followed by addition of a solution (4 M, 30 mL) of hydrochloric acid in dioxane. The reaction solution was reacted at room temperature for 5 hours. The reaction solution was directly concentrated to dryness by rotary evaporation to obtain 2-chloro-N-(4-cyano-1H-pyrazol-5-yl)-6-(trifluoromethyl)nicotinamide Example 20-3 (2.1 g), yield: 98.8%.
MS: m/z (ESI): 315.8 [M+H] ⁺

### Step 4: Preparation of 5-oxo-8-(trifluoromethyl)-4,5-dihydropyrazolo[1,5-a]pyrido[3,2-e]pyrimidine-3-carboni trile

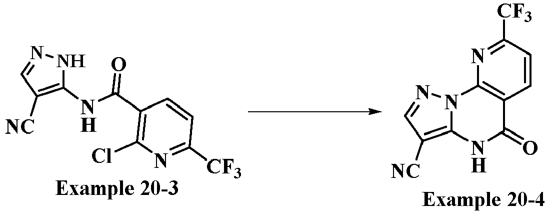

2-Chloro-N-(4-cyano-1H-pyrazol-5-yl)-6-(trifluoromethyl)nicotinamide Example 20-3 (2.1 g, 6.65 mmol) was dissolved in N,N-dimethylformamide (40 mL), followed by addition of potassium carbonate (1.84 g, 13.3 mmol). The reaction solution was heated to 120°C and reacted for 2 hours. The reaction solution was cooled to room temperature, adjusted to pH 5 to 6 with 1M dilute hydrochloric acid, and extracted with ethyl acetate (100 mL * 2). The organic phases were combined, washed successively with water (100 mL * 2) and saturated sodium chloride solution (100 mL), dried over anhydrous sodium sulfate and filtered. The filtrate was concentrated under reduced pressure, and made slurry in ethyl acetate (15 mL) to obtain 5-oxo-8-(trifluoromethyl)-4,5-dihydropyrazolo[1,5-a]pyrido[3,2-e]pyrimidine-3-carboni trile Example 20-4 (1.3 g), yield: 69.9%.

MS: m/z (ESI): 279.8 [M+H] ⁺.

### Step 4: Preparation of 2-(3-cyano-5-oxo-8-(trifluoromethyl)pyrazolo[1,5-a]pyrido[3,2-e]pyrimidin-4(5H)-yl)-N-(5-fluoropyridin-2-yl)acetamide

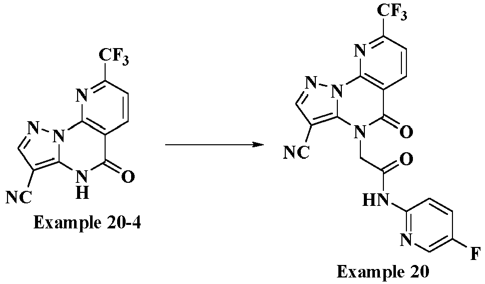

5-Oxo-8-(trifluoromethyl)-4,5-dihydropyrazolo[1,5-a]pyrido[3,2-e]pyrimidine-3-c arbonitrile Example 20-4 (500 mg, 1.79 mmol) was dissolved in N,N-dimethylformamide (20 mL), followed by addition of potassium carbonate (371 mg, 2.69 mmol) and 2-bromo-N-(5-fluoropyridin-2-yl)acetamide (501 mg, 2.15 mmol). The reaction solution was reacted at 40°C for 2 hours. The reaction solution was cooled to room temperature, poured into 100 mL of water, and extracted with ethyl acetate (50 mL * 2). The organic phases were combined, washed successively with water (50 mL * 2) and saturated sodium chloride solution (50 mL), dried over anhydrous sodium sulfate and filtered. The filtrate was concentrated under reduced pressure, and the resulting crude product was made slurry in ethyl acetate. The resulting mother liquor was concentrated under reduced pressure, and purified by reverse HPLC to obtain the title product 2-(3-cyano-5-oxo-8-(trifluoromethyl)pyrazolo[1,5-a]pyrido[3,2-e]pyrimidin-4(5H)-yl)-N-(5-fluoropyridin-2-yl)acetamide Example 20. MS m/z (ESI): 432.3 [M+H]⁺.

¹H NMR (400 MHz, DMSO-d6) δ 11.2 (s, 1H), 8.93 (d, J = 8.0 Hz, 1H), 8.58 (s, 1H), 8.38 (d, J = 3.2 Hz, 1H), 8.20 (d, J = 8.0 Hz, 1H), 8.07 - 8.04 (m, 1H), 7.81 - 7.75 (m, 1H), 5.19 (s, 2H).

### Example 21

### N-(5-Fluoropyridin-2-yl)-2-(2-(hydroxymethyl)-5-oxo-8-(trifluoromethyl)pyrazolo[ 1,5-a]pyrido[3,2-e]pyrimidin-4(5H)-yl)acetamide

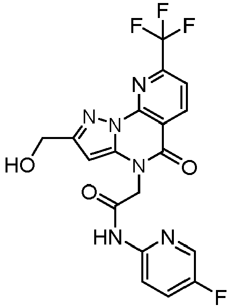

### Step 1: Preparation of N-(5-fluoropyridin-2-yl)-2-(2-(hydroxymethyl)-5-oxo-8-(trifluoromethyl)pyrazolo[1,5-a ]pyrido[3,2-e]pyrimidin-4(5H)-yl)acetamide

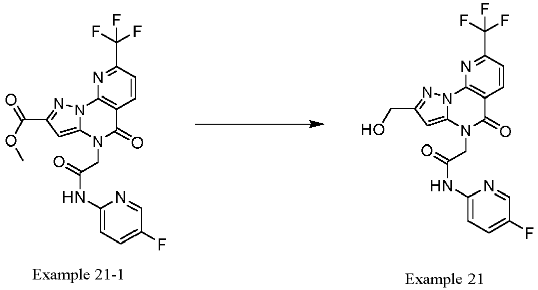

Diisobutylaluminum hydride (1M in toluene, 0.66 mL, 0.66 mmol) was added to a solution of Example 22-1 (100 mg, 0.22 mmol) (Example 21-1 was synthesized according to Example 8-2) in THF (2 mL) at 0°C. The mixture was stirred at room temperature overnight. Rochelle's salt solution (1.0 M, 5 ml) was added, followed by addition of ethyl acetate (5 mL). The resulting suspension was stirred at room temperature until clear phase separation was achieved. The organic phase was separated, and the aqueous phase was extracted with EtOAc (3×40 ml). The combined organic layers were washed with saturated aqueous solution of sodium bicarbonate (50 mL) and saturated brine (50 mL), dried over anhydrous sodium sulfate, concentrated and purified to obtain the target compound (32 mg, yield: 34%).

MS m/z (ESI): 437.1[M+H]⁺.

¹H NMR (400 MHz, DMSO) δ 11.06 (s, 1H), 8.82 (d, J = 7.9 Hz, 1H), 8.37 (s, 1H), 8.02 (m, 2H), 7.76 (s, 1H), 6.40 (s, 1H), 5.44 (s, 1H), 5.00 (s, 2H), 4.56 (s, 2H).

### Biological Assay and Evaluation

The present invention is further illustrated below in combination with the following test examples, which are not intended to limit the scope of the present invention.

### Test Example 1. Determination of the effect of the compounds of the present invention on calcium ion mobility in cells stably expressing 1321N1-hP2X3 receptors

Experimental objective: To determine the inhibitory activity of the compounds on 1321N1-hP2X3 receptor.

### Experimental instruments:

384-well cell plate (Corning; 3712); 384-well compound plate (Corning; 3657);
384-well assay plate (LABCYTE; P-05525); FLIPR (Molecular Devices).

### Experimental reagents:

DMEM (Gibco; 11965); FBS (Gibco; 10099-141);
Hygromycin B (Invitrogen, 10687010); Matrix (Thermo; 5416);
DMSO (Sigma; D2650); HBSS (Invitrogen; 14025);
HEPES (Invitrogen; 15630080); Probenecid (Sigma; P8761);
Versene (Gibco; 15040066); G418 (Sigma; G5013);
FLIPR^{®} Calcium 4 Assay Kit (Molecular Devices; R8141);
α,β-meATP (Sigma; M6517); ATP hydrolytic enzyme (Sigma; A7646);
Stably transfected cell line: 1321N1-hP2X3 (supplied by Shanghai ChemPartner Chemical Research Co., Ltd.).

### Experimental method:

1. Formulation of the reagents:
   Assay buffer: 1* HBSS + 20mM HEPES;
   Cell culture medium: DMEM + 10% FBS +75 µg/mL Hygromycin B + 300 µg/mL G418;
   Plating medium: DMEM + 10% DPBS;
   0.5* Dye: 10* Dye stock + 1.25 Probenecid + 1* assay buffer + 0.5U/mL ATP hydrolytic enzyme;
2. The cells were cultured to 70%-90% confluency in the cell culture medium at 37°C, 5% CO₂. The medium was discarded, and the cells were added with 2 mL of Versene, and the cells were placed in an incubator at 37°C for 2 to 5 min. The cells were collected by addition of 10 mL of plating medium and counted. The cells were seeded to the 384-well assay plate by addition of 50 µL solution (a density of 1 × 10⁴ cells/well) to each well, and incubated for 16 to 24 hours (at least overnight).
3. The medium was discarded, and 30 µL of 1X dye was added. The cells were incubated at 37°C in the dark for 60 minutes.
4. The compound powder was dissolved in DMSO to obtain a 20 mM stock solution. 180 X compound with required concentration was formulated, and diluted in gradient for 10 concentration points.
5. Preparation of compound plate: 500 nL of 180 X compound was transferred to the compound plate (source plate for FLIPR) using ECHO. 30 µL of assay buffer was added to each well, and the plate was shaken gently for 20 to 40 minutes.
6. Determination: 15 µL of 3X compound was taken from each well and added to the cell plate. The samples were added by FLIPR instrument, and the calcium signals were detected. After 15 minutes, 22.5 µL of 3X agonist (EC₈₀ concentration) was added to each well and the calcium signals were detected.

### Processing method of the experimental data:

The calcium signal values were determined by FLIPR. The ratio of the 340/510 nm wavelength signals to 380/510 nm wavelength signals was used as the calculated results for each sampling time point in the experiment. The calculation of maximum minus minimum was derived from the ratio signal curve. The percent inhibition rate and ten-point concentration data were fitted to the parametric nonlinear logistic equation by using GraphPad prism to calculate the IC₅₀ values of the compounds.

Experimental results: The results of the compounds of the Examples of the present invention in the 1321N1-hP2X3 receptor cell function calcium ion mobility assay are shown in Table 10:

**Table 10**

| Example No. | 1321N1-hP2X3 IC₅₀ (nM) |
|---|---|
| 1 | 16.19 |
| 10 | 88.20 |
| 12 | 63.38 |
| 13 | 77.69 |
| 14 | 185.0 |
| 15 | 76.40 |
| 16 | 130.4 |
| 17 | 32.45 |
| 18 | 49.00 |
| 19 | 34.70 |
| 20 | 64.35 |

Experimental conclusion: The above data indicate that the compounds of the present invention show good inhibitory effect in the 1321N1-hP2X3 receptor cell function calcium ion mobility assay.

### Test Example 2. Determination of the effect of the compounds of the present invention on calcium ion mobility in cells stably expressing 1321N1-hP2X2/3 receptors

Experimental objective: To determine the inhibitory activity of the compounds on 1321N1-hP2X2/3 receptor.

### Experimental instruments:

384-well cell plate (Corning; 3712); 384-well compound plate (Corning; 3657);
384-well assay plate (LABCYTE; P-05525); FLIPR (Molecular Devices).

### Experimental reagents:

DMEM (Gibco; 11965); FBS (Gibco; 10099-141);
Hygromycin B (Invitrogen, 10687010); Matrix (Thermo; 5416);
DMSO (Sigma; D2650); HBSS (Invitrogen; 14025);
HEPES (Invitrogen; 15630080); Probenecid (Sigma; P8761);
Versene (Gibco; 15040066); G418 (Sigma; G5013);
FLIPR^{®} Calcium 4 Assay Kit (Molecular Devices; R8141);
α,β-meATP (Sigma; M6517); ATP hydrolytic enzyme (Sigma; A7646);
Stably transfected cell line: 1321N1-hP2X2/3 (supplied by Shanghai ChemPartner Chemical Research Co., Ltd.).

### Experimental method:

1. Formulation of the reagents:
   Assay buffer: 1* HBSS + 20mM HEPES;
   Cell culture medium: DMEM + 10% FBS +75 µg/mL Hygromycin B + 150 µg/mL G418;
   Plating medium: DMEM + 10% DPBS;
   0.5* Dye: 10* Dye stock + 1.25 Probenecid + 1* assay buffer + 0.5U/mL ATP hydrolytic enzyme;
2. The cells were cultured to 70%-90% confluency in the cell culture medium at 37°C, 5% CO₂. The medium was discarded, and the cells were added with 2 mL of Versene, and the cells were placed in an incubator at 37°C for 2 to 5 minutes. The cells were collected by addition of 10 mL of plating medium and counted. The cells were seeded to the 384-well assay plate by addition of 50 µL solution (a density of 1 × 10⁴ cells/well) to each well, and incubated for 16 to 24 hours (at least overnight).
3. The medium was discarded, and 30 µL of 1X dye was added. The cells were incubated at 37°C in the dark for 60 minutes.
4. The compound powder was dissolved in DMSO to obtain a 20 mM stock solution. 180 X compound with required concentration was formulated, and diluted in gradient for 10 concentration points.
5. Preparation of compound plate: 500 nL of 180 X compound was transferred to the compound plate (source plate for FLIPR) using ECHO. 30 µL of assay buffer was added to each well, and the plate was shaken gently for 20 to 40 minutes.
6. Determination: 15 µL of 3X compound was taken from each well and added to the cell plate. The samples were added by FLIPR instrument, and the calcium signals were detected. After 15 minutes, 22.5 µL of 3X agonist (EC₈₀ concentration) was added to each well and the calcium signals were detected.

### Processing method of the experimental data:

The calcium signal values were determined by FLIPR. The ratio of the 340/510 nm wavelength signals to 380/510 nm wavelength signals was used as the calculated results for each sampling time point in the experiment. The calculation of maximum minus minimum was derived from the ratio signal curve. The percent inhibition rate and ten-point concentration data were fitted to the parametric nonlinear logistic equation by using GraphPad prism to calculate the IC₅₀ values of the compounds.

Experimental results: The results of the compounds of the Examples of the present invention in the 1321N1-hP2X2/3 receptor cell function calcium ion mobility assay are shown in Table 11:

**Table 11**

| Example No. | 1321N1-hP2X2/3 IC₅₀ (nM) |
|---|---|
| 1 | 64390 |
| 10 | 14540 |
| 12 | 25240 |
| 13 | >30000 |
| 15 | 6363 |
| 17 | 5629 |
| 18 | 4523 |
| 20 | 3037 |

Experimental conclusion: The above data indicate that the compounds of the present invention show weak inhibitory effect in the 1321N1-h2X2/3 receptor cell function calcium ion mobility assay.

### Test Example 3. Pharmacokinetic assay in Balb/C mice

1. Study objective: Balb/C mice were used as test animals. The pharmacokinetic behavior of the compounds of Examples was studied in mouse body (plasma) by orally administration at a dose of 5 mg/kg.
2. Experimental protocol
2.1 Test compounds: Compounds of the Examples of the present invention, prepared by the applicant.
2.2 Test animals: Male Balb/C mice (6 mice per group), purchased from Shanghai Jiesijie Laboratory Animal Co., LTD, with Certificate No.: SCXK (Shanghai) 2013-0006 N0.311620400001794.
2.3 Formulation of the compound: 5 g of hydroxyethyl cellulose (HEC, CMC-Na, viscosity: 800-1200 Cps) was weighed and dissolved in 1000 mL of purified water, followed by addition of 10 g of Tween80. The mixture was mixed well to obtain a clear solution.
2.4 Administration: After an overnight fast, male Balb/C mice were administered p.o. with the test compound at a dose of 5 mg/kg and a volume of 10 mL/kg.
2.5 Sample collection: 0.04 mL of blood was taken from the orbit of the mouse before administration and at 0, 0.5, 1, 2, 4, 6, 8 and 24 hours after administration. The samples were stored in EDTA-K₂ tubes, and centrifuged for 6 minutes at 4°C, 6000 rpm to separate the plasma. The plasma samples were stored at -80°C.
2.6 Sample process:
1) 160 µL of acetonitrile was added to 20 µL of the plasma sample for precipitation, and then the mixture was centrifuged at 3500 × g for 5 to 20 minutes.
2) After the above process, 100 µL of the supernatant was taken to analyze the concentration of the test compound by LC/MS/MS.

2.7 Liquid chromatography analysis
• Liquid chromatography condition: Shimadzu LC-20AD pump
• Mass spectrometry condition: AB Sciex API 4000 mass spectrometer
• Chromatographic column: phenomenex Gemiu 5 um C18 50 × 4.6 mm
• Mobile phase: Eluent A was 0.1% formic acid in water, and Eluent B was acetonitrile
• Flow rate: 0.8 mL/min
• Elution time: 0-4.0 minutes, the eluent is as follows:

**Table 12**

| Time/minute | Eluent A | Eluent B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

3. Experimental results and analysis
The main parameters of pharmacokinetics were calculated by WinNonlin 8.2. The results of pharmacokinetic test in mice are shown in the following Table 13:

**Table 13 Results of pharmacokinetic test in mice**

| **Example No.** | Pharmacokinetic test (5 mg/kg) | | | | |
|---|---|---|---|---|---|
| | Peak time | Area under curve | Plasma concentration | Half life | Average residence time |
| | tₘₐₓ(h) | AUC₀₋ₜ(ng/mL*h) | Cₘₐₓ(ng/mL) | t_{1/2}(h) | MRT_{0-∞}(h) |
| 10 | 0.50 | 10943.63 | 3546.70 | 1.36 | 2.16 |
| 12 | 1.00 | 7584.0 | 2360.0 | 1.4 | 2.3 |
| 15 | 2.00 | 30539.48 | 3433.3 | 4.60 | 5.60 |
| 17 | 2.00 | 10160.1 | 1826.7 | 1.8 | 3.8 |
| 18 | 1.00 | 5043.0 | 1293.3 | 1.6 | 2.5 |

| | | | | | |
|---|---|---|---|---|---|
| Note: 0.5% CMC-Na (1% Tween 80) | | | | | |

4. Experimental conclusion: It can be seen from the results of pharmacokinetic test in mice in the table that the compounds of the Examples of the present invention show good pharmacokinetic properties, and both the exposure AUC and maximum plasma concentration Cₘₐₓ are good.

### Test Example 4. Pharmacokinetic assay in rats

1. Study objective: SD rats were used as test animals. The pharmacokinetic behavior of the compounds of Examples was studied in rat body (plasma) by orally administration at a dose of 5 mg/kg.
2. Experimental protocol
2.1 Test compounds: Compounds of the Examples of the present invention, prepared by the applicant.
2.2 Test animals: Male SD rats (3 rats per group), purchased from Shanghai Jiesijie Laboratory Animal Co., LTD, with Certificate No.: SCXK (Shanghai) 2013-0006 N0.311620400001794.
2.3 Formulation of the compound: 5 g of hydroxyethyl cellulose (HEC, CMC-Na, viscosity: 800-1200 Cps) was weighed and dissolved in 1000 mL of purified water, followed by addition of 10 g of Tween80. The mixture was mixed well to obtain a clear solution.
2.4 Administration: After an overnight fast, male SD rats (3 rats per group) were administered p.o. with the test compound at a dose of 5 mg/kg and a volume of 10 mL/kg.
2.5 Sample collection: 0.2 mL of blood was taken from the jugular vein of the rat before administration and at 0, 0.5, 1, 2, 4, 6, 8 and 24 hours after administration. The samples were stored in EDTA-K₂ tubes, and centrifuged for 6 minutes at 4°C, 6000 rpm to separate the plasma. The plasma samples were stored at -80°C.
2.6 Sample process:
1) 160 µL of acetonitrile was added to 40 µL of the plasma sample for precipitation, and then the mixture was centrifuged at 3500 × g for 5 to 20 minutes.
2) After the above process, 100 µL of the supernatant was taken to analyze the concentration of the test compound by LC/MS/MS.

2.7 Liquid chromatography analysis
• Liquid chromatography condition: Shimadzu LC-20AD pump
• Mass spectrometry condition: AB Sciex API 4000 mass spectrometer
• Chromatographic column: phenomenex Gemiu 5 um C18 50 × 4.6 mm
• Mobile phase: Eluent A was 0.1% formic acid in water, and Eluent B was acetonitrile
• Flow rate: 0.8 mL/min
• Elution time: 0-4.0 minutes, the eluent is as follows:

**Table 14**

| Time/minute | Eluent A | Eluent B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

3. Experimental results and analysis
The main parameters of pharmacokinetics were calculated by WinNonlin 8.2. The results of pharmacokinetic test in rats are shown in the following Table 15:

**Table 15 Results of pharmacokinetic test in rats**

| **Example No.** | Pharmacokinetic test (5 mg/kg) | | | | |
|---|---|---|---|---|---|
| | Peak time | Area under curve | Plasma concentration | Half life | Average residence time |
| | tₘₐₓ(h) | AUC₀₋ₜ(ng/mL*h) | Cₘₐₓ(ng/mL) | t_{1/2}(h) | MRT_{0-∞}(h) |
| 12 | 4.00 | 5783 | 904 | 3.5 | 6.2 |
| 15 | 2.00 | 11977 | 1547 | 8.0 | 5.8 |
| 17 | 4.00 | 9852 | 1877 | 1.8 | 3.5 |
| 18 | 2.00 | 6811 | 1217 | 1.7 | 3.7 |
| 20 | 4.00 | 21252 | 2193 | 11.3 | 13.6 |

| | | | | | |
|---|---|---|---|---|---|
| Note: 0.5% CMC-Na (1% Tween 80) | | | | | |

4. Experimental conclusion: It can be seen from the results of pharmacokinetic test in rats in the table that the compounds of the Examples of the present invention show good pharmacokinetic properties at the dose of 5 mg/kg, and both the exposure AUC and maximum plasma concentration Cₘₐₓ are good.

### Test Example 5. Assay of metabolic stability in liver microsome

1. Experimental objective: The objective of the experiment is to determine the stability of the compounds of the Examples in liver microsome of mouse, rat, dog and human.
2. Experimental procedure:
2.1 Formulation of the working solution of the compound: Formulation of the working solution of the compound: The stock solution of the compound was added to phosphate buffer, and the final concentration was 20 µM.
2.2 Formulation of the working solution of liver microsome: Liver microsome was diluted with 100 mM phosphate buffer to obtain a final concentration of 0.625 mg/mL.
2.3 Formulation of NADPH and UDPGA: NADPH (reduced nicotinamide adenine dinucleotide phosphate) and UDPGA (uridine diphosphate glucuronic acid) were weighed respectively, followed by addition of 100 mM phosphate buffer. The final concentrations were 20 mM.
2.4 Formulation of the channel-forming reagent: 1 mg of Alamethicin was weighed, to which 200 µL of DMSO was added to obtain a 5 mg/mL solution. The solution was diluted with phosphate buffer to obtain a final concentration of 50 µg/mL.
2.5 Formulation of the reaction stop solution: Stop solution: Cold acetonitrile containing 100 ng/mL labetalol hydrochloride and 400 ng/mL tolbutamide as internal standards.
2.6 Incubation procedure: 400 µL of the prepared liver microsome, 25 µL of the working solution of the compound and 25 µL of Alamethicin were added to a 96-well plate successively, which was then pre-incubated at 37°C for 10 minutes. 50 µL of the prepared NADPH/UDPGA was added to initiate the reaction, and the plate was incubated at 37°C. The total volume of the reaction system was 500 µL. The final contents of the components were as follows:

**Table 16**

| Components | Content |
|---|---|
| Liver microsome | 0.5 mg/mL |
| Compound | 1 µM |
| NADPH | 2 mM |
| UDPGA | 2 mM |
| Alamethicin | 2.5 µg/mL |

2.7 Sample analysis
2.7.1 Chromatographic conditions:
   Instrument: Shimadzu LC-30 AD; Chromatographic column: XBridge^{®} C18 (50*4.6 mm, particle size: 5 µm);
   Mobile phase: A: 0.1% formic acid solution, B: methanol
   Eluent gradient: 0.2-1.6min 5%A to 95%A, 3.0~3.1min 95%Ato 5%A
   Running time: 4.0 min.
2.7.2 Mass spectrometry conditions:
   Instrument: API5500 liquid chromatography-mass spectrometer, AB Sciex;
   Ion source: Electrospray ionization source (ESI); Drying gas: N₂, temperature: 500°C;
   Electrospray voltage: 5000V; Detection method: Positive ion detection;
   Scanning mode: Mode of reaction monitoring (MRM).

3. Experimental results:

**Table 17 Results of the metabolic stability assay of the compounds of the Examples in liver microsome**

| Example No. | Mouse | | Rat | | Dog | | Human | |
|---|---|---|---|---|---|---|---|---|
| | *t*_{1/2} (min) | Remaining (%,60min) | *t*_{1/2} (min) | Remaining (%,60min) | *t*_{1/2} (min) | Remaining (%,60min) | *t*_{1/2} (min) | Remainin g (%,60min) |
| 12 | 186.6 | 81.3 | 1735.0 | 104.4 | / | / | ∞ | 113.9 |
| 15 | 409.1 | 92.5 | 941.8 | 102.3 | 937.5 | 96.0 | ∞ | 100.4 |
| 17 | 165.0 | 91.6 | ∞ | 106.4 | 1352.9 | 99.0 | 1405.4 | 101.2 |
| 18 | 34099.6 | 99.3 | 1964.7 | 98.9 | 671.0 | 93.4 | 884.7 | 97.5 |

4. Experimental conclusion: The above data show that the compounds of the Examples of the present invention have good metabolic stability in liver microsome of mouse, rat, dog and human.

### Test Example 6. Assay of plasma protein binding rate

1. Experimental objective: The objective of the experiment is to determine the plasma protein binding of the compounds of the Examples in plasma.
2. Experimental instruments and materials: Liquid chromatography-mass spectrometer, centrifuge, vortex mixer, pipette, continuous pipette, 96-well plate, tissue homogenizer (used for tissue sample analysis), 50% aqueous solution of methanol, acetonitrile solution containing internal standard, blank medium (plasma, urine or tissue homogenate, etc.)
3. Experimental procedure:
3.1 Formulation of the stock solution A of the test compound: The compound of the Example was formulated into 1 mM solution A with DMSO;
3.2 Formulation of the plasma solution B: The solution A was added to the plasma solution to obtain 5 µM solution B;
3.3 Operation procedure
   1) 200 µL of solution B was added to the inside of the membrane; 2) 350 µL of PBS was added to the outside of the membrane;
   3) Incubation in a water bath at 37°C for 6h; 4) The sample was diluted and analyzed by mass spectrometry.

4. Chromatographic conditions:
Instrument: Shimadzu LC-20 AD; Chromatographic column: Phenomenex Gemiu ^{®} C18 (50*4.6 mm, particle size: 5 µm);
Mobile phase: A: acetonitrile, B: 0.1% formic acid solution; 0-0.5 min: 5% A→90% A, 2.0-2.1 min: 90%A→5% A; flow rate: 0.8 mL/min; running time: 5.0 min; injection volume: 5 µL.

5. Mass spectrometry conditions:
Instrument: API4000 liquid chromatography-mass spectrometer, AB Co., USA;
The ion source was electrospray ionization source (ESI); the temperature of the drying gas (N₂) was 500°C;
The electrospray voltage was 5500V; the detection method was positive ion detection;
The scanning mode was mode of reaction monitoring (MRM); the scan time was 0.1s.

6. Experimental results:

**Table 18: Results of the plasma protein binding rate assay of the compounds of the Examples**

| Example No. | Mouse | Rat | Dog | Human |
|---|---|---|---|---|
| | % Unbound | % Unbound | % Unbound | % Unbound |
| 12 | 4.3 | 4.4 | 3.2 | 1.5 |
| 15 | 4.4 | 2.7 | 3.7 | 1.6 |
| 17 | 16.2 | 11.0 | 8.4 | 6.6 |
| 18 | 8.4 | 6.1 | 14.7 | 10.3 |
| 20 | 25.6 | 23.4 | 13.5 | 13.5 |

7. Experimental conclusion: The above data show that the compounds of the Examples of the present invention have high plasma protein binding rate with little species variation.

### Test Example 7. CYP enzyme single-point inhibition assay

1. Experimental objective: The inhibition of the compounds on CYP450 enzyme isoform was rapidly predicted by single-point method using human liver microsome incubation system.
2. Experimental procedure
2.1 Solution formulation: 2.5 mM NADPH: 4.165 mg of NADPH (reduced nicotinamide adenine dinucleotide phosphate) was weighed, followed by addition of 100 mM phosphate buffer to 2 mL. 0.25 mg/mL microsome solution: 4 mL of 100 mM phosphate buffer was added to 50 µL of 20 mg/mL microsome solution and mixed well.
   Formulation of the reaction solution of the test compound: The test compound of the Example was weighed, diluted to 10 mM with DMSO and then to 100 µM with 100 mM phosphate buffer.
2.2 Experimental procedure:
   1. 40 µL of liver microsome, 10 µL of substrate and 10 µL of the test compound were added to a 96-well plate and pre-incubated for 3 min.
   2. 40 µL of NADPH was added.
   3. 300 µL of acetonitrile stop solution containing internal standard was added at 20 min.
   4. The sample was centrifuged and injected.

3. Experimental results:

**Table 19 Results of the CYP enzyme single-point inhibition assay of the compounds of the Examples**

| **Compound** | **IC₅₀ (µM)** | | | | | |
|---|---|---|---|---|---|---|
| | **1A2** | **2C9** | **2C19** | **2D6** | **3A4-M** | **3A4-T** |
| 12 | 24.9 | >100 | >100 | >100 | >100 | >100 |
| 15 | 72.1 | >100 | >100 | >100 | >100 | >100 |
| 17 | >100 | >100 | >100 | >100 | >100 | >100 |
| 18 | > 100 | > 100 | > 100 | > 100 | > 100 | > 100 |

| | | | | | | |
|---|---|---|---|---|---|---|
| Note: Strong inhibition: IC₅₀<1 µM; moderate inhibition: 1 µM<IC₅₀<10 µM; weak inhibition: IC₅₀>10 µM | | | | | | |

4. Experimental conclusion: The above data show that the compounds of the Examples of the present invention have no strong inhibition on CYP enzyme isoforms, and the risk of DDI is low.

### Test Example 8. hERG potassium channel inhibition activity assay

1. Cell preparation
7.1.1 CHO-hERG cells were cultured in a 175 cm² culture flask. After the cell density reached 60-80%, the culture solution was removed. The cells were washed with 7 mL of PBS once, and dissociated with 3 mL of Detachin.
7.1.2 After completion of dissociation, the cells were neutralized with 7 mL of culture solution. The solution was centrifuged, and the supernate was removed. The cells were resuspended in 5 mL of culture solution. The cell indensity is ensured as 2~5×10⁶/mL.

2. Solution formulation

**Tables 20 Components of intracellular and extracellular fluids**

| Reagents | Extracellular fluid (mM) | Intracellular fluid (mM) |
|---|---|---|
| CaCl₂ | 2 | 5.374 |
| MgCl₂ | 1 | 1.75 |
| KCl | 4 | 120 |
| NaCl | 145 | - |
| Glucose | 10 | - |
| HEPES | 10 | 10 |
| EGTA | - | 5 |
| Na-ATP | - | 4 |
| pH | 7.40 (adjusted with NaOH), Osmolarity∼305 mOsm | 7.25 (adjusted with KOH), Osmolarity∼290 mOsm |

3. Electrophysiological recording process
Single cell sealing impedance and formation of whole-cell mode were automatically performed by Qpatch instrument. After obtaining the whole-cell recording mode, the cell was clamped at -80 mV. The cell first underwent pre-voltage of -50 mV for 50 msec, then underwent depolarization stimulation at +40 mV for 5 sec, and then underwent repolarization at -50 mV for 5 sec, and then the voltage returned to -80 mV. The cell underwent the stimulation at the voltage every 15 sec. The data were recorded for 2 minutes, then extracellular fluid was administrated, and then the data were recorded for 5 minutes. Then, the administration process begun. The concentration of the test compound started from the lowest concentration, and each test concentration was administrated for 2.5 minutes. At least three cells (n ≥ 3) were tested for each concentration.
4. Compound formulation
4.1 20 mM mother liquor of the compound was diluted with extracellular fluid. 2495 µL of extracellular fluid was added to 5 µL of 20 mM mother liquor of the compound to obtain a concentration of 40 µM (500-fold dilution). The solution was subjected to a 3-fold serial dilution with extracellular fluid containing 0.2% DMSO to obtain a required final concentration.
4.2 The highest test concentration was 40 µM. The 6 concentrations were 40, 13.33, 4.44, 1.48, 0.49 and 0.16 µM.
4.3 The DMSO content in the final test concentration did not exceed 0.2%. This concentration of DMSO had no effect on hERG potassium channel.

5. Data analysis: The experimental data was analyzed by XLFit software.
6. Quality control
Environment: humidity 20-50%, temperature 22~25°C
Reagents: the reagents used were purchased from Sigma, with a purity of> 98%
The experimental data in the report must meet the following criteria:
   Whole cell sealing impedance > 100 MΩ
   Tail current amplitude > 400 pA
   Pharmacological parameters: The inhibition effect of Cisapride at multiple concentrations on hERG channel was used as the positive control.

7. Experimental results:

**Table 21: Results of inhibition effect of the compounds of the Examples at multiple concentrations on hERG current**

| Example No. | hERG IC₅₀ (uM) |
|---|---|
| 15 | >10 |
| 17 | 18.38 |
| 18 | >10 |
| 20 | >20 |

8. Experimental conclusion:
Inhibition of cardiac hERG potassium channel by drug is the main cause of drug-induced QT prolongation syndrome. It can be seen from the experimental results that the compounds of the Examples of the present invention have no obvious inhibition effect on cardiac hERG potassium channel. Cardiotoxic effects at high doses can thus be avoided.

### Test Example 9. Taste sensitivity assay in BALB/c mice

1. Experimental objective: In this assay, compounds with less toxic and side effects on animal taste were screened by quinine bitter solution experiment.
2. Main experimental instruments and materials
2.1 Instruments:
   1. Ultra-clean workbench (CJ-2F, Suzhou Fengshi Laboratory Animal Equipment Co., Ltd);
   2. Electronic balance (CPA2202D, Sartorius);
   3. Electronic balance (BSA2202S-CW, Sartorius);
   4. Pure water maker (Pacific TII, Thermo).
2.2 Reagents: Quinine monohydrochloride dihydrate (6119-47-7, Adamas).
2.3 Animals: BALB/c mice, 6 to 8 weeks old, ♂, purchased from Shanghai SIPPR-BK Laboratory Animal Co., Ltd.

3. Experimental procedure:
3.1 Animal screening: One day before the experiment, all BALB/c mice were weighed, and animals with too high or too low body weight were excluded.
3.2 Grouping and water deprivation: BALB/c mice were randomly grouped according to body weight, and were deprived of water 12 to 16 hours before administration with no fasting.
3.3 Formulation of aqueous solution of quinine: An appropriate amount of quinine monohydrochloride dihydrate was weighed and formulated into an aqueous solution quinine hydrochloride (concentration: 3 mmol/L) with ultrapure water for later use.
3.4 Formulation of test compound: An appropriate amount of the test compound was weighed and formulated into the target concentration with the corresponding solvent according to the experimental design for later use.
3.5 Administration and quinine solution intake assay in animal: Administration and fasting: On the day of the experiment, the animals were weighed and fasted, bedding was changed, and the compounds were administered according to the experimental design.
   Quinine solution intake assay:
   1. The corresponding clean mouse drinking bottle was rinsed 2 to 3 times with ultrapure water and the formulated 3 mmol/L aqueous solution of quinine hydrochloride respectively. The bottle was filled and weighed, and the weight was recorded as Wi₀.
   2. According to the experimental design, a certain period after administration, the filled bottle was gently placed in the corresponding mouse cage, and the timing was started. After 30 min, the bottle was gently taken out and weighed, and the weight was recorded as Wi₃₀.
   3. Calculation of solution consumption of animals in each group: ΔWW(g)=Wi₃₀-Wi₀; calculation of solution consumption of single mouse: ΔpWW(g) =ΔWW/N, N is the number of animals in each group.
   4. Dysgeusia rate = (ΔpWW of the group in which the drinking water was the aqueous solution of quinine hydrochloride and the test compound was administered at the same time - ΔpWW of the group in which the drinking water was the aqueous solution of quinine hydrochloride and the solvent control was administered at the same time) / (ΔpWW of the group in which the drinking water was ultrapure water and the solvent control was administered at the same time - ΔpWW of the group in which the drinking water was the aqueous solution of quinine hydrochloride and the solvent control was administered at the same time) ×100%. Data processing was performed with software such as Excel.
   5. The animals were euthanized after completion of the experiment.

4. Experimental results:

**Table 22 Results of the taste sensitivity assay of the compounds of the Examples**

| Compound | Solution consumption of single mouse (g) | | Dysgeusia rate |
|---|---|---|---|
| | Ultrapure water | Quinine solution | |
| Solvent group (20% HP-B-CD) | 0.598 | / | / |
| Solvent group (20% HP-B-CD) | / | 0.068 | / |
| 15 @30 mpk | / | 0.048 | -3.77% |
| 17 @30 mpk | / | 0.056 | -2.26% |
| 18 @30 mpk | / | 0.016 | -9.81% |

5. Experimental conclusion:
It can be seen from the above results that the compounds of the present application have low toxic and side effects on the taste of mice.

### Test Example 10. Pharmacodynamic study on citric acid-induced acute cough of guinea pigs

1. Experimental objective: The objective of this experiment is to evaluate the efficacy of the compounds in a citric acid-induced acute cough model of guinea pigs.
2. Experimental instruments and reagents
2.1 Key instruments

**Table 23**

| **Instrument name** | **Manufacturer** | **Model/Specification** | **Device number** |
|---|---|---|---|
| WBP | DSI | Whole Body Plethysmography | 100301,100249 |
| Electronic balance | Changzhou Tianzhiping Instrument Equipment Co., Ltd. | EL-2KL | 6072710 |
| Ultrasonic cell poulverizer | Ningbo Scientz Biotechnology Co.,Ltd. | SCIENTZ-IID | 10192149 |
| Electronic balance | Mettler Toledo | MS205DL | B844687071 |
| Pipette | Eppendorf | 5mL | I19578I |
| Pipette | Eppendorf | 1000µL | Q12774H |
| Pipette | Eppendorf | 200µL | L33188I |
| Pipette | Eppendorf | 100µL | R12555H |

2.2 Key reagents

**Table 24**

| **Reagent name** | **Manufacturer** | **Article number** |
|---|---|---|
| Sodium carboxymethyl cellulose | Sigma | C5678 |
| Tween 80 | Sigma | P4780 |
| ATP | Sigma | A2383 |
| Citric acid | Sigma | C2404 |

3. Experimental operation and data processing:
3.1 Animals: Hartley Guinea Pigs, male, purchased from Beijing Vital River Laboratory Animal Technology Co., Ltd.
3.2 Experimental procedure: The animals were adaptively feed. After their body weight reached the standard (300 to 400 g), the animals were serially numbered and randomly grouped according to their body weight.
   Cough induction method: The guinea pig was put into the whole body plethysmography box to adapt for 3-5 minutes. ATP atomization was performed for 2 minutes. After an interval of 3 minutes, citric acid atomization was performed for 5 minutes. From the beginning of citric acid atomization, the number of coughs and the cough latency of the animals were recorded within 10 minutes.
3.3 Administration regimen and monitoring of cough indicators
   The test compound was administered to the guinea pig by a single gavage 2 hours before citric acid atomization. The guinea pig was put into the respiratory plethysmography chamber of the DSI Buxco whole body plethysmography (WBP) at the predetermined time, and subjected to cough induction by citric acid atomization. From the beginning of citric acid atomization, the total number of coughs (CCnt) and cough latency (CIP) in the guinea pig within 10 minutes were recorded by the WBP system.
3.4 Data processing
   All data were entered into Excel files and expressed as mean ± standard error. The data of each group were analyzed and compared by one-way ANOVA. If the statistical analysis results showed *p*<0.05, then there was a significant difference. Pairwise comparisons were carried out by t-test method to compare the differences.

The results show that the compounds of the Examples of the present invention can effectively improve cough symptoms in the citric acid-induced acute cough model of guinea pigs, and the reduction rate of the total number of coughs is over 59%.

### III. Study on the salt of compound and the crystal form thereof

As those skilled in the art are well known, when the above compounds of the Examples show good inhibitory effect in the 1321N1-hP2X3 receptor cell function calcium ion mobility assay, their pharmaceutically acceptable salts tend to have the same pharmacological activity. On this basis, the inventor further studied physical and chemical properties of salt form and crystal form of the corresponding compound, but the preparation and characterization of the following specific salt form or crystal form do not represent a limitation of the protection scope of the present invention, and those skilled in the art may obtain more salt forms and crystal forms of the compounds of the present invention based on the present invention, and these salt forms and crystal forms are the technical solutions protected by the present invention. The details are as follows.
1. Experimental instruments
1.1 Some parameters of physical and chemical testing instruments

**Table 25**

| | | |
|---|---|---|
| X-ray powder diffraction (XRPD) | Instrument model | BRUKER D8 ADVANCE |
| | Diffraction ray | CuK (40 kV, 25 mA) |
| | Scan rate | 0.02°/S (2θ value) |
| | Scan range | 4° to 40° (2θ value) |
| Differential scanning calorimetry (DSC) | Instrument model | NETZSCH DSC 214 polyma |
| | Purge gas | Nitrogen |
| | Purge speed | 40 mL/min |
| | Heating rate | 10°C/min |
| | Temperature range | 25 to 300°C |
| | Plate type | Aluminum plate |
| Thermogravimetric analysis (TGA) | Instrument model | NETZSCH TG 209 Tarsus |
| | Purge gas | Nitrogen |
| | Purge speed | 40 mL/min |
| | Heating rate | 10°C /min |
| | Temperature range | 35°C to 350°C |
| | Plate type | Al₂O₃ |

1.2 Instruments and liquid phase analysis conditions
1.2.1 Instruments and devices

**Table 26**

| Instrument name | Model |
|---|---|
| Analytical balance | METTLER TOLEDO XA105 |
| Water purifier | Milli-Q Plus, Millipore |
| High performance liquid chromatograph | Agilent1260 |
| Pump | Agilent G1311B |
| Injector | G1329B |
| Column oven | G1316A |
| Detector | G1315D |

1.2.2 Chromatography conditions
Chromatographic column: Zorbax BONUS RP (3.5µm, 4.6*75 mm)
Flow rate: 1.0 mL/min
Column temperature: 30°C
Detection wavelength: 262 nm
Injection volume: 5.0 µL
Running time: 15 min
Diluent: DMSO
Mobile phase: A: water (0.05% trifluoroacetic acid); B: acetonitrile (0.05% trifluoroacetic acid)

**Table 27**

| T(min) | A(%) | B(%) |
|---|---|---|
| 0.00 | 60 | 40 |
| 12.00 | 25 | 75 |
| 12.01 | 60 | 40 |
| 15.00 | 60 | 40 |

**2. Study on the salt form of the compound**
2.1 Screening of the salt form of the compound
2.1.1 Experimental objective: To screen the salt form of the compound.
2.1.2 Experimental procedures:
1) Instruments and devices

**Table 28**

| Name | Model | Source |
|---|---|---|
| Analytical balance | XA105 | METTLER TOLEDO |
| Ultrasonic cleaner | SK5200LHC | Shanghai Kudos Ultrasonic Instrument |
| Pipettes | Eppendorf (50 mL, 100 µL) | Eppendorf |

2) Operating procedures
Salt forming by solventing out or suspension: 10 mg of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide was weighed, to which 200 µL to 400 µL of solvent was added. The solution was stirred at 40 to 50°C, to which different acids were added respectively. The solution was stirred overnight, cooled to room temperature, filtered, and dried to obtain the salt of the compound.

**Table 29**

| No. | Acid (1 M in EtOH) | Solvent | Phenomenon after the addition of acid | Result |
|---|---|---|---|---|
| 1 | Hydrochloric acid | Tetrahydrofuran (200 µL) | Complete dissolution followed by precipitation | Salt forming |
| 2 | Hydrobromic acid | | Complete dissolution followed by precipitation | Salt forming |
| 3 | Sulfuric acid | | Complete dissolution followed by precipitation | Salt forming |
| 4 | Methanesulfonic acid | | Complete dissolution followed by precipitation | Salt forming |
| 5 | Ethanesulfonic acid | | Complete dissolution followed by precipitation | Salt forming |
| 6 | Hydrochloric acid | 2-Butanone (250 µL) | Complete dissolution followed by precipitation | Salt forming |
| 7 | Hydrobromic acid | | Complete dissolution followed by precipitation | Salt forming |
| 8 | Sulfuric acid | | Complete dissolution followed by precipitation | Salt forming |
| 9 | Methanesulfonic acid | | Complete dissolution followed by precipitation | Salt forming |
| 10 | Ethanesulfonic acid | | Complete dissolution followed by precipitation | Salt forming |
| 11 | Hydrochloric acid | Methanol (400 µL) | Suspension | Salt forming |
| 12 | Hydrobromic acid | | Suspension | Salt forming |
| 13 | Sulfuric acid | | Suspension | Salt forming |
| 14 | Methanesulfonic acid | | Suspension | Salt forming |
| 15 | Ethanesulfonic acid | | Suspension | Salt forming |
| 16 | Hydrochloric acid | Acetone (400 µL) | Complete dissolution followed by precipitation | Salt forming |
| 17 | Hydrobromic acid | | Complete dissolution followed by precipitation | Salt forming |
| 18 | Sulfuric acid | | Complete dissolution followed by precipitation | Salt forming |
| 19 | Methanesulfonic acid | | Complete dissolution followed by precipitation | Salt forming |
| 20 | Ethanesulfonic acid | | Complete dissolution followed by precipitation | Salt forming |
| 21 | Hydrochloric acid | Dichloromethane (400 µL) | Suspension | Salt forming |
| 22 | Hydrobromic acid | | Suspension | Salt forming |
| 23 | Sulfuric acid | | Suspension | Salt forming |
| 24 | Methanesulfonic acid | | Suspension | Salt forming |
| 25 | Ethanesulfonic acid | | Suspension | Salt forming |

2.1.3 Experimental results: It can be seen from the results of the screening of the salt form that the free base of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide can form salts with hydrochloric acid, hydrobromic acid, sulfuric acid, methanesulfonic acid, and ethanesulfonic acid.
As mentioned above, those skilled in the art can obtain more pharmaceutically acceptable salts based on the present invention.
2.2 Quantitative analysis of the salt form of the compound
2.2.1 DAD quantification of the salt form of the compound
2.2.1.1 Experimental objective: To determine the number of acids in the salt of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide.
2.2.1.2 Experimental procedures:
1) Chromatography conditions

**Table 30**

| | |
|---|---|
| Instrument | Thermo Ultimate 3000 |
| Diluent | DMSO |
| Chromatographic column | Waters x-bridge (150*4.6mm,3.5um) |
| Mobile phases | A: 25mM Phosphate buffer(NH₄H₂PO₄, pH2.0), B: MeOH |
| Injection volume | 5 µL |
| Flow rate | 1.0 ml/min |
| Column oven temperature | 35°C |
| Running time | 12 min |

| Gradient elution time (min) | B phase (volume percentage) |
|---|---|
| 0 | 50 |
| 10 | 80 |
| 10.01 | 50 |
| 12 | 50 |

2) Procedures: An appropriate amount of free base was weighed, to which DMSO was added to obtain a series of linear solutions with a concentration range of 0.006 to 0.68 mg/mL as external standard solutions STD.
An appropriate amount of the sulfate, methanesulfonate and ethanesulfonate of the compound was weighed respectively, to which DMSO was added to obtain sample solutions containing the salt of the compound with a concentration of 0.3 to 0.5 mg/mL. The above linear solutions and sample solutions were injected respectively.
2.2.1.3 Experimental results:

**Table 31**

| Sample name | Area of chromatographic main component peak | Sample concentration (mg/ml) | Calculated free base concentration (mg/ml) | Calculated free base content % | Theoretical number of acids | Theoretical free base content % |
|---|---|---|---|---|---|---|
| Free base STD1 | 151.3611 | 0.68 | / | / | / | / |
| Free base STD2 | 30.6005 | 0.13 | / | / | / | / |
| Free base STD3 | 15.5147 | 0.065 | / | / | / | / |
| Free base STD4 | 1.6041 | 0.0065 | / | / | / | / |
| Sulfate (crystal form B) | 72.362 | 0.407 | 0.3249 | 79.82 | 1 | 81.47 |
| Methanesulfonate (crystal form A) | 109.422 | 0.598 | 0.4913 | 82.15 | 1 | 81.77 |
| Ethanesulfonate (crystal form A) | 83.776 | 0.464 | 0.3761 | 81.06 | 1 | 79.67 |

DAD quantification results show that the sulfate, methanesulfonate and ethanesulfonate of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide are 1:1 salts.
2.2.2 ELSD quantification of the methanesulfonate
2.2.2.1 Experimental objective: To determine the number of methanesulfonic acids in the methanesulfonate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide.
2.2.2.2 Experimental procedures:
1) Chromatography conditions

**Table 32**

| | |
|---|---|
| Instrument | Agilent 1200 |
| Diluent | DMSO |
| Chromatographic column | ZIC-HILIC (150*4.6mm,5µm) |
| Mobile phases | A: 75mM ammonium acetate solution (pH 4.80), B: acetonitrile (A: B=30:70) |
| Injection volume | 5 µL |
| Flow rate | 1.0 ml/min |
| ELSD temperature | 80°C |
| Running time (min) | 10 |

2) Procedures: An appropriate amount of methanesulfonic acid was weighed, to which DMSO was added to obtain a series of linear solutions with a methanesulfonic acid concentration ranging from 0.15 to 0.5 mg/mL.
An appropriate amount of methanesulfonate was weighed, to which DMSO was added to obtain a sample solution containing the methanesulfonate of the compound with a concentration of 2.8 mg/mL. The above linear solutions and sample solution were injected respectively.
2.2.2.3 Experimental results:

**Table 33**

| Sample name | Area of chromatographic main component peak | Sample concentration (mg/ml) | Calculated methanesulfonic acid concentration (mg/ml) | Calculated methanesulfonic acid content % | Theoretical number of acids | Theoretical methanesulfonic acid content % |
|---|---|---|---|---|---|---|
| Methanesulfonic acid STD1 | 971.6 | 0.533 | / | / | / | / |
| Methanesulfonic acid STD2 | 540.0 | 0.308 | / | / | / | / |
| Methanesulfonic acid STD3 | 241.1 | 0.154 | / | / | / | / |
| Methanesulfonate (crystal form A) | 1001.7 | 2.821 | 0.5157 | 18.28 | 1 | 18.23 |

After calculation, the number of methanesulfonic acids in the methanesulfonate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide is 1.
2.3 Pharmacokinetic assay of the methanesulfonate in rats
2.3.1. Study objective: SD rats were used as test animals. The pharmacokinetic behavior of various salt forms of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide was studied in rat body (plasma) by orally administration at a dose of 10 mg/kg.
2.3.2. Experimental protocol
2.3.2.1 Test compounds: Compounds of the Examples of the present invention, prepared by the applicant.
2.3.2.2 Test animals: Male SD rats (3 rats per group). Certificate No.: SCXK (Shanghai) 2013-0006 N0.311620400001794.
2.3.2.3 Formulation of the compounds: Methanesulfonate crystal form A, ethanesulfonate crystal form A, sulfate crystal form B and hydrobromide crystal form B were respectively pulverized in 0.5% aqueous HPMC solution and suspended evenly by ultrasonic.
2.3.2.4 Administration: After an overnight fast, male SD rats (3 rats per group) were administered p.o. with the test compound at a dose of 10 mg/kg and a volume of 10 mL/kg.
2.3.2.5 Sample collection: 0.2 mL of blood was taken from the jugular vein of the rat before administration and at 0, 0.5, 1, 2, 4, 6, 8 and 24 hours after administration. The samples were stored in EDTA-K₂ tubes, and centrifuged for 6 minutes at 4°C, 6000 rpm to separate the plasma. The plasma samples were stored at -80°C.
2.3.2.6 Sample process:
1) 160 µL of acetonitrile was added to 40 µL of the plasma sample for precipitation, and then the mixture was centrifuged at 3500 × g for 5 to 20 minutes.
2) After the above process, 100 µL of the supernatant was taken to analyze the concentration of the test compound by LC/MS/MS.

2.3.2.7 Liquid chromatography analysis
• Liquid chromatography condition: Shimadzu LC-20AD pump
• Mass spectrometry condition: AB Sciex API 4000 mass spectrometer
• Chromatographic column: phenomenex Gemiu 5 um C18 50 × 4.6 mm
• Mobile phase: Eluent A was 0.1% formic acid in water, and Eluent B was acetonitrile
• Flow rate: 0.8 mL/min
• Elution time: 0-4.0 minutes, the eluent is as follows:

**Table 34**

| Time/minute | Eluent A | Eluent B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

2.3.3. Experimental results and analysis
The main parameters of pharmacokinetics were calculated by WinNonlin 8.2. The results of pharmacokinetic test in rats are shown in the table below.

**Table 35**

| **No.** | Pharmacokinetic test (10 mg/kg) | | | | |
|---|---|---|---|---|---|
| | Peak time | Area under curve | Plasma concentration | Half life | Average residence time |
| | tₘₐₓ (h) | AUC₀₋ₜ(ng/mL*h) | Cₘₐₓ (ng/mL) | t_{1/2} (h) | MRT_{0-∞} (h) |
| Methanesulfonate (crystal form A) | 4.00 | 25839 | 3323 | 2.7 | 4.8 |
| Ethanesulfonate (crystal form A) | 4.00 | 30301 | 3843 | 2.6 | 4.9 |
| Sulfate (crystal form B) | 2.00 | 21595 | 3080 | 2.8 | 4.6 |
| Hydrobromide (crystal form B) | 1.00 | 16758 | 2473 | 2.6 | 4.4 |

| | | | | | |
|---|---|---|---|---|---|
| Note: Vehicle: 0.5% HPMC | | | | | |

2.3.4. Experimental conclusion:
It can be seen from the results of pharmacokinetic test in rats in the table that the exposure AUC and maximum plasma concentration Cₘₐₓ of the methanesulfonate (crystal form A), ethanesulfonate (crystal form A), sulfate (crystal form B) and hydrobromide (crystal form B) of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide of the present invention at a dose of 10 mg/kg are superior than those of the free base.
**3. Study on the crystal form of the salt of the compound**
3.1 Study on the crystal form of the salt of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide
3.1.1 Experimental objective: To screen the salt of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide that can form a crystal form.
3.1.2 Experimental procedures:
1) Instruments and devices

**Table 36**

| Name | Model | Source |
|---|---|---|
| Analytical balance | XA 105 | METTLER TOLEDO |
| Ultrasonic cleaner | SK5200LHC | Shanghai Kudos Ultrasonic Instrument |
| Pipettes | Eppendorf (50 mL, 1000 µL) | Eppendorf |

2) Operating procedures: Salt forming by solventing out or suspension: 10 mg of the compound was weighed, to which 200 µL to 400 µL of solvent was added. The solution was stirred at 40 to 50°C, to which different acids were added respectively. The solution was stirred overnight, cooled to room temperature, filtered, and dried to obtain the salt of the compound.

**Table 37**

| No. | Acid (1 M in EtOH) | Solvent | Phenomenon after the addition of acid | Results |
|---|---|---|---|---|
| 1 | Hydrochloric acid | Tetrahydrofuran (200 µL) | Complete dissolution followed by precipitation | Crystal form |
| 2 | Hydrobromic acid | | Complete dissolution followed by precipitation | Crystal form |
| 3 | Sulfuric acid | | Complete dissolution followed by precipitation | Crystal form |
| 4 | Methanesulfonic acid | | Complete dissolution followed by precipitation | Crystal form |
| 5 | Ethanesulfonic acid | | Complete dissolution followed by precipitation | Crystal form |
| 6 | Hydrochloric acid | 2-Butanone (250 µL) | Complete dissolution followed by precipitation | Crystal form |
| 7 | Hydrobromic acid | | Complete dissolution followed by precipitation | Crystal form |
| 8 | Sulfuric acid | | Complete dissolution followed by precipitation | Crystal form |
| 9 | Methanesulfonic acid | | Complete dissolution followed by precipitation | Crystal form |
| 10 | Ethanesulfonic acid | | Complete dissolution followed by precipitation | Crystal form |
| 11 | Hydrochloric acid | Methanol (400 µL) | Suspension | Crystal form |
| 12 | Hydrobromic acid | | Suspension | Crystal form |
| 13 | Sulfuric acid | | Suspension | Crystal form |
| 14 | Methanesulfonic acid | | Suspension | Crystal form |
| 15 | Ethanesulfonic acid | | Suspension | Crystal form |
| 16 | Hydrochloric acid | Acetone (400 µL) | Complete dissolution followed by precipitation | Crystal form |
| 17 | Hydrobromic acid | | Complete dissolution followed by precipitation | Crystal form |
| 18 | Sulfuric acid | | Complete dissolution followed by precipitation | Crystal form |
| 19 | Methanesulfonic acid | | Complete dissolution followed by precipitation | Crystal form |
| 20 | Ethanesulfonic acid | | Complete dissolution followed by precipitation | Crystal form |
| 21 | Hydrochloric acid | Dichloromethane (400 µL) | Suspension | Crystal form |
| 22 | Hydrobromic acid | | Suspension | Crystal form |
| 23 | Sulfuric acid | | Suspension | Crystal form |
| 24 | Methanesulfonic acid | | Suspension | Crystal form |
| 25 | Ethanesulfonic acid | | Suspension | Crystal form |

3.1.3 Experimental results: According to the study on the crystal form of the salt of the compound, the salt forms that can form a crystal form are hydrochloride, hydrobromide, sulfate, methanesulfonate and ethanesulfonate.
As mentioned above, those skilled in the art can obtain more pharmaceutically acceptable salts that can form a crystal form based on the present invention by conventional methods.
3.2 Preparation of the crystal form of the salt of the compound
3.2.1 Experimental objective: To prepare the crystal form of the salt of the compound.
3.2.2 Experimental procedures:
1) Instruments and devices

**Table 38**

| Name | Model | Source |
|---|---|---|
| Analytical balance | XA 105 | METTLER TOLEDO |
| Ultrasonic cleaner | SK5200LHC | Shanghai Kudos Ultrasonic Instrument |
| Pipettes | Eppendorf (50 mL, 1000 µL) | Eppendorf |

2) Operating procedures

### (1) Preparation of crystal form A of methanesulfonate

150 mg of the free base of the compound was weighed, to which 8 mL of dichloromethane was added. The mixture was stirred at 40°C, and the solid cannot be dissolved completely. 380 µL (1.1 e.q.) of a solution of methanesulfonic acid in ethanol (1 mol/L in EtOH) was added to precipitate a crystal by suspension method. The mixture was kept at 40°C overnight, cooled to room temperature, and centrifuged rapidly to remove the supernatant. The solid was dried under vacuum at 40°C to constant weight to obtain crystal form A of methanesulfonate. After detection and analysis, it has the XRPD pattern as shown in Figure 1, the DSC spectrum as shown in Figure 2, and the TGA spectrum as shown in Figure 3.

Alternatively, it can be obtained by the following steps:
The free base of the compound (15.0 g) was added to a reaction flask, to which 2-butanone (600 ml) was added. The mixture was warmed to 45 to 55°C, stirred to dissolve completely, and filtered while hot. A solution of methanesulfonic acid in ethanol (3.68 g of methanesulfonic acid dissolved in 35 ml of ethanol) was added dropwise to the filtrate. After the addition was completed, the solution was kept at the temperature and stirred overnight. The solution was cooled to 20 to 30°C, stirred for 30 minutes to precipitate a crystal, and filtered. The filter cake was dried under vacuum at 50°C to obtain 16.2 g of methanesulfonate crystal form A.

### (2) Preparation of crystal form A of ethanesulfonate

150 mg of the free base of the compound was weighed, to which 8 mL of dichloromethane was added. The mixture was stirred at 40°C, and the solid cannot be dissolved completely. 380 µL (1.1 e.q.) of a solution of ethanesulfonic acid in ethanol (1 mol/L in EtOH) was added to precipitate a crystal by suspension method. The system gradually became viscous with poor fluidity, to which 3 mL of solvent was added. The mixture was kept at 40°C overnight, cooled to room temperature, and centrifuged rapidly to remove the supernatant. The solid was dried under vacuum at 40°C to constant weight to obtain crystal form A of ethanesulfonate. After detection and analysis, it has the XRPD pattern as shown in Figure 4, the DSC spectrum as shown in Figure 5, and the TGA spectrum as shown in Figure 6.

### (3) Preparation of crystal form A of sulfate

30 mg of the free base of the compound was weighed, to which 1 mL of tetrahydrofuran was added. The mixture was stirred at 50°C, and the solid was dissolved. 77 µL (1.1 e.q.) of a solution of sulfuric acid in ethanol (1 mol/L in EtOH) was added. Precipitate formed within 5 minutes after adding the acid, and the system gradually became viscous with poor fluidity. The mixture was kept at 50°C overnight, cooled to room temperature, and centrifuged rapidly to remove the supernatant. The solid was dried under vacuum at 40°C to constant weight. After detection and analysis, it has the XRPD pattern as shown in Figure 7, and the DSC spectrum as shown in Figure 8.

### (4) Preparation of crystal form B of sulfate

150.54 mg of the free base of the compound was weighed, to which 8 mL of dichloromethane was added. The mixture was stirred at 40°C, and the solid cannot be dissolved completely. 380 µL (1.1 e.q.) of a solution of sulfuric acid in methanol (1 mol/L in MeOH) was added to precipitate a crystal by suspension method. The mixture was kept at 40°C overnight, cooled to room temperature, and centrifuged rapidly to remove the supernatant. The solid was dried under vacuum at 40°C to constant weight to obtain sulfate crystal form B. After detection and analysis, it has the XRPD pattern as shown in Figure 9, the DSC spectrum as shown in Figure 10, and the TGA spectrum as shown in Figure 11.

### (5) Preparation of crystal form A of hydrochloride

150.76 mg of the free base of the compound was weighed, to which 8 mL of dichloromethane was added. The mixture was stirred at 40°C, and the solid cannot be dissolved completely. 380 µL (1.1 e.q.) of a solution of hydrochloric acid in ethanol (1 mol/L in EtOH) was added to precipitate a crystal by suspension method. The mixture was kept at 40°C overnight, cooled to room temperature, and centrifuged rapidly to remove the supernatant. The solid was dried under vacuum at 40°C to constant weight to obtain crystal form A of hydrochloride. After detection and analysis, it has the XRPD pattern as shown in Figure 12, the DSC spectrum as shown in Figure 13, and the TGA spectrum as shown in Figure 14.

### (6) Preparation of crystal form B of hydrochloride

30 mg of the free base of the compound was weighed, to which 1 mL of tetrahydrofuran was added. The mixture was stirred at 50°C, and the solid was dissolved. 77 µL (1.1 e.q.) of a solution of hydrochloric acid in ethanol (1 mol/L in EtOH) was added, and precipitate immediately formed. The mixture was kept at 50°C overnight, cooled to room temperature, and centrifuged rapidly to remove the supernatant. The solid was dried under vacuum at 40°C to constant weight to obtain crystal form B of hydrochloride. After detection and analysis, it has the XRPD pattern as shown in Figure 15.

### (7) Preparation of crystal form A of hydrobromide

10 mg of the free base of the compound was weighed, to which 400 µL of solvent acetone was added. The mixture was stirred at 40°C, and the solid was substantially dissolved. 1.1 e.q. of a solution of hydrobromic acid in ethanol (1 mol/L in EtOH) was added to precipitate a crystal by suspension method. The mixture was stirred at 40°C overnight, cooled to room temperature, and filtered. The solid was further dried in a vacuum drying oven overnight (vacuum drying under reduced pressure at 50°C) to constant weight to obtain hydrobromide crystal form A. After detection and analysis, it has the XRPD pattern as shown in Figure 16, and the DSC spectrum as shown in Figure 17.

### (8) Preparation of hydrobromide crystal form B

150 mg of the free base of the compound was weighed, to which 8 mL of dichloromethane was added. The mixture was stirred at 40°C, and the solid cannot be dissolved completely. 380 µL (1.1 e.q.) of a solution of hydrobromic acid in methanol (1 mol/L in MeOH) was added to precipitate a crystal by suspension method. The mixture was kept at 40°C overnight, cooled to room temperature, and centrifuged rapidly to remove the supernatant. The solid was dried under vacuum at 40°C to constant weight to obtain hydrobromide crystal form B. After detection and analysis, it has the XRPD pattern as shown in Figure 18, the DSC spectrum as shown in Figure 19, and the TGA spectrum as shown in Figure 20.

### (9) Preparation of crystal form C of hydrobromide

30 mg of the free base of the compound was weighed, to which 1 mL of tetrahydrofuran was added. The mixture was stirred at 50°C, and the solid was dissolved. 77 µL (1.1 e.q.) of a solution of hydrobromic acid in ethanol (1 mol/L in EtOH) was added, and precipitate immediately formed. The mixture was kept at 50°C overnight, cooled to room temperature, and centrifuged rapidly to remove the supernatant. The solid was dried under vacuum at 40°C to constant weight to obtain crystal form C of hydrobromide. After detection and analysis, it has the XRPD pattern as shown in Figure 21.

### (10) Preparation of crystal form I of the free base of the compound

6 g of the free base of the compound was weighed and dissolved in DCM/acetone = 3:1 (250 ml), followed by addition of activated carbon (0.5 g) and anhydrous sodium sulfate (15 g) and stirring at 40°C for 30 minutes. The mixture was filtered. The filtrate was concentrated to dryness under reduced pressure, dissolved in 50 ml of acetone, and concentrated to dryness. 100 ml of acetone was added. The solution was heated to 65°C to reflux, and the solid was still not dissolved well. 200 ml of n-heptane was added dropwise, and the solution was heated and stirred for 1 hour. The heating was turned off, and the solution was cooled to room temperature naturally, and stirred for 1 hour. The mixture was filtered to obtain an off-white solid, which was dried under vacuum to constant weight to obtain crystal form I of the free base. After detection and analysis, it has the XRPD pattern as shown in Figure 22, and the DSC spectrum as shown in Figure 23.

### (11) Preparation of crystal form II of the free base

10 mg of the free base of the compound (crystal form I) was added to a 2 mL glass flask, to which 200 µL of ethanol solvent was added to obtain a suspension. The suspension was pulped on a magnetic stirrer at 40°C for 3 days, and centrifuged to remove the supernatant. The solid was further dried in a vacuum drying oven overnight (vacuum drying under reduced pressure at 50°C) to obtain crystal form II. After detection and analysis, it has the XRPD pattern as shown in Figure 24, and the DSC spectrum as shown in Figure 25.

### 4. Solid stability experiment

4.1 Solid stability experiment of crystal form A of methanesulfonate of the compound
4.1.1 Experimental objective: To investigate the physical and chemical stability of the crystal form of the compound under the conditions of high temperature, high humidity, as well as high temperature and high humidity, so as to provide a basis for crystal form screening and storage of the crystal form of the compound.
4.1.2 Instruments and conditions of liquid chromatography analysis

**Table 39**

| Instrument | HPLC Agilent 1260 | |
|---|---|---|
| Mobile phase A | 0.05% TFA in H₂O | |
| Mobile phase B | 0.05% TFA in ACN | |
| Flow rate | 1.0 mL/min | |
| Injection volume | 5 µL | |
| Chromatographic column | Zorbax BONUS RP (75*4.6mm,3.5um) | |
| Column temperature | 30°C | |
| Detection wavelength | 262 nm | |

| Elution gradient (min) | A% | B% |
|---|---|---|
| 0.00 | 60 | 40 |
| 12.00 | 25 | 75 |
| 12.01 | 60 | 40 |
| 15.00 | 60 | 40 |

4.1.3 Experimental protocol: An appropriate amount of methanesulfonate crystal form A, ethanesulfonate crystal form A or sulfate crystal form B was weighed, and left to stand under the conditions of high temperature (60°C), high humidity (92.5%RH), high temperature and high humidity (50°C&75%RH) for 7 and 14 days. The content of the salt was determined by HPLC using the external standard method, and the change of relevant substances was calculated by using the chromatographic peak area normalization method.
4.1.4 Experimental results

**Table 40**

| Solid stability (% increase of impurities) | | | | | | | |
|---|---|---|---|---|---|---|---|
| | Crystal form A of methanesulfonate | | | Crystal form A of Ethanesulfonate | | Crystal form B of Sulfate | |
| Condition | Impurities | Day 7 | Day 14 | Day 7 | Day 14 | Day 7 | Day 14 |
| High temperature | % increase of total impurities | <0.2 | <0.1 | >0.2 | <0.1 | >0.2 | >0.4 |
| | % maximum increase of single impurity | <0.1 | <0.1 | <0.1 | <0.1 | <0.1 | >0.1 |
| High humidity | % increase of total impurities | <0.1 | <0.1 | <0.1 | <0.1 | <0.1 | <0.1 |
| | % maximum increase of single impurity | <0.1 | <0.1 | <0.1 | <0.1 | <0.1 | <0.1 |
| High temperature and high humidity | % increase of total impurities | <0.1 | <0.1 | >0.3 | >0.2 | >0.2 | >0.2 |
| | % maximum increase of single impurity | <0.1 | <0.1 | >0.1 | >0.1 | >0.1 | >0.1 |

Crystal form A of methanesulfonate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide is stable under the conditions of high temperature, high humidity, as well as high temperature and high humidity. Crystal form A of ethanesulfonate is stable under the conditions of high temperature, and high humidity. Crystal form B of sulfate is stable under the condition of high humidity.

### 5. Solubility experiment in different media

5.1 Solubility experiment of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide in different media
5.1.1 Experimental objective: To investigate the solubility of methanesulfonate crystal form A and the free base of the compound in media with different pH, water, Fasted State Simulated Gastric Fluid (FaSSGF), Fasted State Simulated Intestinal Fluid (FaSSIF) and Fed State Simulated Intestinal Fluid (FeSSIF), so as to provide a basis for evaluating the druggability of the salt.
5.1.2 Experimental protocol: 1 to 2 mg of crystal form II of the free base of the compound and different salt forms were weighed and added to 1.5 mL vials for liquid chromatography, respectively. 1 mL of buffers with different pH, Fasted State Simulated Gastric Fluid (FaSSGF), Fasted State Simulated Intestinal Fluid (FaSSIF), Fed State Simulated Intestinal Fluid (FeSSIF), and pure water were added respectively. The vials were placed on a spin mixer overnight at room temperature. After 24 hours, the sample solution was filtered through a 0.45 µm mixed aqueous fiber membrane. The content of the compound in the filtrate was determined by HPLC.

**Table 41**

| HPLC content analysis method | | |
|---|---|---|
| Instrument | Agilent 1260 | |
| Mobile phase A | 0.05% TFA in H₂O | |
| Mobile phase B | 0.05% TFA in ACN | |
| Flow rate | 1.0 mL/min | |
| Injection volume | 5 µL | |
| Chromatographic column | Zorbax BONUS RP (75*4.6mm,3.5um) | |
| Column temperature | 30°C | |
| Detection wavelength | 262 nm | |

| Elution gradient (min) | A% | B% |
|---|---|---|
| 0.00 | 60 | 40 |
| 12.00 | 25 | 75 |
| 12.01 | 60 | 40 |
| 15.00 | 60 | 40 |

5.1.3 Experimental results are shown in the figure:

**Table 42**

| Medium | Solubility (mg/mL) | |
|---|---|---|
| | Crystal form II of the free base | Crystal form A of methanesulfonate |
| USP Buffer pH 1 | 0.0006 | 0.0023 |
| USP Buffer pH 2 | 0.0006 | 0.0023 |
| USP Buffer pH 3 | 0.0011 | 0.0043 |
| USP Buffer pH 4 | 0.0010 | 0.0036 |
| USP Buffer pH 5 | 0.0008 | 0.0030 |
| USP Buffer pH 6 | 0.0006 | 0.0024 |
| USP Buffer pH 7 | 0.0005 | 0.0022 |
| USP Buffer pH 8 | 0.0005 | 0.0018 |
| FaSSIF | 0.0011 | 0.0078 |
| FeSSIF | 0.0025 | 0.0113 |
| FaSSGF | 0.0007 | 0.0033 |
| H₂O | 0.0006 | 0.0019 |

The solubility of crystal form of methanesulfonate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide in the corresponding media is increased by more than four times compared with that of crystal form II of the free base, indicating that the solubility can be significantly improved after salt formation.

### 6. Thermodynamic stability experiment

6.1 Polymorphism screening of the methanesulfonate of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide
6.1.1 Experimental objective: To obtain thermodynamically stable crystal form of methanesulfonate through polymorphism screening.
6.1.2 Experimental protocol: 3 to 5 mg of crystal form A of methanesulfonate was weighed, to which 100 µl of the corresponding solvent was added. The mixture was magnetically pulped in a metal bath at 35°C for 24 hours, and centrifuged. The resulting solid was dried, and the XRPD thereof was determined.
6.1.3 Experimental results are shown in the table below.

**Table 43**

| **No.** | **Solvent** | **Methanesulfonate** |
|---|---|---|
| | | **XRPD result** |
| - | Initial crystal form | Crystal form A |
| 1 | Ethyl acetate | Crystal form A |
| 2 | Isopropanol | Crystal form A |
| 3 | Isopropyl acetate | Crystal form A |
| 4 | Dioxane | Crystal form A |
| 5 | Methyl *tert*-butyl ether | Crystal form A |
| 6 | Isopropyl ether | Crystal form A |
| 7 | Toluene | Crystal form A |

The above results indicate that crystal form A of methanesulfonate of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide is a thermodynamically stable crystal form of methanesulfonate.

### 7. Hygroscopicity experiment

7.1 Hygroscopicity experiment of crystal forms of different salts of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide
7.1.1 Experimental objective: To investigate the hygroscopicity of crystal forms of different salts of compound 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide at different relative humidity, so as to provide a basis for storage.
7.1.2 Experimental protocol: Crystal forms of different salts of the compound were placed in saturated water vapor with different relative humidity to achieve dynamic equilibrium between the compound and water vapor, and the percentage of hygroscopic weight gain of the compound after equilibrium was calculated.

**Table 44 Dynamic Vapor Sorption (DVS) instrument parameters**

| | |
|---|---|
| Instrument mode | SMS Intrinsic |
| Experimental temperature | 25 °C |
| Drying time | 0%RH 120 min |
| Balanced dm/dt | 0.02%/min (min. 10 min, max. 180 min) |
| RH(%) step size of measurement | 10% |
| Gradient of measurement | 0-95-0% |
| Number of cycles | 2 |

7.1.3 Experimental results

**Table 45 Experimental results of crystal form A of methanesulfonate**

| Cycle | **Target relative humidity %** | **% percentage of mass change** | |
|---|---|---|---|
| | | **Sorption** | **Desorption** |
| Cycle 1 | 0.0 | 0.000 | -0.029 |
| | 10.0 | 0.051 | 0.030 |
| | 20.0 | 0.096 | 0.082 |
| | 30.0 | 0.138 | 0.133 |
| | 40.0 | 0.179 | 0.190 |
| | 50.0 | 0.222 | 0.253 |
| | 60.0 | 0.272 | 0.320 |
| | 70.0 | 0.340 | 0.395 |
| | 80.0 | 0.446 | 0.502 |
| | 90.0 | 0.700 | 0.760 |
| | 95.0 | 1.109 | 1.109 |
| | | | |
| Cycle 2 | 0.0 | -0.029 | -0.046 |
| | 10.0 | 0.024 | 0.014 |
| | 20.0 | 0.073 | 0.067 |
| | 30.0 | 0.118 | 0.118 |
| | 40.0 | 0.162 | 0.177 |
| | 50.0 | 0.209 | 0.241 |
| | 60.0 | 0.263 | 0.305 |
| | 70.0 | 0.331 | 0.378 |
| | 80.0 | 0.433 | 0.480 |
| | 90.0 | 0.675 | 0.734 |
| | 95.0 | 1.076 | 1.076 |

**Table 46 Experimental results of crystal form A of ethanesulfonate**

| Cycle | **Target relative humidity %** | **% percentage of mass change** | |
|---|---|---|---|
| | | **Sorption** | **Desorption** |
| Cycle 1 | 0.0 | 0.000 | -0.012 |
| | 10.0 | 0.051 | 0.042 |
| | 20.0 | 0.094 | 0.090 |
| | 30.0 | 0.139 | 0.137 |
| | 40.0 | 0.183 | 0.192 |
| | 50.0 | 0.231 | 0.260 |
| | 60.0 | 0.297 | 0.334 |
| | 70.0 | 0.401 | 0.442 |
| | 80.0 | 0.572 | 0.625 |
| | 90.0 | 1.025 | 1.135 |
| | 95.0 | 1.927 | 1.927 |
| | | | |
| Cycle 2 | 0.0 | -0.012 | -0.016 |
| | 10.0 | 0.037 | 0.035 |
| | 20.0 | 0.083 | 0.083 |
| | 30.0 | 0.127 | 0.130 |
| | 40.0 | 0.174 | 0.184 |
| | 50.0 | 0.228 | 0.250 |
| | 60.0 | 0.295 | 0.321 |
| | 70.0 | 0.388 | 0.421 |
| | 80.0 | 0.541 | 0.584 |
| | 90.0 | 0.942 | 1.020 |
| | 95.0 | 1.683 | 1.683 |

**Table 47 Experimental results of crystal form B of sulfate**

| Cycle | **Target relative humidity (%)** | **% percentage of mass change** | |
|---|---|---|---|
| | | **Sorption** | **Desorption** |
| Cycle 1 | 0.0 | -0.002 | -0.027 |
| | 10.0 | 0.099 | 0.087 |
| | 20.0 | 0.171 | 0.168 |
| | 30.0 | 0.236 | 0.250 |
| | 40.0 | 0.303 | 0.341 |
| | 50.0 | 0.376 | 0.456 |
| | 60.0 | 0.455 | 0.540 |
| | 70.0 | 0.552 | 0.652 |
| | 80.0 | 0.691 | 0.777 |
| | 90.0 | 1.010 | 1.118 |
| | 95.0 | 1.608 | 1.608 |
| | | | |
| Cycle 2 | 0.0 | -0.027 | -0.051 |
| | 10.0 | 0.070 | 0.065 |
| | 20.0 | 0.147 | 0.149 |
| | 30.0 | 0.218 | 0.230 |
| | 40.0 | 0.287 | 0.322 |
| | 50.0 | 0.369 | 0.422 |
| | 60.0 | 0.450 | 0.529 |
| | 70.0 | 0.545 | 0.632 |
| | 80.0 | 0.682 | 0.761 |
| | 90.0 | 1.007 | 1.087 |
| | 95.0 | 1.618 | 1.618 |

**Table 48 Experimental results of crystal form A of hydrochloride**

| Cycle | **Target relative humidity %** | **% percentage of mass change** | |
|---|---|---|---|
| | | **Sorption** | **Desorption** |
| Cycle 1 | 0.0 | 0.000 | -0.041 |
| | 10.0 | 0.131 | 0.130 |
| | 20.0 | 0.275 | 0.313 |
| | 30.0 | 4.325 | 7.258 |
| | 40.0 | 7.346 | 7.532 |
| | 50.0 | 7.466 | 7.722 |
| | 60.0 | 7.581 | 7.825 |
| | 70.0 | 7.709 | 7.898 |
| | 80.0 | 7.840 | 7.968 |
| | 90.0 | 8.018 | 8.071 |
| | 95.0 | 8.141 | 8.141 |
| | | | |
| Cycle 2 | 0.0 | -0.041 | -0.062 |
| | 10.0 | 0.095 | 0.098 |
| | 20.0 | 0.236 | 0.273 |
| | 30.0 | 5.345 | 7.191 |
| | 40.0 | 7.269 | 7.462 |
| | 50.0 | 7.383 | 7.649 |
| | 60.0 | 7.492 | 7.738 |
| | 70.0 | 7.610 | 7.806 |
| | 80.0 | 7.745 | 7.872 |
| | 90.0 | 7.925 | 7.972 |
| | 95.0 | 8.047 | 8.047 |

**Table 49 Experimental results of crystal form B of hydrobromide**

| Cycle | **Target relative humidity %** | **% percentage of mass change** | |
|---|---|---|---|
| | | **Sorption** | **Desorption** |
| Cycle 1 | 0.0 | 0.000 | -0.007 |
| | 10.0 | 0.077 | 0.140 |
| | 20.0 | 0.166 | 0.238 |
| | 30.0 | 3.980 | 4.093 |
| | 40.0 | 4.140 | 4.247 |
| | 50.0 | 4.233 | 4.359 |
| | 60.0 | 4.321 | 4.448 |
| | 70.0 | 4.415 | 4.532 |
| | 80.0 | 4.529 | 4.627 |
| | 90.0 | 4.715 | 4.787 |
| | 95.0 | 4.947 | 4.947 |
| | | | |
| Cycle 2 | 0.0 | -0.007 | -0.019 |
| | 10.0 | 0.070 | 0.124 |
| | 20.0 | 0.153 | 0.220 |
| | 30.0 | 3.900 | 3.962 |
| | 40.0 | 4.030 | 4.112 |
| | 50.0 | 4.120 | 4.221 |
| | 60.0 | 4.207 | 4.310 |
| | 70.0 | 4.297 | 4.393 |
| | 80.0 | 4.405 | 4.484 |
| | 90.0 | 4.579 | 4.641 |
| | 95.0 | 4.796 | 4.796 |

The above results indicate that crystal form A of methanesulfonate of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide has the lowest hygroscopicity, and the hygroscopic weight gain at 80% relative humidity does not exceed 0.5%. According to the Chinese Pharmacopoeia's description of hygroscopicity (measured at a temperature of 25°C±1°C and a relative humidity of 80%±2%) and the definition of hygroscopic weight gain, crystal form A of methanesulfonate is slightly hygroscopic.

## Claims

1. An acid salt of a compound of formula (I-a) or a stereoisomer thereof, 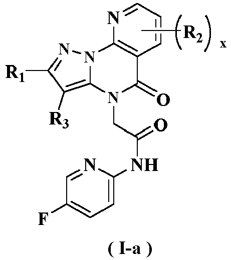 wherein:
R₁ is selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₁₂ cycloalkyl, -(CH₂)ₙC(O)Rₐ, 3 to 12 membered heterocyclyl, C₆₋₁₄ aryl and 5 to 14 membered heteroaryl, the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₁₂ cycloalkyl, -(CH₂)ₙC(O)Rₐ, 3 to 12 membered heterocyclyl, C₆₋₁₄ aryl and 5 to 14 membered heteroaryl are each optionally substituted by one or more substituents selected from the group consisting of deuterium, halogen, amino, hydroxy, cyano, nitro, oxo, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3 to 12 membered heterocyclyl, C₆₋₁₂ aryl and 5 to 12 membered heteroaryl;
Rₐ is selected from the group consisting of hydrogen, deuterium, halogen, amino, nitro, hydroxy, cyano, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally substituted by one or more substituents selected from the group consisting of deuterium, halogen, amino, nitro, hydroxy, cyano, carboxy, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
R₂ is selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₁₂ cycloalkyl, 3 to 12 membered heterocyclyl, C₆₋₁₄ aryl and 5 to 14 membered heteroaryl, the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₁₂ cycloalkyl, 3 to 12 membered heterocyclyl, C₆₋₁₄ aryl and 5 to 14 membered heteroaryl are each optionally substituted by one or more substituents selected from the group consisting of deuterium, halogen, amino, hydroxy, cyano, nitro, oxo, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3 to 12 membered heterocyclyl, C₆₋₁₂ aryl and 5 to 12 membered heteroaryl;
R₃ is selected from the group consisting of hydrogen, deuterium, halogen, amino, hydroxy, cyano, oxo, thioxo, C₁₋₆ alkyl, C₂₋₆ alkenyl, C₂₋₆ alkynyl, C₁₋₆ deuterated alkyl, C₁₋₆ haloalkyl, C₁₋₆ hydroxyalkyl, C₁₋₆ alkoxy, C₁₋₆ haloalkoxy, C₃₋₈ cycloalkyl, 3 to 12 membered heterocyclyl, C₆₋₁₄ aryl and 5 to 14 membered heteroaryl; and
x is an integer from 0 to 3, preferably 0, 1 or 2, more preferably 0 or 1;
n is an integer from 0 to 3, preferably 0, 1 or 2, more preferably 0 or 1;
the acid in the acid salt is an inorganic acid or an organic acid; preferably, the inorganic acid is selected from the group consisting of hydrochloric acid, sulfuric acid, nitric acid, hydrobromic acid, hydrofluoric acid, hydroiodic acid and phosphoric acid; the organic acid is selected from the group consisting of 2,5-dihydroxybenzoic acid, 1-hydroxy-2-naphthoic acid, acetic acid, ethanesulfonic acid, dichloroacetic acid, trichloroacetic acid, acetohydroxamic acid, adipic acid, benzenesulfonic acid, 4-chlorobenzenesulfonic acid, benzoic acid, 4-acetamidobenzoic acid, 4-aminobenzoic acid, capric acid, caproic acid, caprylic acid, cinnamic acid, citric acid, cyclohexane sulfamic acid, camphorsulfonic acid, aspartic acid, camphoric acid, gluconic acid, glucuronic acid, glutamic acid, erythorbic acid, lactic acid, malic acid, mandelic acid, pyroglutamic acid, tartaric acid, dodecyl sulfuric acid, dibenzoyl tartaric acid, ethane-1,2-disulfonic acid, ethanesulfonic acid, formic acid, fumaric acid, galactonic acid, gentisic acid, glutaric acid, 2-oxoglutaric acid, glycolic acid, hippuric acid, isethionic acid, lactobionic acid, ascorbic acid, aspartic acid, lauric acid, camphoric acid, maleic acid, malonic acid, methanesulfonic acid, 1,5-naphthalenedisulfonic acid, naphthalene-2-sulfonic acid, nicotinic acid, oleic acid, orotic acid, oxalic acid, palmitic acid, pamoic acid, propionic acid, salicylic acid, 4-aminosalicylic acid, sebacic acid, stearic acid, succinic acid, thiocyanic acid, undecenoic acid, trifluoroacetic acid, benzenesulfonic acid, *p*-toluenesulfonic acid and L-malic acid; and preferably selected from the group consisting of hydrochloric acid, sulfuric acid, phosphoric acid, ethanesulfonic acid, benzenesulfonic acid, methanesulfonic acid, fumaric acid, isethionic acid, oxalic acid and hydrobromic acid.

2. The acid salt according to claim 1, **characterized in that** the formula (I-a) is further as shown in formula (II-a):

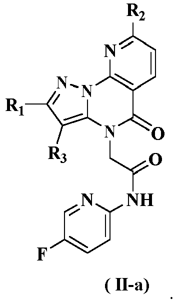

3. The acid salt according to claim 1 or 2, **characterized in that**,
R₁ is selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₈ cycloalkyl, -(CH₂)ₙC(O)Rₐ, 3 to 8 membered heterocyclyl, C₆₋₁₀ aryl and 5 to 10 membered heteroaryl, the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₈ cycloalkyl, -(CH₂)ₙC(O)Rₐ, 3 to 8 membered heterocyclyl, C₆₋₁₀ aryl and 5 to 10 membered heteroaryl are each optionally substituted by one or more substituents selected from the group consisting of deuterium, halogen, amino, hydroxy, cyano, nitro, oxo, C₁₋₃ alkyl, C₂₋₃ alkenyl, C₂₋₃ alkynyl, C₁₋₃ deuterated alkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3 to 8 membered heterocyclyl, C₆₋₁₀ aryl and 5 to 10 membered heteroaryl;
Rₐ is selected from the group consisting of hydrogen, deuterium, halogen, amino, nitro, hydroxy, cyano, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally substituted by one or more substituents selected from the group consisting of deuterium, halogen, amino, nitro, hydroxy, cyano, carboxy, oxo, thioxo, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
preferably, R₁ is selected from the group consisting of hydrogen, halogen, amino, cyano, C₁₋₃ alkyl, C₂₋₆ alkenyl, C₁₋₆ haloalkyl, C₁₋₆ alkoxy, C₁₋₆ haloalkoxy, C₁₋₆ hydroxyalkyl, C₃₋₈ cycloalkyl, -(CH₂)ₙC(O)Rₐ, 3 to 8 membered heterocyclyl containing 1 to 3 atoms selected from the group consisting of nitrogen, oxygen and sulfur, C₆₋₁₀ aryl and 5 to 10 membered heteroaryl containing 1 to 3 atoms selected from the group consisting of nitrogen, oxygen and sulfur, the amino, C₁₋₆ alkyl, C₂₋₆ alkenyl, C₁₋₆ haloalkyl, C₁₋₆ alkoxy, C₁₋₆ haloalkoxy, C₁₋₆ hydroxyalkyl, -(CH₂)ₙC(O)Rₐ, C₃₋₈ cycloalkyl, 3 to 8 membered heterocyclyl containing 1 to 3 atoms selected from the group consisting of nitrogen, oxygen and sulfur, C₆₋₁₀ aryl and 5 to 10 membered heteroaryl containing 1 to 3 atoms selected from the group consisting of nitrogen, oxygen and sulfur are each optionally substituted by one or more substituents selected from the group consisting of deuterium, halogen, amino, hydroxy, cyano, nitro, oxo, C₁₋₃ alkyl, C₁₋₃ deuterated alkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3 to 8 membered heterocyclyl, C₆₋₁₀ aryl and 5 to 10 membered heteroaryl;
Rₐ is selected from the group consisting of hydrogen, deuterium, halogen, amino, cyano, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl and 4 to 6 membered heterocyclyl containing 1 to 2 atoms selected from the group consisting of N and O, the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₃₋₆ cycloalkyl and 4 to 6 membered heterocyclyl containing 1 to 2 atoms selected from the group consisting of N and O are each optionally substituted by one or more substituents selected from the group consisting of deuterium, halogen, amino, hydroxy, cyano, nitro, oxo, C₁₋₃ alkyl, C₁₋₃ deuterated alkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3 to 8 membered heterocyclyl, C₆₋₁₀ aryl and 5 to 10 membered heteroaryl;
more preferably, R₁ is selected from the group consisting of: 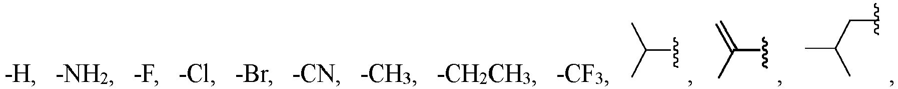 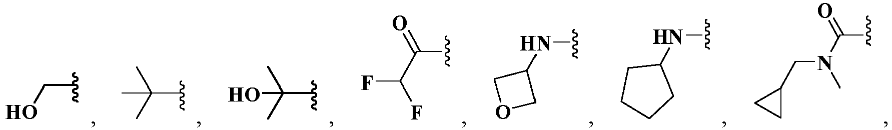 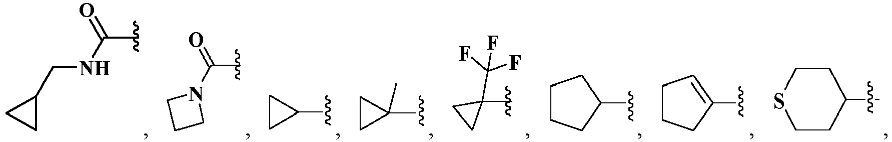 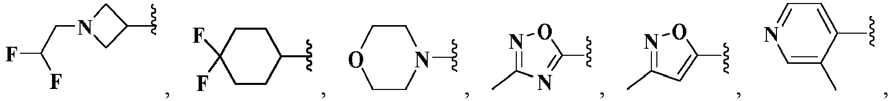 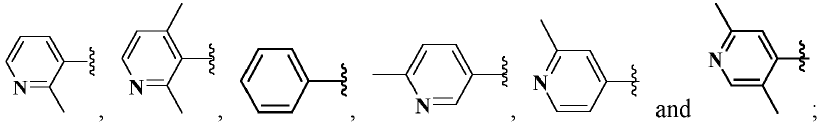
R₂ is selected from the group consisting of hydrogen, deuterium, halogen, amino, hydroxy, cyano, nitro, C₁₋₃ alkyl, C₂₋₃ alkenyl, C₂₋₃ alkynyl, C₁₋₃ deuterated alkyl, C₁₋₃ haloalkyl, C₁₋₃ alkoxy, C₁₋₃ haloalkoxy, C₁₋₃ hydroxyalkyl, C₃₋₈ cycloalkyl, 3 to 8 membered heterocyclyl, C₆₋₁₀ aryl and 5 to 10 membered heteroaryl; and preferably selected from the group consisting of hydrogen, amino, cyano, fluorine, chlorine, bromine, methyl, isopropyl, trifluoromethyl, methoxy, cyclopropyl and morpholinyl;
R₃ is selected from the group consisting of hydrogen, deuterium, halogen, amino, hydroxy, cyano, oxo, thioxo, C₁₋₃ alkyl, C₂₋₃ alkenyl, C₂₋₃ alkynyl, C₁₋₃ deuterated alkyl, C₁₋₃ haloalkyl, C₁₋₃ hydroxyalkyl, C₁₋₃ alkoxy, C₁₋₃ haloalkoxy, C₃₋₈ cycloalkyl, 3 to 8 membered heterocyclyl, C₆₋₁₀ aryl and 5 to 1 membered heteroaryl; and preferably selected from the group consisting of hydrogen and cyano.

4. The acid salt according to any one of claims 1 to 3, **characterized in that**, the specific structure of the compound is as follows: 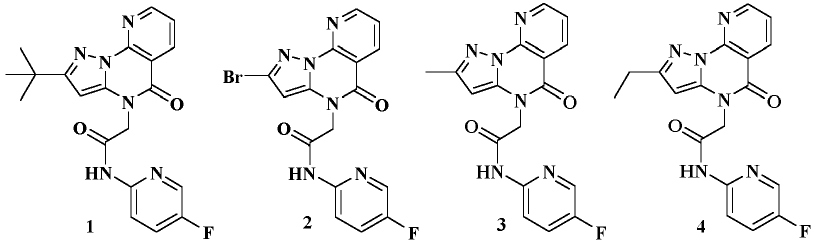 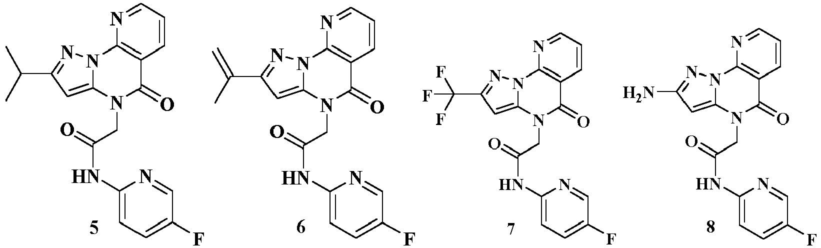 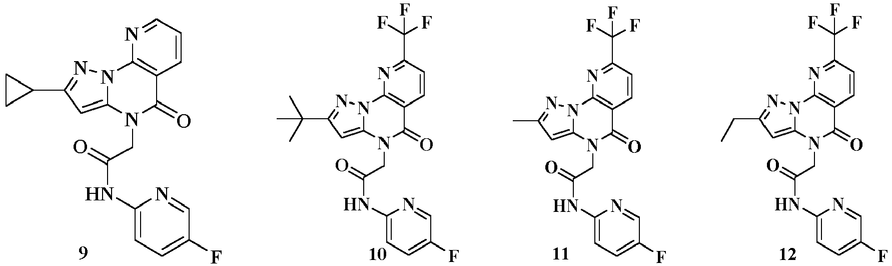 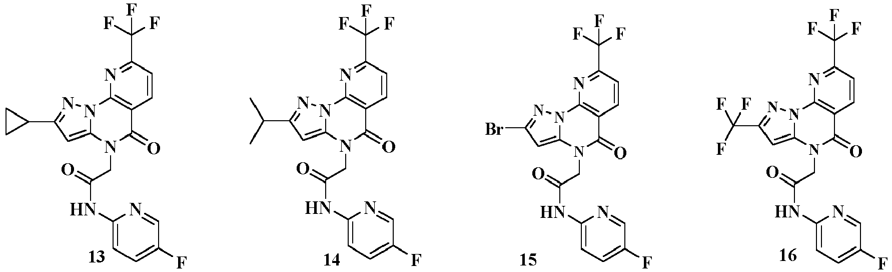 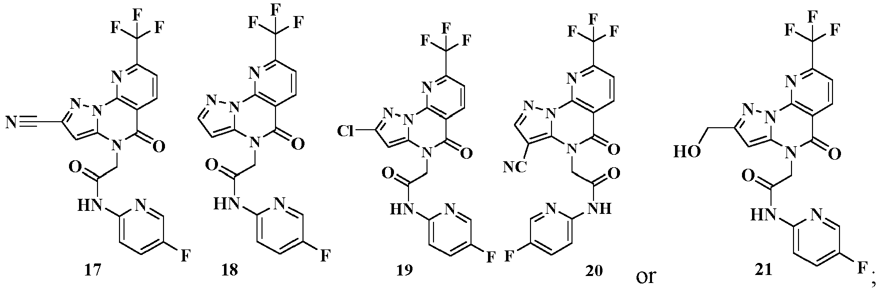 the acid in the acid salt is selected from the group consisting of isethionic acid, hydrochloric acid, sulfuric acid, 1,5-naphthalene disulfonic acid, methanesulfonic acid, hydrobromic acid, ethanesulfonic acid, phosphoric acid, benzenesulfonic acid, oxalic acid, maleic acid, adipic acid, hydrochloric acid, citric acid, malonic acid, L-malic acid, pamoic acid, *p*-toluenesulfonic acid and fumaric acid; and preferably selected from the group consisting of hydrochloric acid, sulfuric acid, methanesulfonic acid, hydrobromic acid and ethanesulfonic acid.

5. The acid salt according to any one of claims 1 to 4, **characterized in that**, the number of acid in the acid salt is 0.2 to 3; preferably 0.2, 0.5, 1, 1.5, 2, 2.5 or 3; more preferably 0.5, 1, 2 or 3; and further preferably 1.

6. The acid salt according to any one of claims 1 to 5, **characterized in that**, the acid salt is a hydrate or anhydrate; when the acid salt is a hydrate, the number of water is 0.2 to 3; preferably 0.2, 0.5, 1, 1.5, 2, 2.5 or 3; and more preferably 0.5, 1, 2 or 3.

7. The acid salt according to any one of claims 1 to 6, **characterized in that**, the acid salt is a crystal form;
preferably, the crystal form is a crystal form of the acid salt of compound 2-(2-(*tert*-butyl)-5-oxopyrazolo[1,5-a]pyrido[3,2-e]pyrimidin-4(5H)-yl)-N-(5-fluoropyri din-2-yl)acetamide;
a crystal form of the acid salt of 2-(2-(*tert*-butyl)-5-oxo-8-(trifluoromethyl)pyrazolo[1,5-a]pyrido[3,2-e]pyrimidin-4(5H) -yl)-N-(5-fluoropyridin-2-yl)acetamide;
a crystal form of the acid salt of 2-(2-ethyl-5-oxo-8-(trifluoromethyl)pyrazolo[1,5-a]pyrido[3,2-e]pyrimidin-4(5H)-yl)-N -(5-fluoropyridin-2-yl)acetamide;
a crystal form of the acid salt of 2-(2-cyclopropyl-5-oxo-8-(trifluoromethyl)pyrazolo[1,5-a]pyrido[3,2-e]pyrimidin-4(5H )-yl)-N-(5-fluoropyridin-2-yl)acetamide;
a crystal form of the acid salt of 2-(2,5-dimethylpyridin-4-yl)-5-oxo-8-(trifluoromethyl)pyrazolo[1,5-a]pyrido[3,2-e]pyri midin-4(5H)-yl)-N-(5-fluoropyridin-2-yl)acetamide;
a crystal form of the acid salt of N-(5-fluoropyridin-2-yl)-2-(2-(1-methylcyclopropyl)-5-oxo-8-(trifluoromethyl)pyrazolo [1,5-a]pyrido[3,2-e]pyrimidin-4(5H)-yl)acetamide;
a crystal form of the acid salt of 2-(2-bromo-5-oxo-8-(trifluoromethyl)pyrazolo[1,5-a]pyrido[3,2-e]pyrimidin-4(5H)-yl)-N-(5-fluoropyridin-2-yl)acetamide;
a crystal form of the acid salt of 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide;
a crystal form of the acid salt of N-(5-fluoropyridin-2-yl)-2-(5-oxo-8-(trifluoromethyl)pyrazolo[1,5-a]pyrido[3,2-e]pyri midin-4(5H)-yl)acetamide;
a crystal form of the acid salt of 2-(3-cyano-5-oxo-8-(trifluoromethyl)pyrazolo[1,5-a]pyrido[3,2-e]pyrimidin-4(5H)-yl)-N-(5-fluoropyridin-2-yl)acetamide;
more preferably, the crystal form of the acid salt is a crystal form of isethionate, a crystal form of sulfate, a crystal form of hydrochloride, a crystal form of 1,5-naphthalene disulfonate, a crystal form of methanesulfonate, a crystal form of ethanesulfonate, a crystal form of hydrobromide, a crystal form of phosphate, a crystal form of benzenesulfonate, a crystal form of oxalate, a crystal form of maleate, a crystal form of adipate, a crystal form of hydrochloride, a crystal form of citrate, a crystal form of malonate, a crystal form of L-malate, a crystal form of pamoate, a crystal form of *p*-toluenesulfonate or a crystal form of fumarate.

8. The crystal form of the acid salt according to claim 7, **characterized in that**,
the crystal form of the acid salt 2-(2-cyano-5-oxo-8-(trifluoromethyl)pyrazolo[1,5-a]pyrido[3,2-e]pyrimidin-4(5H)-yl)-N-(5-fluoropyridin-2-yl)acetamide is:
crystal form A of methanesulfonate, the X-ray powder diffraction pattern of which has a diffraction peak of 13.7±0.2°, or a diffraction peak of 21.9±0.2°, or a diffraction peak of 20.4±0.2°, or a diffraction peak of 15.4±0.2°, or a diffraction peak of 19.6±0.2°, or a diffraction peak of 16.4±0.2°, or a diffraction peak of 9.3±0.2°, or a diffraction peak of 5.3±0.2°, or a diffraction peak of 7.9±0.2°, or a diffraction peak of 11.9±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form A of methanesulfonate comprises at least one or more diffraction peaks at 2θ of 13.7±0.2°, 16.4±0.2°, 21.9±0.2°, preferably comprises 2 of the above diffraction peaks, more preferably comprises 3 of the above diffraction peaks; optionally, can further comprise at least one diffraction peak at 2θ of 13.9±0.2°, 20.4±0.2°, 15.4±0.2°, 5.3±0.2°, 11.9±0.2°, 9.3±0.2°, preferably comprises 2, 3, 4 or 5 of the above diffraction peaks;
more preferably, the X-ray powder diffraction pattern of crystal form A of methanesulfonate optionally further comprises one or more diffraction peaks at 2θ of 7.9±0.2°, 19.6±0.2°, 17.6±0.2°, 18.8±0.2°, 21.0±0.2°, 23.3±0.2°, 24.1±0.2°; preferably comprises at least any 2 to 3, or 4 to 5, or 6 to 7 of the above diffraction peaks; further preferably comprises at least any 2, 3, 4, 5, 6, 7 of the above diffraction peaks;
further preferably, the X-ray powder diffraction pattern of crystal form A of methanesulfonate comprises one or more diffraction peaks at 2θ of 5.3±0.2°, 7.9±0.2°, 9.3±0.2°, 11.9±0.2°, 13.7±0.2°, 13.9±0.2°, 15.4±0.2°, 16.4±0.2°, 17.6±0.2°, 18.8±0.2°, 19.6±0.2°, 20.4±0.2°, 21.0±0.2°, 21.9±0.2°, 23.3±0.2°, 24.1±0.2°, preferably comprises any 4, 5, 6, 8 or 10 of the above diffraction peaks;
more further preferably, the X-ray powder diffraction pattern of crystal form A of methanesulfonate is as shown in Figure 1, or the DSC spectrum thereof is substantially as shown in Figure 2, or the TGA spectrum thereof is substantially as shown in Figure 3;
or, crystal form A of ethanesulfonate, the X-ray powder diffraction pattern of which has a diffraction peak of 15.0±0.2°, or a diffraction peak of 21.1±0.2°, or a diffraction peak of 23.1±0.2°, or a diffraction peak of 19.8±0.2°, or a diffraction peak of 12.5±0.2°, or a diffraction peak of 9.0±0.2°, or a diffraction peak of 12.3±0.2°, or a diffraction peak of 24.6±0.2°, or a diffraction peak of 10.3±0.2°, or a diffraction peak of 6.1±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form A of ethanesulfonate comprises at least one or more diffraction peaks at 2θ of 15.0±0.2°, 21.1±0.2°, 23.1±0.2°, preferably comprises 2 of the above diffraction peaks, more preferably comprises 3 of the above diffraction peaks; optionally, can further comprise at least one diffraction peak at 2θ of 19.8±0.2°, 12.5±0.2°, 9.0±0.2°, 12.3±0.2°, 24.6±0.2°, preferably comprises 2, 3, 4 or 5 of the above diffraction peaks;
more preferably, the X-ray powder diffraction pattern of crystal form A of ethanesulfonate optionally further comprises one or more diffraction peaks at 2θ of 10.3±0.2°, 6.1±0.2°, 16.1±0.2°, 19.2±0.2°, 23.6±0.2°, 30.7±0.2°, 9.6±0.2°; preferably comprises at least any 2 to 3, or 4 to 5, or 6 to 7 of the above diffraction peaks; further preferably comprises at least any 2, 3, 4, 5, 6, 7 of the above diffraction peaks;
further preferably, the X-ray powder diffraction pattern of crystal form A of ethanesulfonate comprises one or more diffraction peaks at 2θ of 15.0±0.2°, 21.1±0.2°, 23.1±0.2°, 19.8±0.2°, 12.5±0.2°, 9.0±0.2°, 12.3±0.2°, 24.6±0.2°, 10.3±0.2°, 6.1±0.2°, 16.1±0.2°, 19.2±0.2°, 23.6±0.2°, 30.7±0.2°, 9.6±0.2°, preferably comprises any 4, 5, 6, 8 or 10 of the above diffraction peaks;
more further preferably, the X-ray powder diffraction pattern of crystal form A of ethanesulfonate is as shown in Figure 4, or the DSC spectrum thereof is substantially as shown in Figure 5, or the TGA spectrum thereof is substantially as shown in Figure 6;
or, crystal form A of sulfate, the X-ray powder diffraction pattern of which has a diffraction peak of 22.5±0.2°, or a diffraction peak of 15.9±0.2°, or a diffraction peak of 22.3±0.2°, or a diffraction peak of 16.8±0.2°, or a diffraction peak of 22.9±0.2°, or a diffraction peak of 32.1±0.2°, or a diffraction peak of 14.0±0.2°, or a diffraction peak of 21.1±0.2°, or a diffraction peak of 11.2±0.2°, or a diffraction peak of 26.1±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form A of sulfate comprises at least one or more diffraction peaks at 2θ of 22.5±0.2°, 15.9±0.2°, 22.3±0.2°, preferably comprises 2 of the above diffraction peaks, more preferably comprises 3 of the above diffraction peaks; optionally, can further comprise at least one diffraction peak at 2θ of 16.8±0.2°, 22.9±0.2°, 32.1±0.2°, 14.0±0.2°, 21.1±0.2°, preferably comprises 2, 3, 4 or 5 of the above diffraction peaks;
more preferably, or, the X-ray powder diffraction pattern of crystal form A of sulfate optionally also comprises one or more diffraction peaks at 2θ of 11.2±0.2°, 26.1±0.2°, 28.2±0.2°, 37.8±0.2°, 15.5±0.2°, 26.5±0.2°, 36.4±0.2°; preferably comprises at least any 2 to 3, or 4 to 5, or 6 to 7 of the above diffraction peaks; further preferably comprises at least any 2, 3, 4, 5, 6, 7 of the above diffraction peaks;
further preferably, the X-ray powder diffraction pattern of crystal form A of sulfate comprises one or more diffraction peaks at 2θ of 22.5±0.2°, 15.9±0.2°, 22.3±0.2°, 16.8±0.2°, 22.9±0.2°, 32.1±0.2°, 14.0±0.2°, 21.1±0.2°, 11.2±0.2°, 26.1±0.2°, 28.2±0.2°, 37.8±0.2°, 15.5±0.2°, 26.5±0.2°, 36.4±0.2°, preferably comprises any 4, 5, 6, 8 or 10 of the above diffraction peaks;
more further preferably, the X-ray powder diffraction pattern of crystal form A of sulfate is as shown in Figure 7, the DSC spectrum thereof is substantially as shown in Figure 8;
or, crystal form B of sulfate, the X-ray powder diffraction pattern of which has a diffraction peak of 15.3±0.2°, or a diffraction peak of 21.5±0.2°, or a diffraction peak of 10.6±0.2°, or a diffraction peak of 19.8±0.2°, or a diffraction peak of 20.1±0.2°, or a diffraction peak of 12.6±0.2°, or a diffraction peak of 25.2±0.2°, or a diffraction peak of 9.2±0.2°, or a diffraction peak of 9.9±0.2°, or a diffraction peak of 23.4±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form B of sulfate comprises at least one or more diffraction peaks at 2θ of 15.3±0.2°, 21.5±0.2°, 10.6±0.2°, preferably comprises 2 of the above diffraction peaks, more preferably comprises 3 of the above diffraction peaks; optionally, can further comprise at least one diffraction peak at 2θ of 19.8±0.2°, 20.1±0.2°, 12.6±0.2°, 25.2±0.2°, 9.2±0.2°, preferably comprises 2, 3, 4 or 5 of the above diffraction peaks;
more preferably, the X-ray powder diffraction pattern of crystal form B of sulfate optionally further comprises one or more diffraction peaks at 2θ of 9.9±0.2°, 23.4±0.2°, 6.3±0.2°, 16.7±0.2°, 23.9±0.2°, 33.8±0.2°, 16.3±0.2°; preferably comprises at least any 2 to 3, or 4 to 5, or 6 to 7 of the above diffraction peaks; further preferably comprises at least any 2, 3, 4, 5, 6, 7 of the above diffraction peaks;
further preferably, the X-ray powder diffraction pattern of crystal form B of sulfate comprises one or more diffraction peaks at 2θ of 15.3±0.2°, 21.5±0.2°, 10.6±0.2°, 19.8±0.2°, 20.1±0.2°, 12.6±0.2°, 25.2±0.2°, 9.2±0.2°, 9.9±0.2°, 23.4±0.2°, 6.3±0.2°, 16.7±0.2°, 23.9±0.2°, 33.8±0.2°, 16.3±0.2°, preferably comprises any 4, 5, 6, 8 or 10 of the above diffraction peaks;
more further preferably, the X-ray powder diffraction pattern of crystal form B of sulfate is as shown in Figure 9, the DSC spectrum thereof is substantially as shown in Figure 10, the TGA spectrum thereof is substantially as shown in Figure 11;
or, crystal form A of hydrochloride, the X-ray powder diffraction pattern of which has a diffraction peak of 15.0±0.2°, or a diffraction peak of 23.9±0.2°, or a diffraction peak of 9.7±0.2°, or a diffraction peak of 5.3±0.2°, or a diffraction peak of 24.8±0.2°, or a diffraction peak of 29.5±0.2°, or a diffraction peak of 7.5±0.2°, or a diffraction peak of 21.8±0.2°, or a diffraction peak of 21.3±0.2°, or a diffraction peak of 10.6±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form A of hydrochloride comprises at least one or more diffraction peaks at 2θ of 15.0±0.2°, 23.9±0.2°, 9.7±0.2°, preferably comprises 2 of the above diffraction peaks, more preferably comprises 3 of the above diffraction peaks; optionally, can further comprise at least one diffraction peak at 2θ of 5.3±0.2°, 24.8±0.2°, 29.5±0.2°, 7.5±0.2°, 21.8±0.2°, preferably comprises 2, 3, 4 or 5 of the above diffraction peaks;
more preferably, the X-ray powder diffraction pattern of crystal form A of hydrochloride optionally also comprises one or more diffraction peaks at 2θ of 21.3±0.2°, 10.6±0.2°, 16.9±0.2°, 16.0±0.2°, 18.4±0.2°, 25.8±0.2°, 28.4±0.2°; preferably comprises at least any 2 to 3, or 4 to 5, or 6 to 7 of the above diffraction peaks; further preferably comprises at least any 2, 3, 4, 5, 6, 7 of the above diffraction peaks;
further preferably, the X-ray powder diffraction pattern of crystal form A of hydrochloride comprises one or more diffraction peaks at 2θ of 15.0±0.2°, 23.9±0.2°, 9.7±0.2°, 5.3±0.2°, 24.8±0.2°, 29.5±0.2°, 7.5±0.2°, 21.8±0.2°, 21.3±0.2°, 10.6±0.2°, 16.9±0.2°, 16.0±0.2°, 18.4±0.2°, 25.8±9.2°, 28.4±0.2°, preferably comprises any 4, 5, 6, 8 or 10 of the above diffraction peaks;
more further preferably, the X-ray powder diffraction pattern of crystal form A of hydrochloride is as shown in Figure 12, or the DSC spectrum thereof is substantially as shown in Figure 13, or the TGA spectrum thereof is substantially as shown in Figure 14;
or, crystal form B of hydrochloride, the X-ray powder diffraction pattern of which has a diffraction peak of 15.9±0.2°, or a diffraction peak of 22.2±0.2°, or a diffraction peak of 5.2±0.2°, or a diffraction peak of 21.7±0.2°, or a diffraction peak of 26.0±0.2°, or a diffraction peak of 4.6±0.2°, or a diffraction peak of 28.4±0.2°, or a diffraction peak of 9.2±0.2°, or a diffraction peak of 17.3±0.2°, or a diffraction peak of 15.2±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form B of hydrochloride comprises at least one or more diffraction peaks at 2θ of 15.9±0.2°, 22.2±0.2°, 5.2±0.2°, preferably comprises 2 of the above diffraction peaks, more preferably comprises 3 of the above diffraction peaks; optionally, can further comprise at least one diffraction peak at 2θ of 21.7±0.2°, 26.0±0.2°, 4.6±0.2°, 28.4±0.2°, 9.2±0.2°, preferably comprises 2, 3, 4 or 5 of the above diffraction peaks;
more preferably, the X-ray powder diffraction pattern of crystal form B of hydrochloride optionally further comprises one or more diffraction peaks at 2θ of 17.3±0.2°, 15.2±0.2°, 10.5±0.2°, 38.0±0.2°, 20.3±0.2°, 23.8±0.2°, 29.5±0.2°; preferably comprises at least any 2 to 3, or 4 to 5, or 6 to 7 of the above diffraction peaks; further preferably comprises at least any 2, 3, 4, 5, 6, 7 of the above diffraction peaks;
further preferably, the X-ray powder diffraction pattern of crystal form B of hydrochloride comprises one or more diffraction peaks at 2θ of 15.9±0.2°, 22.2±0.2°, 5.2±0.2°, 21.7±0.2°, 26.0±0.2°, 4.6±0.2°, 28.4±0.2°, 9.2±0.2°, 17.3±0.2°, 15.2±0.2°, 10.5±0.2°, 38.0±0.2°, 20.3±0.2°, 23.8±0.2°, 29.5±0.2°, preferably comprises any 4, 5, 6, 8 or 10 of the above diffraction peaks;
more further preferably, the X-ray powder diffraction pattern of crystal form B of hydrochloride is as shown in Figure 15;
or, crystal form A of hydrobromide, the X-ray powder diffraction pattern of which has a diffraction peak of 5.3±0.2°, or a diffraction peak of 22.7±0.2°, or a diffraction peak of 14.8±0.2°, or a diffraction peak of 10.5±0.2°, or a diffraction peak of 22.5±0.2°, or a diffraction peak of 28.0±0.2°, or a diffraction peak of 30.0±0.2°, or a diffraction peak of 23.4±0.2°, or a diffraction peak of 23.3±0.2°, or a diffraction peak of 26.5±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form A of hydrobromide comprises at least one or more diffraction peaks at 2θ of 5.3±0.2°, 22.7±0.2°, 14.8±0.2°, preferably comprises 2 of the above diffraction peaks, more preferably comprises 3 of the above diffraction peaks; optionally, can further comprise at least one diffraction peak at 2θ of 10.5±0.2°, 22.5±0.2°, 28.0±0.2°, 30.0±0.2°, 23.4±0.2°, preferably comprises 2, 3, 4 or 5 of the above diffraction peaks;
more preferably, the X-ray powder diffraction pattern of crystal form A of hydrobromide optionally also comprises one or more diffraction peaks at 2θ of 23.3±0.2°, 26.5±0.2°, 34.9±0.2°, 15.8±0.2°, 25.0±0.2°, 31.9±0.2°, 37.0±0.2°; preferably comprises at least any 2 to 3, or 4 to 5, or 6 to 7 of the above diffraction peaks; further preferably comprises any 2, 3, 4, 5, 6, 7 of the above diffraction peaks;
further preferably, the X-ray powder diffraction pattern of crystal form A of hydrobromide comprises one or more diffraction peaks at 2θ of 5.3±0.2°, 22.7±0.2°, 14.8±0.2°, 10.5±0.2°, 22.5±0.2°, 28.0±0.2°, 30.0±0.2°, 23.4±0.2°, 23.3±0.2°, 26.5±0.2°, 34.9±0.2°, 15.8±0.2°, 25.9±9.2°, 31.9±0.2°, 37.0±0.2°, preferably comprises any 4, 5, 6, 8 or 10 of the above diffraction peaks;
more further preferably, the X-ray powder diffraction pattern of crystal form A of hydrobromide is as shown in Figure 16, or the DSC spectrum thereof is substantially as shown in Figure 17;
or, crystal form B of hydrobromide, the X-ray powder diffraction pattern of which has a diffraction peak of 23.4±0.2°, or a diffraction peak of 15.9±0.2°, or a diffraction peak of 16.2±0.2°, or a diffraction peak of 14.2±0.2°, or a diffraction peak of 5.3±0.2°, or a diffraction peak of 10.6±0.2°, or a diffraction peak of 23.1±0.2°, or a diffraction peak of 24.1±0.2°, or a diffraction peak of 14.8±0.2°, or a diffraction peak of 9.5±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form B of hydrobromide comprises at least one or more diffraction peaks at 2θ of 23.4±0.2°, 15.9±0.2°, 16.2±0.2°, preferably comprises 2 of the above diffraction peaks, more preferably comprises 3 of the above diffraction peaks; optionally, can further comprise at least one diffraction peak at 2θ of 14.2±0.2°, 5.3±0.2°, 10.6±0.2°, 23.1±0.2°, 24.1±0.2°, preferably comprises 2, 3, 4 or 5 of the above diffraction peaks;
more preferably, the X-ray powder diffraction pattern of crystal form B of hydrobromide optionally also comprises one or more diffraction peaks at 2θ of 14.8±0.2°, 9.5±0.2°, 16.9±0.2°, 13.9±0.2°, 29.5±0.2°, 32.2±0.2°, 22.2±0.2°; preferably comprises at least any 2 to 3, or 4 to 5, or 6 to 7 of the above diffraction peaks; further preferably comprises any 2, 3, 4, 5, 6, 7 of the above diffraction peaks;
further preferably, the X-ray powder diffraction pattern of crystal form B of hydrobromide comprises one or more diffraction peaks at 2θ of 23.4±0.2°, 15.9±0.2°, 16.2±0.2°, 14.2±0.2°, 5.3±0.2°, 10.6±0.2°, 23.1±0.2°, 24.1±0.2°, 14.8±0.2°, 9.5±0.2°, 16.9±0.2°, 13.9±0.2°, 29.5±0.2°, 32.2±0.2°, 22.2±0.2°, preferably comprises any 4, 5, 6, 8 or 10 of the above diffraction peaks;
more further preferably, the X-ray powder diffraction pattern of crystal form B of hydrobromide is as shown in Figure 18, or the DSC spectrum thereof is substantially as shown in Figure 19, or the TGA spectrum thereof is substantially as shown in Figure 20;
or, crystal form C of hydrobromide, the X-ray powder diffraction pattern of which has a diffraction peak of 5.2±0.2°, or a diffraction peak of 15.7±0.2°, or a diffraction peak of 22.3±0.2°, or a diffraction peak of 10.5±0.2°, or a diffraction peak of 17.4±0.2°, or a diffraction peak of 38.0±0.2°, or a diffraction peak of 26.3±0.2°, or a diffraction peak of 28.0±0.2°; preferably comprises any 2 to 5, or 3 to 5, or 3 to 6, or 3 to 8, or 5 to 8, or 6 to 8 of the above diffraction peaks, more preferably comprises any 6, 7 or 8 of the above diffraction peaks;
preferably, the X-ray powder diffraction pattern of crystal form C of hydrobromide comprises at least one or more diffraction peaks at 2θ of 5.2±0.2°, 15.7±0.2°, 22.3±0.2°, preferably comprises 2 of the above diffraction peaks, more preferably comprises 3 of the above diffraction peaks; optionally, can further comprise at least one diffraction peak at 2θ of 10.5±0.2°, 17.4±0.2°, 38.0±0.2°, 26.3±0.2°, 28.0±0.2°, preferably comprises 2, 3, 4 or 5 of the above diffraction peaks;
more preferably, the X-ray powder diffraction pattern of crystal form C of hydrobromide comprises one or more diffraction peaks at 2θ of 5.2±0.2°, 15.7±0.2°, 22.3±0.2°, 10.5±0.2°, 17.4±0.2°, 38.0±0.2°, 26.3±0.2°, 28.0±0.2°, preferably comprises any 4, 5, 6 or 8 of the above diffraction peaks;
further preferably, the X-ray powder diffraction pattern of crystal form C of hydrobromide is as shown in Figure 21.

9. The crystal form of the acid salt according to claim 8, **characterized in that**, positions of diffraction peaks with relative peak intensity of top ten in the X-ray powder diffraction pattern of each crystal form have a 2θ error of ±0.2° to ±0.5°, preferably ±0.2° to ±0.3°, most preferably ±0.2° relative to the diffraction peaks at the corresponding positions in the X-ray powder diffraction pattern figure.

10. The crystal form of the acid salt according to claim 8 or 9, **characterized in that**, the crystal form of the acid salt is a hydrate or anhydrate.

11. A method for preparing the acid salt according to any one of claims 1 to 7 or the crystal form of acid salt according to any one of claims 8 to 10, comprising the following steps of:
1) weighing an appropriate amount of free base and dissolving it in a solvent;
2) adding an appropriate amount of acid and stirring;
3) centrifuging rapidly or standing to obtain the acid salt;
or, comprising the following steps of:
1) weighing an appropriate amount of free base and dissolving it in a solvent;
2) adding an appropriate amount of acid and stirring;
3) centrifuging and drying to obtain the acid salt crystal form;
the solvent is an organic solvent, preferably at least one of methanol, ethanol, tetrahydrofuran, 2-methyltetrahydrofuran, toluene, isopropyl acetate, *tert*-butanol, *n*-butanol, acetone, 2-butanone, dichloromethane, ethyl acetate or 1,4-dioxane;
the acid is selected from the group consisting of hydrochloric acid, sulfuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetamidobenzoic acid, 4-aminobenzoic acid, capric acid, caproic acid, caprylic acid, cinnamic acid, citric acid, cyclohexane sulfamic acid, camphorsulfonic acid, aspartic acid, camphoric acid, gluconic acid, glucuronic acid, glutamic acid, erythorbic acid, lactic acid, malic acid, mandelic acid, pyroglutamic acid, tartaric acid, dodecyl sulfuric acid, dibenzoyl tartaric acid, ethane-1,2-disulfonic acid, methanesulfonic acid, ethanesulfonic acid, formic acid, fumaric acid, galactonic acid, gentisic acid, glutaric acid, 2-oxoglutaric acid, glycolic acid, hippuric acid, isethionic acid, lactobionic acid, ascorbic acid, aspartic acid, lauric acid, camphoric acid, maleic acid, malonic acid, 1,5-naphthalenedisulfonic acid, naphthalene-2-sulfonic acid, nicotinic acid, oleic acid, orotic acid, oxalic acid, palmitic acid, pamoic acid, propionic acid, salicylic acid, 4-aminosalicylic acid, sebacic acid, stearic acid, succinic acid, thiocyanic acid, undecenoic acid, trifluoroacetic acid, benzenesulfonic acid, *p*-toluenesulfonic acid and L-malic acid; and preferably selected from the group consisting of hydrochloric acid, sulfuric acid, phosphoric acid, ethanesulfonic acid, benzenesulfonic acid, methanesulfonic acid, fumaric acid, isethionic acid, oxalic acid and hydrobromic acid.

12. A pharmaceutical composition, comprising a therapeutically effective amount of the acid salt according to any one of claims 1 to 7 or the crystal form of the acid salt according to any one of claims 8 to 10, and one or more pharmaceutically acceptable carriers, diluents or excipients.

13. Use of the acid salt according to any one of claims 1 to 7 or the crystal form of acid salt according to any one of claims 8 to 10 or the pharmaceutical composition according to claim 11 in the preparation of a P2X3 inhibitor drug.

14. Use of the acid salt according to any one of claims 1 to 7 or the crystal form of the acid salt according to any one of claims 8 to 10 or the pharmaceutical composition according to claim 11 in the preparation of a medicament for treating neurogenic disease;
preferably, the neurogenic disease is selected from the group consisting of gynecological disease, urinary tract disease state, respiratory disorder and pain-related disease or condition;
more preferably, the neurogenic disease is selected from the group consisting of endometriosis, overactive bladder, pulmonary fibrosis and chronic cough; the pain-related disease or condition is selected from the group consisting of neuropathic pain and uterine fibroid-related pain or discomfort.

15. A compound of formula (I) or a pharmaceutically acceptable salt thereof: 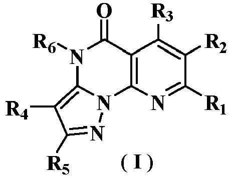 wherein:
R₁, R₂, R₃ are each independently selected from the group consisting of hydrogen, deuterium, halogen, amino, nitro, hydroxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl, heteroaryloxy and -(CH₂)ₙ₁C(O)Rₐ;
R₄, R₅ are each independently selected from the group consisting of hydrogen, deuterium, halogen, amino, nitro, hydroxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl, heteroaryloxy and -(CH₂)ₙ₁C(O)Rₐ;
R₆ is selected from the group consisting of hydrogen and a protecting group;
Rₐ is selected from the group consisting of hydrogen, deuterium, halogen, amino, nitro, hydroxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl and heteroaryloxy;
the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl, heteroaryloxy and -(CH₂)ₙ₁- are each optionally substituted by one or more substituents selected from the group consisting of hydrogen, deuterium, halogen, amino, nitro, hydroxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl and heteroaryloxy; n1 is 0, 1, 2, 3 or 4;
preferably,
when R₁, R₂, R₃, R₄ and R₆ are hydrogen at the same time, R₅ is not -C(CH₃)₃ or -COOCH₃;
when R₁ is -CF₃, and R₂, R₃, R₄ and R₆ are hydrogen at the same time, R₅ is not -CH₂CH₃, H or Br;
when R₁ is -CF₃, and R₂, R₃, R₅ and R₆ are hydrogen at the same time, R₄ is not -CN;
more preferably, the structure of the compound of formula (I) is shown in formula (I-1): 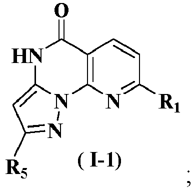
further preferably, the structure of the compound is selected from the group consisting of formula (I-1-1), formula (I-1-2) and formula (I-1-3): 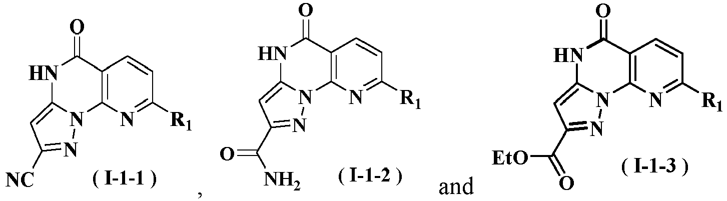
wherein,
R₁ is selected from the group consisting of hydrogen, deuterium, halogen, cyano, C₁₋₈ alkyl, C₁₋₈ alkoxy, C₁₋₈ haloalkyl and C₁₋₈ haloalkoxy, preferably selected from the group consisting of cyano, C₁₋₃ alkyl, C₁₋₃ alkoxy, C₁₋₃ haloalkyl and C₁₋₃ haloalkoxy, and more preferably selected from the group consisting of methyl, ethyl, trifluoromethyl, methoxy and cyano;
R₅ is selected from the group consisting of hydrogen, deuterium, halogen, cyano, C₁₋₈ alkyl, C₁₋₈ alkoxy, C₁₋₈ haloalkyl, C₁₋₈ haloalkoxy and -C(O)Rₐ, preferably selected from the group consisting of hydrogen, deuterium, halogen, cyano and -C(O)Rₐ, and more preferably selected from the group consisting of hydrogen, deuterium, fluorine, chlorine, bromine and -C(O)Rₐ;
Rₐ is selected from the group consisting of hydrogen, deuterium, halogen, amino, C₁₋₃ alkyl, C₁₋₃ alkoxy, C₁₋₃ haloalkyl and C₁₋₃ haloalkoxy, and preferably selected from the group consisting of amino, methoxy, ethoxy and isopropoxy.

16. A method for preparing a compound of formula (I'), **characterized by** comprising step (a): 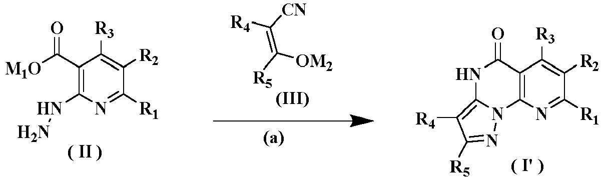
reacting a compound of formula (II) with a compound of formula (III) under a condition that can obtain the compound of formula (I') or a salt thereof to obtain the compound of formula (I') or a salt thereof;
M₁, M₂ are each independently selected from the group consisting of H, Li, Na, K and Cs, M₁ is preferably H, M₂ is preferably Na or K;
R₁, R₂, R₃, R₄, R₅ are as defined in claim 15.

17. A method for preparing a compound of formula (VII), **characterized by** comprising step (b): 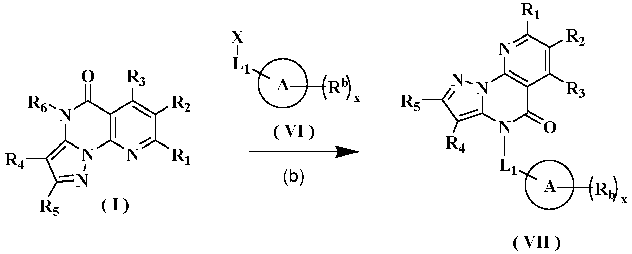
reacting a compound of formula (I) with a compound of formula (VI) to obtain the compound of formula (VII);
X is halogen, preferably fluorine, chlorine or bromine, and more preferably chlorine or bromine;
L₁ is selected from the group consisting of -(CH₂)ₙ₂-, -(CH₂)ₙ₂O-, -(CH₂)ₙ₂S-, -(CH₂)ₙ₂NR_{c}-, -(CH₂)ₙ₂C(O)NR_{c}- and -(CH₂)ₙ₂NR_{c}C(O)-, preferably -CH₂C(O)NH-;
n2 is 0, 1, 2 or 3;
x is 0, 1, 2 or 3;
R_{b}, R_{c} are each independently selected from the group consisting of hydrogen, deuterium, halogen, amino, nitro, hydroxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl and heteroaryloxy;
the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl, heteroaryloxy and -(CH₂)ₙ₂- are each optionally substituted by one or more substituents selected from the group consisting of hydrogen, deuterium, halogen, amino, nitro, hydroxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl and heteroaryloxy; R_{b} is preferably halogen; R_{c} is preferably H;
ring A is selected from the group consisting of cycloalkyl, heterocyclyl, aryl and heteroaryl, preferably
and/or, 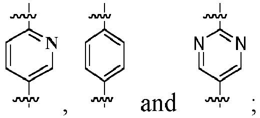
the compound of formula (VI) is preferably 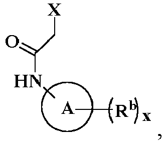 more preferably 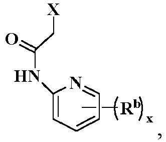 and further preferably 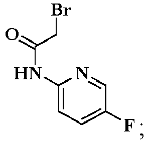
R₁, R₂, R₃, R₄, R₅, R₆ are as defined in claim 15;
optionally, the method for preparing the compound of formula (VII) also comprises the step of preparing the compound of formula (I), the step of preparing the compound of formula (I) comprises the method according to claim 16.

18. A method for preparing a compound of formula (I-3), **characterized by** comprising step (c-1): 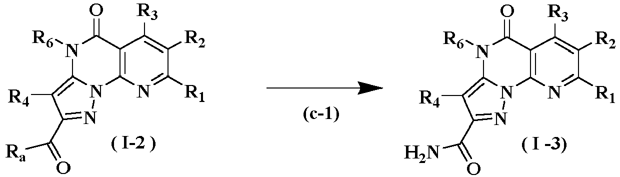
subjecting a compound of formula (I-2) to an amination reduction reaction in the presence of ammonia gas or ammonia gas equivalents to obtain the compound of formula (I-3);
the ammonia gas equivalent is an organic solution of ammonia or aqueous ammonia; the organic solution of ammonia is preferably ammonia in methanol, ammonia in ethanol, ammonia in isopropanol, or ammonia in dioxane;
Rₐ is selected from the group consisting of hydrogen, deuterium, halogen, hydroxy, alkoxy, haloalkoxy, aryloxy and heteroaryloxy, the alkoxy, haloalkoxy, aryloxy and heteroaryloxy are each optionally substituted by one or more substituents selected from the group consisting of deuterium, halogen, amino, nitro, hydroxy, cyano, alkyl, deuterated alkyl, haloalkyl, hydroxyalkyl, alkoxy, haloalkoxy, alkenyl, alkynyl, heterocyclylalkyl, cycloalkyl, heterocyclyl, aryl, aryloxy, heteroaryl and heteroaryloxy;
R₁, R₂, R₃, R₄, R₆ are as defined in claim 15.

19. A method for preparing a compound of formula (I-4), **characterized by** comprising step (d-1): 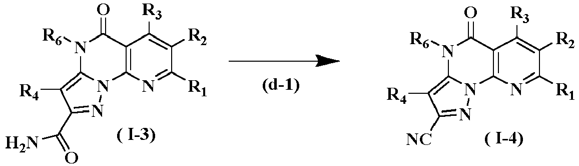
reacting a compound of formula (I-3) in the presence of a dehydrating agent to obtain the compound of formula (I-4); the dehydrating agent is preferably one or more of acetic anhydride, trifluoroacetic anhydride, P₂O₅, cyanuric chloride, phosphorus oxychloride, phosphorus trichloride, and concentrated sulfuric acid;
R₁, R₂, R₃, R₄, R₆ are as defined in claim 15.

20. Use of a compound of formula (I), a compound of formula (II) and a compound of formula (III), the compound of formula (I), the compound of formula (II) and the compound of formula (III) are used as intermediates in the preparation of P2X3 inhibitor, the P2X3 inhibitor is preferably a P2X3 inhibitor containing a core structure of 4,5-dihydropyrazolo[1,5-a]pyrido[3,2-e]pyrimidine, and more preferably a compound of formula (VII-4): 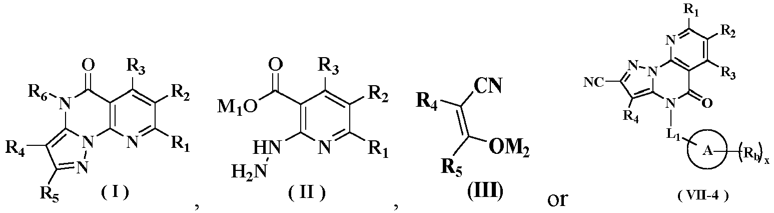
L₁, x, R_{b}, ring A, R₁, R₂, R₃, R₄, R₅, R₆ are as defined in claim 17;
M₁, M₂ are as defined in claim 16.
